(19) Europäisches Patentamt
European Patent Office
Office européen des brevets

(11) Publication number: **0 215 355 B1**

(12) # EUROPEAN PATENT SPECIFICATION

(45) Date of publication of patent specification: **06.04.94**

(51) Int. Cl.⁵: **C07H 17/08**, A61K 31/70

(21) Application number: **86111940.2**

(22) Date of filing: **28.08.86**

The file contains technical information submitted after the application was filed and not included in this specification

(54) **Erythromycin derivative and process for preparing the same.**

(30) Priority: **31.08.85 JP 190957/85**
**28.02.86 JP 41412/86**
**31.05.86 JP 124738/86**

(43) Date of publication of application:
**25.03.87 Bulletin 87/13**

(45) Publication of the grant of the patent:
**06.04.94 Bulletin 94/14**

(84) Designated Contracting States:
**AT BE CH DE FR GB IT LI LU NL SE**

(56) References cited:
**EP-A- 0 180 347**
**FR-A- 2 189 014**

**JOURNAL OF ANTIMICROBIAL CHEMOTHERAPY, vol. 16, Suppl. A, 1985, pages 175-179, The British Society for Antimicrobial Chemotheraphy; G.P. ZARA et al.: "Effects of erythromycin on gastrointestinal tract motility"**

(73) Proprietor: **KITASATO KENKYUSHO**
**9-1, Shirogane 5-chome**
**Minato-ku, Tokyo 108(JP)**

(72) Inventor: **Omura, Satoshi**
**5-12-7, Seta**
**Setagaya-ku**
**Tokyo 158(JP)**
Inventor: **Itoh, Zen**
**5-10, Chiyoda -machi 1-chome**
**Maebashi-shi Gumma 371(JP)**

(74) Representative: **von Kreisler, Alek, Dipl.-Chem. et al**
**Patentanwälte**
**von Kreisler-Selting-Werner**
**Postfach 10 22 41**
**D-50462 Köln (DE)**

EP 0 215 355 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ANTIMICROBIAL AGENTS AND CHEMOTHERAPY, vol. 26, no. 6, December 1984, pages 863-869, American Society for Microbiology; Z. ITOH et al.: "Gastrointestinal motor-stimulating activity of macrolide antibiotics and analysis of their side effects on the canine gut"

THE JOURNAL OF ANTIBIOTICS, vol. XXVI, no. 10, 1973, pages 587-592; K. KROWICKI et al.: "Chemical modification of erythromycins IV. 8-Hydroxyerythromycin B"

THE JOURNAL OF ANTIBIOTICS, vol. XXVI, no. 10, 1973, pages 569-574; K. KROWICKI et al.: "Chemical modification of erythromycins I. 8,9-Anhydro-6-hemiketal of erythromycin A"

CHEMICAL ABSTRACTS, vol. 89, no. 5, 31st July 1978, page 694, abstract no. 44035g, Columbus, Ohio, US; M. BIEDRZYCKI et al.: "Erythromycin derivatives. Part VI. Carbamates of cyclic 11,12-carbonate of erythromycin A", & POL. J. CHEM. 1978, 52(2), 315-9

THE JOURNAL OF ANTIBIOTICS, vol. XXXVIII, no. 11, 1985, pages 1631-1632; S. OMURA et al.: "Gastrointestinal motor-stimulating activity of macrolide antibiotics and the structure-activity relationship"

Macrolide Antibiotics, ed. Satoshi Omura, Academic Press Inc., 1984

## Description

BACKGROUND OF THE INVENTION

(1) Technical Field

The present invention relates to erythromycin derivatives and salts thereof useful as stimulants for contractive motion of the digestive tract, exhibiting action for stimulating contractive motion of the digestive tract of mammals, and also to processes for producing the same.

(2) Background Information

The digestive tract consists of the stomach, the duodenum, the small intestine etc., and plays an important role in the digestion of food taken from the mouth. The contractive motion of the digestive tract is essential in order to perform the digestion smoothly. In a healthy man, the autonomous nerve system and digestive tract hormones function effectively to induce contraction of the digestive tract not only immediately after the intake of foods but also in a state where the digestive tract is empty, when such contraction has been considered absent. The movement in such empty digestive tract is transmitted from the stomach to the duodenum and to the small intestine, and plays an important role cleaning the digestive tract, thus preparing for next intake of foods (Z. Itoh, "Iden", 33, 29, 1979).

A stimulant for contraction of the digestive tract is expected to induce a normal movement of the digestive tract, in a human with weakened function of the digestive tract, thereby a healthy body being maintained.

Motilin is already known as a digestive tract hormone for stimulating the contraction of the digestive tract. This substance is a peptide, consisting of 22 amino acids and extracted by J. C. Brown in 1966 from the mucous membrane of a pig duodenum (J. C. Brown et al., Gastroenterology, 50, 333, 1966) , and is already synthesized chemically (E. Wünsch et al., Zeitschrift für Naturforsch, 28c, 235, 1973).

However the supply of motilin by extraction from natural substance or by chemical synthesis is not sufficient, and has not been possible in a large amount.

Further known gastrointestinal motor-stimulants are macrolide antibiotics, e.g. erythromycin, oleandomycin, kitasamycin and spiramycin (J. Antimicrobial Agents and Chemotherapy, Vol. 26, no. 6, 863-869 (1984)).

Effects of erythromycin propionate on the gastrointestinal tract motility is additionally disclosed in J. Antimicrobial Chemotherapy, 16, Suppl. A., 175-179 (1985).

SUMMARY OF THE INTENTION

In the course of a survey for providing a substance capable of stimulating the contraction of the digestive tract and adapted for a large supply, the present inventors have synthesized various derivatives from antibiotic erythromycin A and have found that said derivatives have a strong stimulating effect on the contraction of the digestive tract.

Based on this finding, the present inventors have made intensive efforts and have reached the present invention.

The present invention provides:

(1) a compound, or a salt thereof, represented by the general formula:

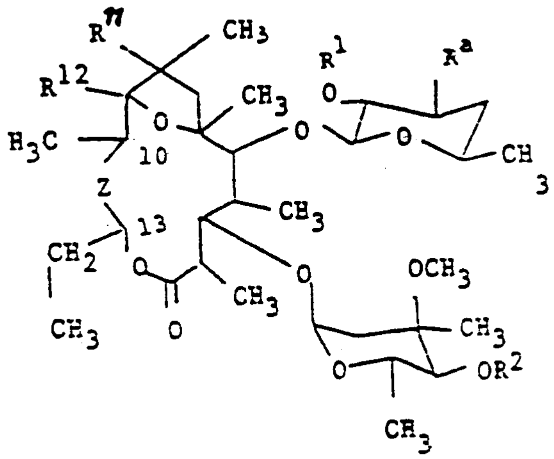

[I]

wherein $R^1$ is hydrogen;
$R^2$ is hydrogen;
Z is a group of the formula

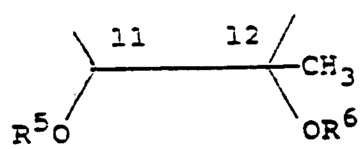

wherein $R^5$ is hydrogen and $R^6$ is hydrogen;
$R^a$ is a group of the formula

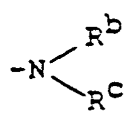

in which $R^b$ is methyl, and $R^c$ is ethyl or isopropyl,
or $R^a$ is a group of the formula

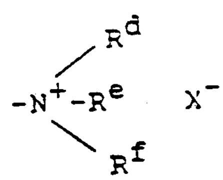

in which $R^d$ is methyl, and $R^e$ and $R^f$ which may be the same or different, are (1) a methyl, ethyl or isopropyl radical which may be substituted by hydroxyl, cyano, halogen or cylopropyl, or (2) a propargyl radical,
or together $R^e$ and $R^f$ form pyrrolidino or piperidino with the adjacent nitrogen atom;
X is a halogen anion;
and
$R^{11}$ and $R^{12}$ both taken together form a chemical bond,
or a salt thereof.

(2) a process for preparing a compound represented by the following formula or a salt thereof:

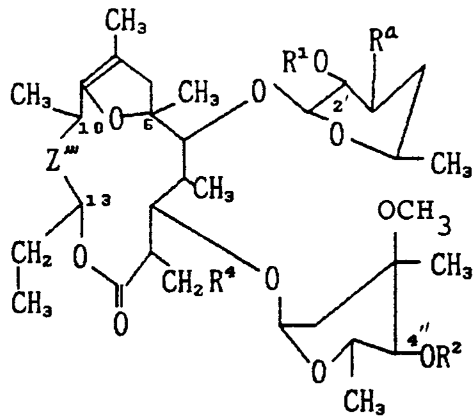

[4]

wherein Z''' is as defined for Z, $R^4$ is hydrogen, and the other symbols have the same meanings as defined above, which comprises treating a compound of the following formula or a salt thereof under an acidic condition:

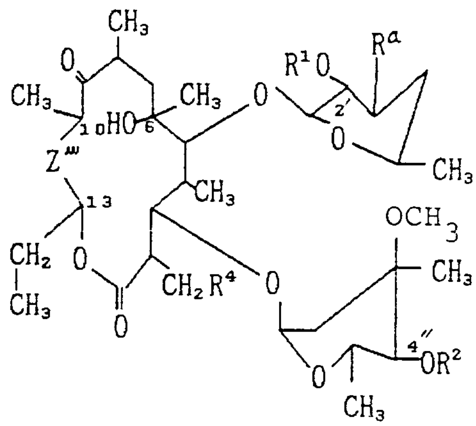

[3]

wherein Z''' is as defined for Z, $R^4$ is hydrogen, and the other symbols have the same meanings as defined above;

(3) a process for preparing a compound represented by the formula:

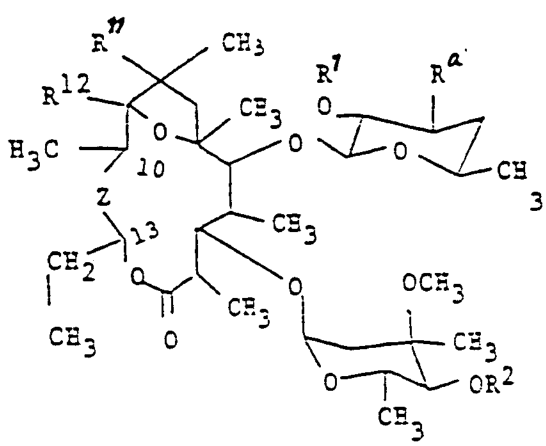

[I]

wherein the symbols ore as defined above, or a salt thereof, which comprises subjecting a compound of

the following formula to N-alkylation or N-alkynylation reaction:

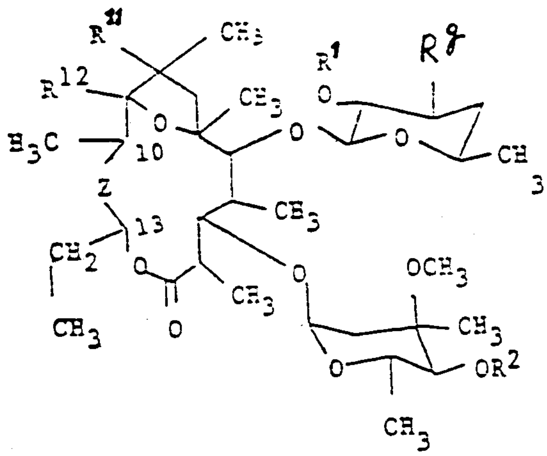

(5)

wherein $R^g$ is (1) a group of the formula -NH-$R^b$ or

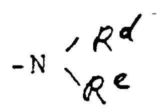

in which $R^b$, $R^d$ and $R^e$ are as defined above
(2) pyrrolidino or (3) piperidino, and the other symbols are as defined above.

DESCRIPTION OF THE PREFERRED EMBODIMENTS

In the foregoing formula, examples of the halogen anions represented by $X^{\ominus}$ include iodide ion, bromo ion and chloro ion.

In the compound (1) of the present invention, $R^a$ is preferable to be an N-methyl-N-ethylamino radical or a quaternary ammonium salt, i.e. $R^a$ having the formula

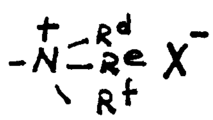

. Particularly, it is preferable that $R^d$ and $R^e$ form together with adjacent nitrogen atom a cyclic alkylamino radical such as pyrrolidine and piperidine. As X of the quaternary ammonium salt, there are preferably mentioned chlorine, bromine and iodine.

Preferred compounds are N-ethyl-de(N-methyl)-8,9-anhydroerythromycin A 6,9-hemiketal, 8,9-anhydroerythromycin A 6,9-hemiketal propargyl bromide, 8,9-anhydroerythromycin A 6,9-hemiketal propargyl chloride, 8,9-anhydroerythromycin A 6,9-hemiketal ethyl bromide, 8,9-anhydroerythromycin A 6,9-hemiketal 2-hydroxyethyl bromide, N-isopropyl de(N-methyl)-8,9-anhydroerythromycin A 6,9 hemiketal, dipropargyl-bis-(de-(N-methyl))-8,9-arhydroerythromycin A 6,9-hemiketal propargyl bromide and 8,9-anhydroerythromycin A 6,9-hemiketal allyl bromide

In the reaction in which the compound (3) is treated under acidic conditions to prepare the compound (4), there can be employed, for acidification, an organic acid such as acetic acid, pyridinium chloride or pyridinium paratoluene sulfonate.

The reaction temperature is 0 °C to 30 °C, the reaction time is 30 minutes to 1 hour, and the range of pH in reaction is 1 to 6. The solvent employable in the reaction is, for example, acetic acid, chloroform, dichloromethane or ether, and the reaction is preferably conducted under agitation.

By subjecting a compound (5) in which $R^g$ is the formula -NH-$R^b$ (wherein $R^b$ is the same meaning as defined above) to N-alkylation, or N-alkynylation, a compound (I) in which $R^a$ is the formula

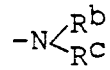

(wherein $R^b$ and $R^c$ have the same meanings as defined above) can be prepared.

The reaction is carried out by reacting a corresponding ketone or aldehyde to the compound (5) under the reduction conditions. As the reduction conditions, catalytic reduction can be used (see R. K. Clark Jr. and M. Flyfelder, ANTIBIOTICS AND CHEMOTHERAPY, 7, 483 (1957)). The catalyst usable therefore may be platinum catalysts such as platinum sponge, platinum asbestos, platinum black, platinum oxide and colloidal platinum; palladium catalysts such as palladium sponge, palladium black, palladium oxide, palladium on barium sulfate, palladium on barium carbonate, palladium on activated carbon, colloidal palladium and palladium on silica gel; reduced nickel, nickel, oxide, Raney nickel and Urushibara nickel, particularly preferable being palladium black, palladium carbon, and Raney nickel. The reaction can be preferably carried out in alcohols (such as methanol and ethanol), ethers (such as tetrahydrofuran and dimethoxyethane) and aqueous mixtures thereof, in the presence of hydrogen gas, under ice cooling to 80 ° C, preferably around room temperature.

As the reduction condition, reduction by use of a metal hydride may also be used. As the metal hydride, sodium borohydride and sodium cyanoborohydride are preferred.

The reaction is carried out preferably in a solvent such as alcohols (e.g. methanol and ethanol), ethers (e.g. tetrahydrofuran and dimethoxyethane), nitriles (e.g. acetonitrile) and aqueous mixtures thereof, more preferably while maintaining the pH of the reaction mixture at neutral to weakly acidic (pH 3 to 6), and it is preferable for control of the pH, to add a buffer solution or mineral acid (such as hydrochloric acid), an organic acid (such as acetic acid) or an aqueous solution thereof.

The amount of the metal hydride used is varied, depending on the carbonyl compound used, but it is a slight excess over to 100 times the theoretical amount, preferably a slight excess to 10 times, thereof, and it is added suitably with the progress of the reaction.

The reaction is carried out at -20 ° C to 80 ° C, preferably at 0 ° C to 30 ° C.

The compound (I) can also be synthesized by allowing the compound (5) to react with, for example, corresponding alkyl halide, an ester, or trioxonium salt, in the presence of a base.

Examples of the bases include sodium hydroxide, potassium hydroxide, sodium hydrogen carbonate, potassium carbonate, butyl lithium, phenyl lithium and sodium hydride.

Examples of the halogen atoms in the halide include chlorine, bromine and iodine, particularly preferably iodine.

Examples of the esters are sulfate esters.

Typical examples of the trioxonium salts are trimethyloxonium fluoroborate, and triethyloxonium fluoroborate.

The reaction reagent is used in an amount of 1 to 100 mol equivalent, preferably 2 to 25 mol equivalent per one mol of the starting compound.

The solvent to be used in the reaction include, for example, haloganated hydrocarbones (such as chloroform and dichloromethane), ether, (such as ethyl ether and tetrahydrofuran), esters (such as ethyl acetate), and alcohols (such as methanol and ethanol).

The reaction is carried out under ice cooling (0 ° C) to the boiling point of the solvent (100 ° C), preferably at room temperature (15 to 25 ° C) to 80 ° C.

The reaction time is 2 to 48 hours.

By subjecting the foregoing compound (5), in which $R^g$ is a group of the formula

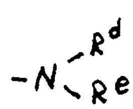

(wherein $R^d$ and $R^e$ are as defined above), pyrrolidino, or piperidino as the starting compound to N-alkylation, N-alkenylation or N-alkynylation reaction, a compound (I) can be prepared, wherein $R^a$ in the compound (I) is the formula

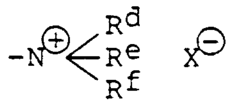

in which $R^d$, $R^e$, $R^f$ and $X^{\ominus}$ have the same meanings as defined above.

As the reagent employable in the reaction, there can be mentioned, for example, the corresponding alkyl, alkenyl or alkynyl halide, ester or trioxonium salt.

Examples of the halogen atoms in the halide include chlorine, bromine and iodine, particularly preferably iodine.

Examples of the esters include a sulfate esters.

Typical examples of the trioxonium salts include trimethyloxonium fluoroborate and triethyloxonium fluorobrate.

The reaction reagent is used in an amount of 1 to 100 mol equivalent, preferably 2 to 25 mol equivalent per one mol of the starting compound.

The solvent to be used in the reaction include, for example, haloganated hydrocarbons (such as chloroform and dichloromethane), ether (such as ethyl ether and tetrahydrofuran), ester (such as ethyl acetate), and alcohols (such as methanol and ethanol).

The reaction is carried out under ice cooling (0 ° C) to the boiling point of the solvent (100 ° C), preferably at room temperature (15 to 25 ° C) to 80 ° C.

The reaction time is 2 hours to 1 week.

From the reaction mixture, after carrying out optionally washing with aqueous sodium carbonate, or aqueous sodium chloride, drying or concentration, the product can be isolated by filtration of the precipitate formed by addition of an ether or the like to obtain the desired product as a salt of the anion from the reagent used in quaternarization.

When the reaction mixture is subjected to column chromatography with silica gel or ion exchange resin, using, for example, a mixture of chloroform-metanol added with conc. aqueous ammonia, a compound with hydroxide ion ($OH^-$) as the anion can be obtained.

The anions of the compound thus obtained can be exchanged with other anions by a conventional means.

The starting compound (5) used in the above reaction can be prepared by treating, for example, de(N-methyl)erythromycin [E. H. Flynn, et al., Journal of the American Chemical Society, 77, 3104 (1955), Japanese Laid-open Patent Application No. 9129/1972] under acidic conditions.

The compound (1) thus obtained can be isolated and purified in per se already known methods, for example, concentration, pH alteration, solvent-transformation, solvent extraction, lyophilization, crystallization, recrystallization, distillation, and chromatography.

The compound (1) may form a salt with an acid. Examples of such acids include organic acids (for example, ethylsuccinic acid, glycopeptonic acid, stearic acid, propionic acid, lactobionic acid, oxalic acid, maleic acid, fumaric acid, succinic acid, citric acid, lactic acid, trifluoroacetic acid, acetic acid, methanesulfonic acid, paratoluenesulfonic acid, and benzenesulfonic acid) and mineral acids (for example, sulfuric acid, hydrochloric acid, hydriodic acid, hydrobromic acid, phosphoric acid, and nitric acid.

The starting compound in the process of the present invention can be prepared, for example, by methods reported by V. C. Stephens et at., Antibiotics Annual, 1958-1959, 346; C. W. Pettinga et al., Journal of the American Chemical Society, 76, 569, 1954; or similar methods or by subjecting the compounds described in the above-mentioned references to the above-described process of the present invention or the conventional known means.

The compound (1) or its salt of the present invention has an excellent effect on stimulating the gastrointestinal contraction. Also, no lethal case was observed when the compound (55) described later is orally administered to mouse at a dose of 2300 mg/kg. Accordingly, the compound (1) of the present invention may be considered to be low in toxicity.

Thus, the compound (1) or its salt shows an excellent effect for stimulating the gastrointestinal contraction with a low toxicity, and can therefore be utilized as a gastrointestinal contractive motion stimulant for the therapy of digestive malfunctions (nausea, vomiting, want of apetite in gastritis, gastric ulcer, duodenal ulcer, diseases in gallbladder and biliary tract in mammals (mouse, rat, dog, cow, pig, man).

The compound (1) of the present invention can be administered orally or non-orally to the above-mentioned mammals. The daily dose thereof, in case of oral administration, is 0.001 - 100 mg/kg in the form of the compound (1) , and, in case of non-oral administration, for example, intravenous injection, is 0.0001 - 10 mg/kg.

For example, a compound (32), to be explained later, induces an extremely strong contraction in the stomach, duodenum and small intestine in dog, by an intravenous administration of a dose of 1.0 mg/kg. The contractive motion is comparable to the strongest one in the gastrointestinal contraction in normal dog. Also a reduced dose in the order of 3μg/kg induces, instead of continuous strong contraction, a contractive motion of an identical pattern with that of the natural contraction into digestive state.

The compound (1) of the present invention can be administered in various forms of preparations, such as emulsion, hydrated mixture, tablet, solution, powder, granules, capsule, and pill, containing additional components. The additional components include pharmacologically permitted vehicle, disintegrator, lubricant, binder, dispersant, and plasticizer. As examples of the additional components, the examples of vehicles are lactose, glucose and white sugar; those of disintegrators are starch, sodium alginate, agar powder and carboxymethyl cellulose calcium; those of lubricants are magnesium stearate, talc and liquid paraffin; those of binders are syrup, gelatin solution, ethanol and polyvinyl alcohol; those of dispersants are methyl cellulose, ethyl cellulose and shellac; and those of plasticizers are glycerin and starch.

These preparations can be obtained by methods usually employed in the field of pharmaceutics.

## PREFERRED EMBODIMENTS OF THE INVENTION

The gastrointestinal motion was measured in the following manner (Z. Itoh, Nihon Heikatsu-kin Gakkai Zasshi, 13, 33, 1976). A mongrel adult dog of a weight of 10-15 kg was anesthetized and the abdominal cavity was opened, and force transducers were chronically sutured on the serosa of the gastrointestinal tract such as gastric body, gastric antrum, duodenum, jejunum, in directions capable of recording the contraction of circular muscles. The lead wires were extracted from the back and fixed to the skin. The experiment could be started 5 days after recovery from such operation, and a dog prepared in this manner can be subjected to experiments for 6 months. The force transducer, when subjected to a bending stress by the contraction of the gastrointestinal tract where the transducer is sutured, allows to record the wave form corresponding to the applied force, on a pen-recording oscillograph, and this method allows to measure the nature and magnitude of the contraction.

The dog was maintained in an experimental cage, and the wave form of contraction can be immediately recorded by connecting the wires of the transducer to the polygraph. The gastrointestinal contractive motion can be divided, from the pattern of contraction, into the one in a period after food intake and it in an interdigestive period. The experiments were conducted, during the interdigestive period and in an inactive period lacking the contraction in the stomach. The sample was injected through a silicone tube placed in advance in the superior vena cava over 10 seconds.

The sample was dissolved in physiological saline to a total volume of 10 ml and was slowly injected intravenously for a period of 10 seconds.

The gastrointestinal motor stimulating activity (GMSA) is summarized in Table 1.

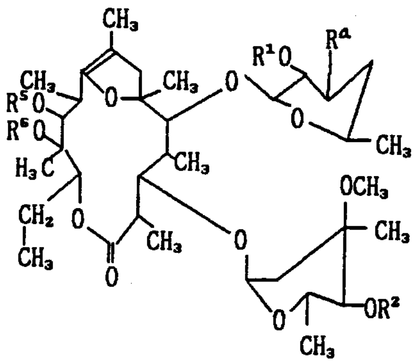

**Table 1**

| Compound No. | $R^1$ | $R^2$ | $R^5$ | $R^6$ | $R^4$ | GMSA |
|---|---|---|---|---|---|---|
| (5) | H | $CH_3CO$ | H | H | 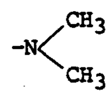 | + |
| (9) | H | CHO | H | H | " | ++ |
| (14) | H | $CH_3CO$ | $CH_3CO$ | H | " | + |
| (25) | H | CHO | $CH_3SO_2$ | H | " | +++ |
| (26) | H | $CH_3SO_2$ | $CH_2SO_2$ | H | " | ++ |
| (28) | $CH_3CH_2CH_2CO$ | H | H | H | " | ++ |
| (30) | H | $CH_3SO_2$ | H | H | " | + |
| (32) | H | H | $CH_3CO$ | $CH_3CO$ | " | +++ |
| (33) | H | H | $CH_3CH_2CO$ | $CH_3CH_2CO$ | " | ++ |
| (36) | H | H | >= S | | 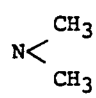 | + |
| (37) | H | H | >S = O | | " | + |

| Compound No. | $R^1$ | $R^2$ | $R^5$ | $R^6$ | $R^4$ | GMSA |
|---|---|---|---|---|---|---|
| (39) | H | H | >B-Ph | | " | ++ |
| (47) | H | H | H | $CH_3SCH_2$ | " | + |
| (50) | H | H | $CH_3CO$ | H | " | ++ |
| (51) | H | H | $CH_3CH_2CO$ | H | " | ++ |
| (52) | H | H | $CH_3CH_2CH_2CO$ | H | " | ++ |
| (54) | H | H | 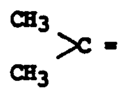 | | " | + |
| (55) | H | H | H | H | 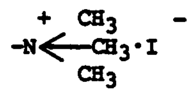 | +++ |
| (56) | H | H | $CH_3CO$ | $CH_3CO$ | " | +++ |
| (58) | H | H | $CH_3SO_2$ | H | " | +++ |
| (59) | H | CHO | $CH_3SO_2$ | H | " | +++ |
| (60) | H | H | H | H | 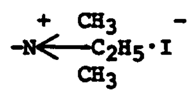 | +++ |
| (62) | $CH_3CO$ | H | H | H | 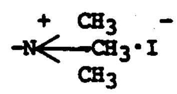 | ++ |
| (73) | H | H | $CH_3(CH_2)_3CO$ | H | 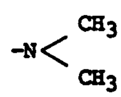 | + |
| (74) | H | H | $CH_3(CH_2)_4CO$ | H | " | ++ |
| (75) | H | H | H | H | 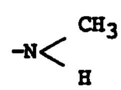 | +++ |
| (77) | H | H | H | H | 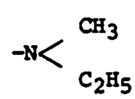 | +++ |
| (79) | H | H | H | H | 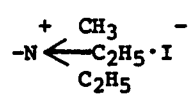 | +++ |

| Compound No. | $R^1$ | $R^2$ | $R^5$ | $R^6$ | $R^4$ | GMSA |
|---|---|---|---|---|---|---|
| (80) | H | H | H | H | 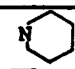 | + |
| (81) | H | H | H | H | 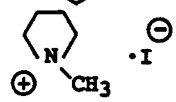 | +++ |
| (82) | H | H | H | H | 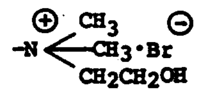 | +++ |
| (83) | H | H | H | H | 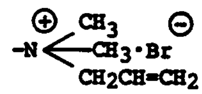 | +++ |
| (89) | H | H | H | H | 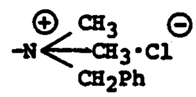 | +++ |
| (90) | H | H | $SO_2CH_3$ | H | 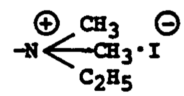 | +++ |
| (91) | H | H | $SO_2CH_3$ | H | 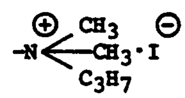 | +++ |
| (92) | H | H | H | H | 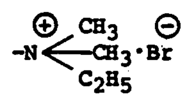 | +++ |
| (93) | H | H | H | H | 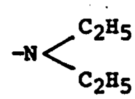 | ++ |
| (94) | H | H | H | H | 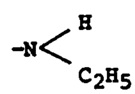 | +++ |
| (95) | H | H | H | H | 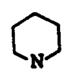 | + |
| (96) | H | H | H | H | 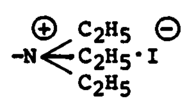 | ++ |
| (97) | H | H | H | H | 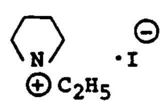 | + |

| Compound No. | $R^1$ | $R^2$ | $R^5$ | $R^6$ | $R^4$ | GMSA |
|---|---|---|---|---|---|---|
| (98) | H | H | H | H | 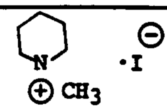 | +++ |
| (99) | H | H | H | H | 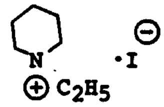 | + |
| (100) | H | H | H | H | 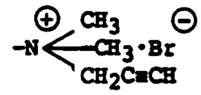 | ++++ |
| (101) | H | H | $COCH_3$ | $COCH_3$ | 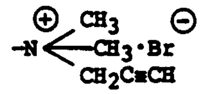 | ++++ |
| (102) | H | H | $CH_3$ | H | $-N(CH_3)_2$ | ++ |
| (104) | H | H | $COC_2H_5$ | $COCH_3$ | $-N(CH_3)_2$ | ++ |
| (105) | H | H | $COC_3H_7$ | $COCH_3$ | $-N(CH_3)_2$ | ++ |
| (106) | H | H | $COCH_3$ | $COCH_3$ | 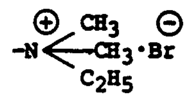 | +++ |
| (107) | H | H | H | H | 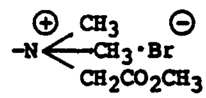 | ++ |
| (108) | H | H | H | H | 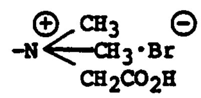 | ++ |
| (109) | H | H | H | H | 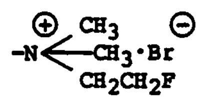 | +++ |
| (110) | H | H | H | H | 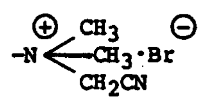 | +++ |
| (113) | H | H | H | H | 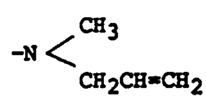 | ++ |
| (115) | H | H | H | H | 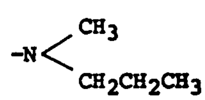 | +++ |

| Compound No. | $R^1$ | $R^2$ | $R^5$ | $R^6$ | $R^4$ | GMSA |
|---|---|---|---|---|---|---|
| (120) | H | H | H | H | 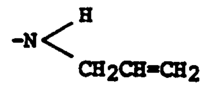 | ++ |
| (124) | H | H | H | H | 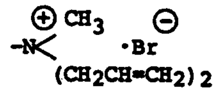 | ++ |
| (125) | H | H | H | H | 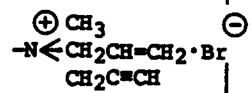 | +++ |
| (126) | H | H | H | H | 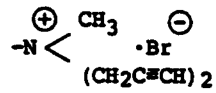 | +++ |
| (136) | H | H | H | H | 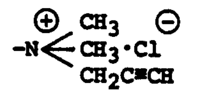 | + |

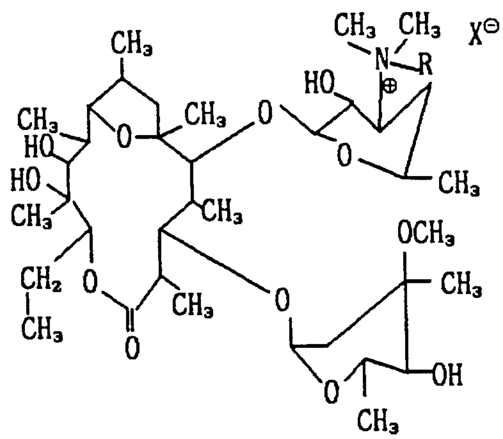

Table 1'

| Compound No. | R | $X^{\ominus}$ | GMSA |
| --- | --- | --- | --- |
| (85) | $CH_3$ | $I^{\ominus}$ | +++ |
| (86) | $C_2H_5$ | $I^{\ominus}$ | +++ |
| (87) | $(CH_2)_2CH_3$ | $I^{\ominus}$ | ++ |
| (88) | $(CH_2)_3CH_3$ | $I^{\ominus}$ | +++ |
| (111) | $CH_3CH=CH_2$ | $Br^{\ominus}$ | +++ |
| (112) | $CH_2C≡CH$ | $Br^{\ominus}$ | +++ |

In Table 1 and and Table 1', ++++, +++, ++ and + of GMSA indicate that the minimum effective concentration required for inducing a gastrointestinal contractive motion in dog, comparable to the spontaneous one in the interdigestive period is in a range of 0.01 - 0.1 µg/kg, 0.1 - 10 µg/kg, 10 -30 µg/kg and 30 - 50 µg/kg, respectively.

*) The numbers of compounds correspond to those in the examples.

Example 1

250 mg of 2'-O-acetyl-8,9-anhydroerythromycin A 6,9-hemiketal (compound 1) (V. C Stephens et al., Antibiotics Annual, 1958-1959, 346) was dissolved in 2 ml of dry pyridine, and 0.3 ml of acetyl chloride was added at a time at room temperature and under vigorous agitation. After agitation for 15 minutes, 30 ml of ethyl acetate was added. The obtained ethyl acetate solution was washed with the saturated aqueous solution of sodium hydrogen carbonate, then with the saturated aqueous solution of sodium chloride, then dried with anhydrous sodium sulfate, and the solvent was distilled off to obtain a crude product.

The crude product was purified by silica gel column chromatography (developed with a 50 : 1 : 0.01 mixed solvent of chloroform, methanol and concentrated aqueous ammonia) to obtain 100 mg (yield 38%) of 2',4''-di-O-acetyl-8,9-anhydroerythromycin A 6,9-hemiketal (compound 2) as white powder.

Example 2

303 mg of the compound 1, 0.3 ml of propionyl chloride and 2 ml of dry pyridine were employed in the process of Example 1 to obtain 143 mg (yield 44%) of 2'-O-acetyl-4''-O-propionyl-8,9-anhydroerythromycin A 6,9-hemiketal (compound 3) as white powder.

Example 3

303 mg of the compound 1 was dissolved in 1 ml of dry pyridine and agitated overnight with 0.07 ml of benzoyl chloride. Thereafter the same process as in Example 1 was adopted to obtain 127 mg (yield 37%) of 2'-O-acetyl-4''-O-benzoyl-8,9-anhydroerythromycin A 6,9-hemiketal (compound 4) in white powder.

Example 4

100 mg of the compound 2 obtained in Example 1 was dissolved in 2 ml of methanol, and agitated overnight at room temperature. A crude product, obtained by distilling off the solvent, was purified by silica gel column chromatography (developed by a 50 : 1 : 0.01 mixture of chloroform, methanol and concentrated aqueous ammonia) to obtain 35 mg (yield 37%) of 4''-O-acetyl-8,9-anhydroerythromycin A 6,9-hemiketal (compound 5) in white powder.

Example 5

143 mg of the compound 3 obtained in Example 2 was dissolved in 2 ml of methanol, and processed in the same manner as in Example 4 to obtain 83 mg (yield 61%) of 4''-O-propionyl-8,9-anhydroerythromycin A 6,9-hemiketal (compound 6) in white powder.

Example 6

127 mg of the compound 4 obtained in Example 3 was dissolved in 2 ml of methanol, and was processed in the same manner as in Example 4 to obtain 92 mg (yield 77%) of 4''-O-benzoyl-8,9-anhydroerythromycin A 6,9-hemiketal (compound 7) in white powder.

Example 7

59 mg of 2'-O-acetyl-4''-O-formyl-8,9-anhydroerythromycin A 6,9-hemiketal (compound 8) (J. Tadanier et al., Journal of Organic Chemistry, 39, 2495, 1974) was dissolved in 1 ml of methanol, and was processed in the same manner as in Example 4 to obtain 29 mg of 4''-O-formyl-8,9-anhydroerythromycin A 6,9-hemiketal (compound 9) in white powder.

Example 8

303 mg of the compound 1 was dissolved in 2 ml of dry pyridine, and 0.3 ml of crotonyl chloride was added at a time under vigorous agitation at room temperature. After agitation for 15 minutes, 30 ml of ethyl acetate was added. The obtained ethyl acetate solution was washed with the saturated aqueous solution of sodium hydrogen carbonate and with the saturated aqueous solution of sodium chloride, then dried with anhydrous sodium sulfate and the solvent was distilled off.

The obtained residue was dissolved in 2 ml of methanol, and agitated overnight at room temperature. A crude product obtained by removing the solvent by distillation was purified by silica gel column chromatography (developed with a 50 : 1 : 0.01 mixture of chloroform, methanol and concentrated aqueous ammonia) to obtain 31 mg (yield 10%) of 4''-O-crotonyl-8,9-anhydroerythromycin A 6,9-hemiketal (compound 10) in white powder.

Example 9

205 mg of the compound 1, 2 ml of dry pyridine and 0.3 ml of butyryl chloride were processed in the same manner as in Example 8 to obtain 18 mg (yield 8%) of 4''-O-butyryl-8,9-anhydroerythromycin A 6,9-hemiketal (compound 11) in white powder.

Example 10

303 mg of the compound 1, 2 ml of dry pyridine and 0.4 ml of isovaleryl chloride were processed in the same manner as in Example 8 to obtain 40 mg (yield 12%) of 4''-O-isovaleryl-8,9-anhydroerythromycin A 6,9-hemiketal (compound 12) in white powder.

Example 11

303 mg of the compound 1, 2 ml of dry pyridine, and 0.4 ml. of ethylmalonyl chloride were processed in the same manner as in Example 8 to obtain 40 mg (yield 12%) of 4''-O-ethylmalonyl-8,9-anhydroerythromycin A 6,9-hemiketal (compound 13) in white powder.

Example 12

205 mg of the compound 1 was dissolved in 1 ml of dry pyridine, and agitated for 4 days at room temperature with 0.25 ml of acetic anhydride. The mixture was diluted with 30 ml of ethyl acetate, then washed with the saturated aqueous solution of sodium hydrogen carbonate and the saturated aqueous solution of sodium chloride, and dried with anhydrous sodium sulfate. The residue, obtained by distilling off the solvent, was dissolved in 1 ml of methanol and agitated overnight at room temperature. A crude product, obtained by removing the solvent by distillation, was purified with silica gel column chromatography (developed with a 50 : 1 : 0.01 mixture of chloroform, methanol and concentrated aqueous ammonia water to obtain 129 mg (yield 60%) of 11,4''-di-O-acetyl-8,9-anhydroerythromycin A 6,9-hemiketal (compound 14) in white powder.

Example 13

205 mg of the compound 1, 1 ml of dry pyridine and 0.25 ml of propionic anhydride were processed in the same manner as in Example 12 to obtaine 105 mg (yield 47%) of 11,4''-di-O-propionyl-8,9-anhydroerythromycin A 6,9-hemiketal (compound 15) in white powder.

Example 14

205 mg of the compound 1 was dissolved in 1 ml of dry pyridine, and agitated with 0.5 ml of butyric anhydride for 7 days at room temperature. It was thereafter processed in the same manner as in Example 12 to obtain 113 mg (yield 40%) of 11,4''-di-O-butyryl-8,9-anhydroerythromycin A 6,9-hemiketal (compound 16) in white powder.

Example 15

205 mg of the compound 1 was dissolved in 1 ml of dry pyridine, and agitated with 0.5 ml of benzoyl chloride for 3 days at room temperature. The mixture was then processed in the same manner as in Example 12 to obtain 107 mg (yield 35%) of 11,4''-di-O-benzoylerythromycin A 6,9-hemiketal (compound 17) in white powder.

Example 16

184 mg of the compound 1 was dissolved in 2 ml of dry pyridine, and agitated with 440 mg of benzylsulfonyl chloride for 5 hours at room temperature. The mixture was then diluted with 30 ml of ethyl acetate, washed with the saturated aqueous solution of sodium hydrogen carbonate and with the saturated aqueous solution of sodium chloride, and dried with anhydrous sodium sulfate. The residue obtained by removing the solvent by distillation was dissolved in 2 ml of methanol, and agitated overnight at room temperature. A crude product obtained by removing the solvent by distillation was purified by silica gel column chromatography (developed by a 50 : 1 : 0.01 mixture of chloroform, methanol and concentrated aqueous ammonia ) to obtain 127 mg (yield 51%) of 11,4'-di-O-benzylsulfonyl-8,9-anhydroerythromycin A 6,9-hemiketal (compound 18) in white powder.

Example 17

227 mg of the compound 1 was dissolved in 2 ml of dry pyridine, and agitated with 527 mg of paratoluenesulfonyl chloride for 2 days at 50 °C. The mixture was processed in the same manner as in Example 16 to obtain 81 mg (yield 26%) of 11,4''-di-O-paratoluenesulfonyl-8,9-anhydroerythromycin A 6,9-hemiketal (compound 19) in white powder.

Example 18

9 g of 8,9-anhydroerythromycin A 6,9-hemiketal cyclic-11,12 carbonate (compound 20) (W. Slawinski et al., Journal of the Royal Netherlands Chemical Society, 94, 236, 1975) was dissolved in 100 ml of chloroform and agitated with 4 ml of pyridine and 3 ml of acetic anhydride for 45 minutes at room temperature. This reaction solution was washed with the saturated aqueous solution of sodium hydrogen carbonate and with the saturated aqueous solution of sodium chloride, then dried with anhydrous sodium sulfate, and the solvent was distilled off to obtain white powder of 2'-O-acetyl-8,9-anhydroerythromycin A 6,9-hemiketal cyclic-11,12-carbonate (compound 21) quantatively in substantially pure state.

Example 19

235 mg of the compound 21 obtained in Example 18 was dissolved in 1 ml of dry pyridine, and agitated with 0.5 ml of butyric anhydride for 2 days at room temperature. The reaction solution was diluted with 30 ml of ethyl acetate, then washed with the saturated aqueous solution of sodium hydrogen carbonate and with the saturated aqueous solution of sodium chloride, dried with anhydrous sodium sulfate and the solvent was distilled off to obtain a crude product.

The crude product was purified by silica gel column chromatography (developed by a 50 : 1 : 0.01 mixture of chloroform, methanol and concentrated aqueous ammonia) to obtain 78 mg (yield 31%) of 2'-O-acetyl-4''-O-butyryl-8,9-anhydroerythromycin A 6,9-hemiketal-cyclic-11,12-carbonate (compound 22) in white powder.

Example 20

59 mg of the compound 22 obtained in Example 19 was dissolved in 1 ml of methanol, and agitated overnight at room temperature. A crude product obtained by removing the solvent by distillation was purified by silica gel column chromatography (developed by a 50 : 1 : 0.01 mixture of chloroform, methanol and concentrated aqueous ammonia) to obtain 40 mg (yield 72%) of 4''-O-butyryl-8,9-anhydroerythromycin A 6,9-hemiketal-cyclic-11,12-carbonate (compound 23) in white powder.

Example 21

79 mg of 11-O-methanesulfonyl-2'-O-acetyl-4''-O-formyl-8,9-anhydroerythromycin A 6,9-hemiketal (compound 24) (J. Tadeniel et al., Journal of Organic Chemistry, 39, 2495, 1974) was dissolved in 1 ml of methanol, and agitated overnight at room temperature. A crude product obtained by removing the solvent by distillation was purified by silica gel column chromatography (developed by a 50 : 1 : 0.01 mixture of chloroform, methanol and concentrated aqueous ammonia) to obtain 40 mg (yield 52%) of 11-O-methanesulfonyl-4''-O-formyl-8,9-anhydroerythromycin A 6,9-hemiketal (compound 25) in white powder.

Example 22

150 mg of the compound 1 was dissolved in 2 ml of dry pyridine, and 46 $\mu$l of methanesulfonyl chloride was added thereto under agitation and under cooling with ice. After completion of the addition, agitation was continued for 1 hour under cooling with ice, and then for 2 hours at room temperature. The same process as in Example 16 was thereafter conducted to obtain 123 mg (yield 78%) of 11,4''-di-O-methanesulfonyl-8,9-anhydroerythromycin A 6,9-hemiketal (compound 26) in which powder.

Low mass (SIMS) m/e 872 $(M + H)^+$

The structure, specific rotatory power and NMR spectrum values of the compounds obtained in Example 1 to 22 are summarized in Tables 2 and 3.

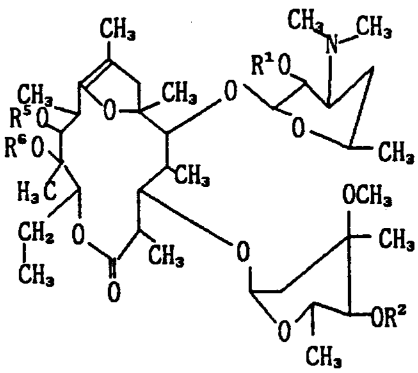

Table 2

| Compound No. | $R^1$ | $R^2$ | $R^5$ | $R^6$ | $[\alpha]^{24}_{D}$ (c 1.0, $CHCl_3$) |
|---|---|---|---|---|---|
| 2 | $CH_3CO$ | $CH_3CO$ | H | H | -44.4° |
| 3 | $CH_3CO$ | $CH_3CH_2CO$ | H | H | -46.0° (c 0.5) |
| 4 | $CH_3CO$ | PhCO | H | H | -56.2° |
| 5 | H | $CH_3CO$ | H | H | -43.4° |
| 6 | H | $CH_3CH_2CO$ | H | H | -38.0° |
| 7 | H | PhCO | H | H | -59.2° |
| 9 | H | CHO | H | H | -41.8° |
| 10 | H | 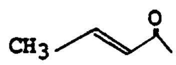 | H | H | -43.4° |
| 11 | H | $CH_3CH_2CH_2CO$ | H | H | -33.4° |
| 12 | H | 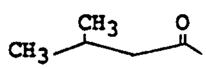 | H | H | -35.0° |
| 13 | H | 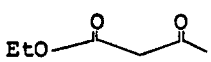 | H | H | -34.8° |
| 14 | H | $CH_3CO$ | $CH_3CO$ | H | -21.4° |
| 15 | H | $CH_3CH_2CO$ | $CH_3CH_2CO$ | H | -25.6° |
| 16 | H | $CH_3CH_2CH_2CO$ | $CH_3CH_2CH_2CO$ | H | -25.4° |

| Compound No. | $R^1$ | $R^2$ | $R^5$ | $R^6$ | $[\alpha]_D^{24}$ (c 1.0, $CHCl_3$) |
|---|---|---|---|---|---|
| 17 | H | PhCO | PhCO | H | -50.0° |
| 18 | H | $PhCH_2SO_2$ | $PHCH_2SO_2$ | H | -37.6° |
| 19 | H | 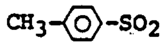 | 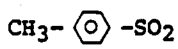 | H | -9.0° |
| 21 | $CH_3CO$ | H | 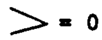 | | -33.6° |
| 22 | $CH_3CO$ | $CH_3CH_2CH_2CO$ | 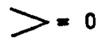 | | -41.2° |
| 23 | H | $CH_3CH_2CH_2CO$ | 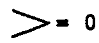 | | -42.6° |
| 25 | H | CHO | $CH_3SO_2$ | H | -32.4° |
| 26 | H | $CH_3SO_2$ | $CH_3SO_2$ | H | -34.8° |

In Table 2, Ph is phenyl and Et is ethyl.

The numbers of compounds corresponds to those in the Examples.

Table 3 $^{3}H$-NMR peak ( $\delta$ value ppm, solvent $CDCl_3$)

| Compound No. | 3"-OMe (s, 3H) | 3'-$NMe_2$ (s, 6H) | 8-Me (s, 3H) | Others |
|---|---|---|---|---|
| 2 | 3.35 | 2.28 | 1.55 | 2'-OAc: 2.05 (s, 3H), 4"-OAc 2.10 (s, 3H) |
| 3 | 3.35 | 2.28 | 1.55 | Ac: 2.04 (s, 3H) |
| 4 | 3.34 | 2.33 | 1.55 | Ac: 2.06 (s, 3H) ph: 7.45 (m, 3H) and 8.00 (m, 2H) |
| 5 | 3.33 | 2.31 | 1.57 | Ac: 2.10 (s, 3H) |
| 6 | 3.32 | 2.32 | 1.57 | |
| 7 | 3.40 | 2.35 | 1.56 | Ph: 7.49 (m, 3H) and 8.01 (m, 2H) |
| 9 | 3.33 | 2.30 | 1.56 | CHO: 8.19 (s, 1H) |
| 10 | 3.33 | 2.31 | 1.56 | 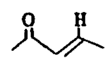 $\underline{CH_3}$: 1.90 (d, 3H J=7Hz), 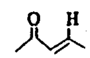 $\underline{CH_3}$: 7.00 (m, 1H)<br>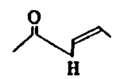 $CH_3$: 5.85 (d, 1H J=16Hz) |
| 11 | 3.32 | 2.29 | 1.55 | |
| 12 | 3.33 | 2.31 | 1.57 | |
| 13 | 3.32 | 2.30 | 1.57 | 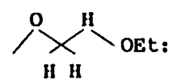 OEt: 3.38 (s, 2H), $O\underline{CH_2}CH_3$: 4.19 (q, 1H J = 8 Hz) |

| Compound No. | 3"-OMe (s, 3H) | 3'-$NMe_2$ (s, 6H) | 8-Me (s, 3H) | Others |
|---|---|---|---|---|
| 14 | 3.32 | 2.30 | 1.57 | 4"-OAc: 2.09 (s, 3H), 11-OAc: 2.12 (s, 3H) |
| 15 | 3.31 | 2.28 | 1.57 | |
| 16 | 3.32 | 2.35 | 1.57 | |
| 17 | 3.41 | 2.35 | 1.56 | Ph: 7.49 (m, 6H) and 8.03 (m, 4H) |
| 18 | 3.33 | 2.28 | 1.53 | $SO_2$-$CH_2$-Ph: 4.34 & 4.52 respectively (s, 2H), Ph: 7.40 (s, 10H) |
| 19 | 3.30 | 2.29 | 1.52 | 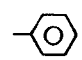 $CH_3$: 2.44 (s, 6H), Ph: 7.30 & 7.80 respectively (m, 4H) |
| 21 | 3.46 | 2.36 | 1.58 | Ac: 2.05 (s, 3H) |
| 22 | 3.34 | 2.28 | 1.58 | Ac: 2.05 (s, 3H) |
| 23 | 3.28 | 2.26 | 1.57 | |
| 25 | 3.34 | 2.33 | 1.58 | $SO_2CH_3$: 3.18 (s, 3H), CHO: 8.10 (s, 1H) |
| 26 | 3.32 | 2.27 | 1.56 | 4"-$SO_2CH_3$: 3.04 (s, 3H), 11-$SO_2CH_2$: 3.15 (s, 3H) |

In Table 3, Ac is acetyl and Phe is phenyl.

Example 23

200 mg of 8,9-anhydroerythromycin A 6,9-hemiketal (compound 27) (V. C. Stephens et al., Antibiotics Annual, 1958-1959, 346) was dissolved in 3.4 ml of $CHCl_3$, then added with 0.22 ml of anhydrous pyridine and 0.34 ml of butyric anhydride, and was allowed to stand for 20 minutes at room temperature. The

reaction solution was diluted with 20 ml of $CHCl_3$, and washed with 20 ml of the saturated aqueous solution of sodium hydrogen carbonate and 20 ml of water. The $CHCl_3$ layer was dried with anhydrous sodium sulfate, and concentrated under a reduced pressure to obtain a colorless glass-like substance. Said substance was purified by silica gel column chromatography, utilizing a developing mixed solvent of $CHCl_3$ : $CH_3OH$ : conc. $NH_4OH$ = 40 : 1 0.01, to obtain 209 mg (yield 95.2%) of 2'-O-butyryl-8,9-anhydroerythromycin A 6,9-hemiketal (compound 28) in white powder.

Rf value : 0.36 ($CHCl_3$ : $CH_3OH$ : conc. $NH_4OH$ = 10 : 1 : 0.01) Carrier : silica gel (Merck, West Germany), High mass : 785.4936 (calcd. for $C_{41}H_{71}NO_{13}$: 785.4921).

The same carrier was employed also in the thin layer chromatography in the following Examples.

## Example 24

200 mg of 2'-O-acetyl-8,9-anhydroerythromycin A 6,9-hemiketal (compound 29) (V. C. Stephens et al., Antibiotics Annual, 1958-1959, 346) was dissolved in 4 ml of anhydrous pyridine, and added with 0.12 ml of methanesulfonyl chloride under cooling with ice. After 30 minutes, the same process as that for producing the compound 28 was conducted to obtain a colorless glass-like substance. This substance was dissolved, without purification in 8 ml of methanol and was let to stand at room temperature. After one day, the reaction solution was concentrated under reduced pressure to obtain a colorless glass-like substance. This substance was purified by silica gel column chromatography, utilizing a mixed developing solvent of $CHCl_3$ : $CH_3OH$ : conc. $NH_4OH$ = 30 : 1 : 0.01, to obtain 116 mg (yield 52.3%) of 4''-O-methanesulfonyl-8,9-anhydroerythromycin A 6,9-hemiketal (compound 30) in white powder.

Rf value : 0.20 ($CHCl_3$ : $CH_3OH$ : conc. $NH_4OH$ = 10 : 1 : 0.01), high mass : 793.427 (calcd. for $C_{38}H_{67}NO_{14}S$ : 793.427).

## Example 25

300 mg of 2'-O-acetyl-4''-O-formyl-8,9-anhydroerythromycin A 6,9-hemiketal (compound 31) (=compound 8) (Journal of The Chemical Society, 39, 2495, 1974) was dissolved in 8.1 ml of $CHCl_3$, and heated under reflux with 5 mg of 4-dimethylaminopyridine, 15 ml of triethylamine and 1.2 ml of acetic anhydride. The reaction mixture was cooled to room temperature after 3 days, and the same process as that for obtaining the compound 28 was conducted to obtain a pale yellow glass-like substance. This substance was dissolved, without purification, in 12 ml of methanol, and heated under reflux. The solution was cooled to room temperature after 3 days and concentrated under reduced pressure to obtain a pale yellow glass-like substance. This substance was purified by silica gel column chromatography, utilizing a developing solvent system of $CHCl_3$ : $CH_3OH$ : conc. $NH_4OH$ = 50 : 1 : 0.01, to obtain 136 mg (yield 44.5%) of 11,12-di-O-acetyl-8,9-anhydroerythromycinA 6,9-hemiketal (compound 32) in white powder.

Rf value : 0.15 ($CHCl_3$ : $CH_3OH$ : conc. $NH_4OH$ = 10 : 1 : 0.01), low mass : $M^+$ 799, high mass : 799.4703 (calcd. for $C_{41}H_{69}NO_{14}$ : 799.4713).

## Example 26

300 mg of the compound 31 was dissolved in 8.1 ml of $CHCl_3$, then added with 5 mg of 4-dimethylaminopyridine, 2.2 ml of triethylamine and 2.2 ml of propionic anhydride, and processed in the same manner as in the preparation of the compound 32 to obtain 68 mg (yield 21.5%) of 11,12-di-O-propionyl-8,9-anhydroerythromycin A 6,9-hemiketal (compound 33) in white powder.

Rf value : 0.16 ($CHCl_3$ : $CH_3OH$ : conc. $NH_4OH$ = 10 : 1 : 0.01), high mass : 827.502 (calcd. for $C_{43}H_{73}NO_{14}$ : 827.502).

## Example 27

300 mg of the compound 31 was dissolved in 8.1 ml of $CHCl_3$, then added with 5 mg of 4-dimethylaminopyridine, 2.2 ml of triethylamine and 2.6 ml of butyric anhydride, and processed in the same manner as in the preparation of the compound 32 to obtain 141 mg (yield 43.2%) of 11,12-di-O-butyryl-8,9-anhydroerythromycin A 6,9-hemiketal (compound 34) in white powder.

Rf value : 0.18 ($CHCl_3$ : $CH_3OH$ : conc. $NH_4OH$ = 10 : 1 : 0.01), low mass : $M^+$ 855, high mass : 855.5343 (calcd. for $C_{45}H_{77}NO_{14}$ : 855.5339.

Example 28

1.0 g of the compound 31 was dissolved in 10 ml of toluene, and heated under reflux with 929 mg of thiocarbonyl diimidazole. The solution was cooled to room temperature after 4 hours and processed in the same manner as in the preparation of the compound 28 to obtain a yellow glass-like substance. The obtained glass-like substance was purified by silica gel column chromatography, utilizing a developing solvent system of $CHCl_3$ : $CH_3OH$ : conc. $NH_4OH$ = 100 : 1 : 0.01, to obtain 373 mg (yield 36.0%) of 2'-O-acetyl-4''-O-formyl-8,9-anhydroerythromycin A 6,9-hemiketal-cyclic-11,12-thiocarbonate (compound 35) in white powder.

Rf value : 0.45 ($CHCl_3$ : $CH_3OH$ : conc. $NH_4OH$ = 10 : 1 : 0.01), high mass: 827.4091 (calcd. for $C_{41}H_{65}NO_{14}S$ : 827.4121).

Example 29

100 mg of the compound 35 was dissolved in 4 ml of methanol and heated under reflux. After 3 days, the solution was cooled to room temperature, and concentrated under reduced pressure to obtain a colorless glass-like substance. The obtained glass-like substance was purified by silica gel column chromatography, utilizing a developing solvent system of $CHCl_3$ : $CH_3OH$ : conc. $NH_4OH$ = 50 : 1 : 0.01, to obtain 63 mg (yield 68.8%) of 8,9-anhydroerythromycin A 6,9-hemiketal-cyclic-11,12-thiocarbonate (compound 36) in white powder.

Rf value : 0.20 ($CHCl_3$ : $CH_3OH$ : conc. $NH_4OH$ = 10 : 1 : 0.01), high mass : 757.406 (calcd. for $C_{38}H_{63}NO_{12}S$ : 757.407).

Example 30

170 mg of the compound 27 was dissolved in 1,1 ml of methanol, then added with 213 mg of potassium carbonate and 27 1 of ethylene sulfite and agitated at room temperature. After 2 days, the solution was processed in the same manner as in the preparation of the compound 28 to obtain a colorless glass-like substance. The obtained glass-like substance was purified by silica gel column chromatography, utilizing a developing solvent system of $CHCl_3$ : $CH_3OH$ : conc. $NH_4OH$ = 10 : 1 : 0.01, to obtain 72 mg (yield 39.8%) of 8,9-anhydroerythromycin A 6,9-hemiketal-11,12-sulfite (compound 37) in white powder.

Rf. value : 0.09 ($CHCl_3$ : $CH_3OH$ : conc. $NH_4OH$ = 10 : 1 : 0.01), high mass : 761.401 (calcd. for $C_{37}H_{63}NO_{13}S$ : 761.401).

Example 31

200 mg of the compound 29 was dissolved in 10 ml of benzene, and heated under reflux with 32 mg of phenylboric acid. The solution was cooled to room temperature after 2 hours and processed in the same manner as in the preparation of the compound 28 to obtain 216 mg (yield 97.8%) of 2'-O-acetyl-8,9-anhydroerythromycin A 6,9-hemiketal-11,12-phenylboronate (compound 38) in white powder.

This compound was so pure that it did not require purification.

Rf value : 0.40 ($CHCl_3$ : $CH_3OH$ : conc. $NH_4OH$ = 10 : 1 : 0.01).

Example 32

216 mg of the compound 38 obtained in Example 31 was dissolved in 8.6 ml of methanol and was let to stand at room temperature. After 1 day, the solution was concentrated under a reduced pressure to obtain a colorless glass-like substance. The obtained glass-like substance was purified by silica gel column chromatography, utilizing a developing solvent system of $CHCl_3$ $CH_3OH$ : conc. $NH_4OH$ = 50 : 1 : 0.01, to obtain 199 mg (yield 97.0%) of 8,9-anhydroerythromycin A 6,9-hemiketal-11,12-phenylboronate (compound 39) in white powder.

Rf value : 0.40 ($CHCl_3$ : $CH_3OH$ : conc. $NH_4OH$ = 10 : 1 : 0.01).

Example 33

1.40 g of the compound 29 was dissolved in 14 ml of dry pyridine, then added with 1.1 ml of chlorotrimethylsilane and was let to stand at room temperature. After 2 hours, the solution was processed in the same manner as in the preparation of the compound 28 to obtain 1.50 g (yield 90.0%) of 2'-O-acetyl-11,4''-di-O-trimethylsilyl-8,9-anhydroerythromycin A 6,9-hemiketal (compound 40) as a colorless glass-like substance.

Rf value : 0.43 ($CHCl_3$ : $CH_3OH$ : conc. $NH_4OH$ = 10 : 1 : 0.01).

Example 34

750 mg of the compound 40 was dissolved in 3 ml of 1,2-dichloromethane, then added with 2.40 g of tribenzylamine and 0.72 ml of acetyl chloride under cooling, and, after 10 minutes, heated at 75 ° C under agitation. After 3 days, the solution was processed in the same manner as in the preparation of the compound 28 to obtain a pale yellow solid substance. The obtained solid substance was dissolved, without purification, in 30 ml of methanol and heated at 50 ° C. The solution was cooled to room temperature after 1 day and concentrated under reduced pressure to obtain a pale yellow solid substance. The obtained solid substance was purified by silica gel column chromatography, utilizing a developing solvent system of $CHCl_3$ : $CH_3OH$ : conc. $NH_4OH$ = 50 : 1 : 0.01, to obtain 163 mg (yield 25.9%) of 12-0-acetyl-8,9-anhydroerythromycin A 6,9-hemiketal (compound 41) as white powder.

Rf value : 0.15 ($CHCl_3$ : $CH_3OH$ : conc. $NH_4OH$ = 10 : 1 : 0.01), high mass : 757.460 (calcd. for $C_{39}H_{67}NO_{13}$ : 757.460)

Example 35

800 mg of the compound 40 was dissolved in 3.2 ml of 1,2-dichloroethane, then added with 2,56 g of tribenzylamine and 0.85 ml of propionyl chloride under cooling, and, after 10 minutes, heated at 75 ° C under agitation. After 3 days, the solution was processed in the same manner as in the preparation of the compound 30 to obtain 273 mg (yield 39.9%). of 12-O-propionyl-8,9-anhydroerythromycin A 6,9-hemiketal (compound 42) as white powder.

Rf value : 0.17 ($CHCl_3$ : $CH_3OH$ : conc. $NH_4OH$ = 10 : 1 : 0.01), high mass : 771.476 (calcd. for $C_{40}H_{69}NO_{13}$ : 771,476).

Example 36

400 mg of the compound 29 was dissolved in 0.8 ml of dichloromethane, then added with 0.2 ml of N,N-diisopropylethylamine and 0.22 ml of methoxyethoxymethyl chloride under cooling, and, after 10 minutes, was let to stand at room temperature. After 3 hours, the same process as in the preparation of the compound 28 was conducted to obtain a colorless glass-like substance. The obtained glass-like substance was purified by silica gel column chromatography, utilizing a developing solvent system of $CHCl_3$ : $CH_3OH$ : conc. $NH_4OH$ = 100 : 1 : 0.01, to obtain 250 mg (yield 56.0%) of 2'-0-acetyl-4''-O-methoxyethoxymethyl-8,9-anhydroerythromycinA 6,9-hemiketal (compound 43) as white powder.

Rf value : 0.43 ($CHCl_3$ : $CH_3OH$ : conc. $NH_4OH$ = 10 : 1 : 0.01), high mass : 845.513 (calcd. for $C_{43}H_{75}NO_{15}$ : 845.513).

Example 37

150 mg of the compound 43 obtained in Example 36 was dissolved in 6 ml of methanol and was let to stand at room temperature. After one day, the reaction solution was concentrated under reduced pressure to obtain a colorless glass-like substance. The obtained glass-like substance was purified by silica gel column chromatography, utilizing a developing solvent system of $CHCl_3$ : $CH_3OH$ : conc. $NH_4OH$ = 30 : 1 : 0.01, to obtain 85 mg (yield 59.6%) of 4''-O-methoxyethoxymethyl-8,9-anhydroerythromycin A 6,9-hemiketal (compound 44) in white powder.

Rf value : 0.27 ($CHCl_3$ : $CH_3OH$ : conc. $NH_4OH$ = 10 : 1 : 0.01), high mass : 803.502 (calcd. for $C_{41}H_{73}NO_{14}$ : 803.502).

The structure, specific rotatory power and NMR spectrum of the compounds obtained in Examples 23 - 37 are summarized in Tables 4 and 5.

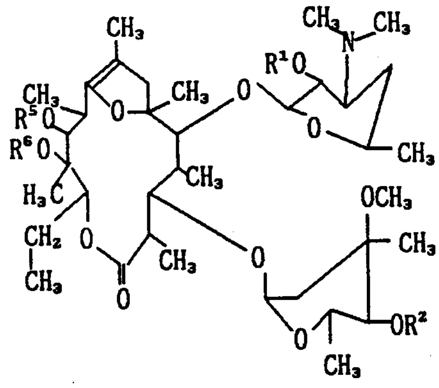

Table 4

| Compound No. | $R^1$ | $R^2$ | $R^5$ | $R^6$ | $[\alpha]_D^{23}$(c1.0, $CHCl_3$) |
|---|---|---|---|---|---|
| 28 | $CH_3CH_2CH_2CO$ | H | H | H | −37.4° |
| 30 | H | $CH_3SO_2$ | H | H | −44.6° |
| 32 | H | H | $CH_3CO$ | $CH_3CO$ | −30.0° |
| 33 | H | H | $CH_3CH_2CO$ | $CH_3CH_2CO$ | −22.0° |
| 34 | H | H | $CH_3CH_2CH_2CO$ | $CH_3CH_2CH_2CO$ | −19.0° |
| 35 | $CH_3CO$ | CHO | >=S | | + 8.6° |
| 36 | H | H | >=S | | +25.0° |
| 37 | H | H | >S=O | | −30.2° |
| 38 | $CH_3CO$ | H | >B−Ph | | −54.0° |
| 39 | H | H | >B−Ph | | −60.2° |
| 40 | $CH_3CO$ | $(CH_3)_3Si$ | $(CH_3)_3Si$ | H | |
| 41 | H | H | H | $CH_3CO$ | −35.6° |
| 42 | H | H | H | $CH_3CH_2CO$ | −65.2° (c 0.5) |
| 43 | $CH_3CO$ | $CH_3OCH_2CH_2OCH_2$ | H | H | −30.4° |
| 44 | H | $CH_3OCH_2CH_2OCH_2$ | H | H | −34.0° |

In Table 4 Ph is phenyl, Si is sylyl.

The number of compounds correspond to those in Examples.

Table 5

$^{1}$H-NMR peak ($\delta$ value ppm, solvent $CDCl_3$)

| Compound No. | 8-Me(s,3H) | 3'-$NMe_2$(s,6H) | 3''-OMe(s,3H) | Others |
|---|---|---|---|---|
| 28 | 1.51 | 2.25 | 3.20 | |
| 30 | 1.56 | 2.28 | 3.32 | 4''-$SCH_3$ 3.08(s,3H) |
| 32 | 1.53 | 2.20 | 3.34 | 12-$COCH_3$ 2.04(s,3H), 11-$COCH_3$, 2.14(s,3H) |
| 33 | 1.52 | 2.28 | 3.34 | |
| 34 | 1.48 | 2.24 | 3.30 | |
| 35 | 1.58 | 2.28 | 3.34 | 2'-$COCH_3$ 2.00(s,3H), 4''-CHO 8.28(s,1H) |
| 36 | 1.58 | 2.28 | 3.25 | |
| 37 | 1.57 | 2.20 | 3.20 | |
| 38 | 1.57 | 2.31 | 3.36 | 8-Ph 7.4~7.0(m,5H), 2'-$COCH_3$ 2.07(s,3H) |
| 39 | 1.63 | 2.33 | 3.30 | 8-Ph 7.4~7.0(m,5H) |
| 41 | 1.51 | 2.20 | 3.31 | 12-$COCH_3$ 1.08(s,3H) |
| 42 | 1.56 | 2.32 | 3.32 | |
| 43 | 1.55 | 2.26 | 3.38 | 2'-$COCH_3$ 2.04(s,3H), $OCH_2OCH_2CH_2OCH_3$ 3.38(s,3H), 4.83(s,2H) |
| 44 | 1.56 | 2.27 | 3.32 | $OCH_2OCH_2CH_2OCH_3$ 3.38(s,3H), 4.83(s,2H) |

Example 38

300 mg of the compound 27 was dissolved in 3 ml of dry pyridine, and added with 0.4 ml of acetic anhydride. The reaction mixture was heated at 50 °C for 24 hours. The reaction solution was poured into 10 ml of the cold saturated aqueous solution of sodium hydrogen carbonate, and the resulting product was extracted with chloroform (3 x 10 ml) The extracting solution was dried with anhydrous sodium sulfate, and the solvent was removed under reduced pressure to obtain a crude product. This product was purified by silica gel column chromatography (Merck Art 7734 silica gel 20 g ; eluting solvent:chloroform-methanol (50 : 1)) to obtain 290 mg of 11,2',4''-tri-O-acetyl-8,9-anhydroerythromycin A 6,9-hemiketal (compound 45) as white powder.

Rf value : 0.38 ($CHCl_3$ : $CH_3OH$ = 20 : 1).

Example 39

290 mg of the compound 45 obtained in Example 38 was dissolved in 3 ml of dry dimethyl sulfoxide, and added with 1 ml. of acetic anhydride. The reaction mixture was let to stand for 96 hours at room temperature. The reaction solution was concentrated under reduced pressure (<267Pa(<2mm Hg)), and the residue was dissolved in 20 ml of chloroform. The obtained chloroform solution was washed with 10 ml of the saturated aqueous solution of sodium hydrogen carbonate, then dried with anhydrous sodium sulfate, and the solvent was distilled off under reduced pressure. The crude produce was purified by silica gel column chromatography (Merck Art 7734 silica gel 20 g.; eluting solvent: chloroform-methanol (50 : 1)), to obtain 173 mg of 11,2',4''-tri-O-acetyl-12-O-methylthiomethyl-8,9-anhydroerythromycin A 6,9-hemiketal (compound 46) as white powder.

Rf value : 0.39 ($CHCl_3$ : $CH_3OH$ = 20 : 1).

Example 40

173 mg of the compound 46 obtained in Example 39 was dissolved in 5 ml of methanol, and added with 20 mg of lithium hydroxide. The reaction solution was heated at 50 °C for 4 hours under agitation. After concentration under reduced pressure, the residue was dissolved in 20 ml of chloroform. The chloroform solution was washed with 10 ml of water, then dried with anhydrous sodium sulfate, and the solvent was distilled off under reduced pressure. The crude product was purified by silica gel column chromatography (Merck Art 7734 silica gel 15 g; eluting solvent : chloroform-methanol (30 : 1)), to obtain 118 mg of 12-O-methylthiomethyl-8,9-anhydroerythromycin A 6,9-hemiketal (compound 47) was white powder.

Rf value : 0.16 ($CHCl_3$ : $CH_3OH$ = 10 : 1)

Example 41

300 mg of the compound 8 was dissolved in 3 ml of dry pyridine, and added with 0.3 ml of acetic anhydride. The mixture was heated at 50 °C for 24 hours. The reaction solution was poured into 10 ml of the cold saturated aqueous solution of sodium hydrogen carbonate, and the resulting product was extracted with chloroform (3 x 10 ml). The extracting solution was dried with anhydrous sodium sulfate, and the solvent was distilled off under reduced pressure to obtain a crude product. This product was purified by silica gel column chromatography (Merck Art 7734 silica gel 20 g, eluting solvent : chloroform-methanol (50 : 1)) to obtain 195 mg of 11,2'-di-O-acetyl-4''-O-formyl-8,9-anhydroerythromycin A 6,9-hemidetal (compound 48) as white powder.

Rf value : 0.37 ($CHCl_3$ : $CH_3OH$ = 10 : 1) high mass : 827.4689 (calcd. for $C_{42}H_{69}NO_{15}$ : 827.4663).

Example 42

195 mg of the compound 48 obtained in Example 41 was dissolved in 5 ml of methanol, and the solution was heated under reflux for 1 hour. Then the solvent was distilled off under reduced pressure to obtain a crude product. This product was purified by silica gel column chromatography (Merck Art 7734 silica gel 20 g, eluting solvent : chloroform-methanol (30 : 1)) to obtain 155 mg of 11-O-acetyl-4''-O-formyl-8,9-anhydroerythromycin A 6,9-hemiketal (compound 49) as white powder.

Rf value : 0.28 ($CHCl_3$ : $CH_3OH$ = 10 : 1)

Example 43

210 mg of the compound 48 obtained in Example 41 was dissolved in 5 ml of metahnol, and the solution was heated under reflux for 45 hours. Then the solvent was distilled off under reduced pressure to obtain a crude product. This product was purified by silica gel column chromatography (Merck Art 7734 silica gel 20 g, eluding solvent : chloroform-methanol (30 : 1)) to obtain 158 mg of 11-O-acetyl-8,9-anhydroerythromycin A 6,9-hemiketal (compound 50) as white powder.

Rf value : 0.21 ($CHCl_3$ : $CH_3OH$ = 10 : 1).

Example 44

155 mg of the compound 49 obtained in Example 42 was processed in the same manner as in Example 43 to obtain 115 mg of 11-O-acetyl-8,9-anhydroerythromycin A 6,9-hemiketal (compound 50) as white powder.

Example 45

300 mg of the compound 8 was dissolved in 3 ml of dry pyridine, and added with 0.3 ml of acetic anhydride. The reaction mixture was heated at 50 °C for 24 hours. The reaction solution was poured into 10 ml of the cold saturated aqueous solution of sodium hydrogen carbonate, and the resulting product was extracted with chloroform (3 x 10 ml). The extract was dried with anhydrous sodium sulfate, and the solvent was distilled off under reduced pressure. The residue was dissolved in 5 ml of methanol, and heated under reflux for 45 hours. The solvent was distilled off under reduced pressure, and the residue was purified by silica gel column chromatography to obtain 156 mg of 11-O-acetyl-8,9-anhydroerythromycinA 6,9-hemiketal (compound 50) as white powder.

Example 46

300 mg of the compound 8 and 0.3 ml of propionic anhydride were reacted according to the method of Example 45, and the protection was removed with methanol. The crude product was purified by silica gel column chromatography (Merck Art 7734 silica gel 20 g, eluting solvent : chloroform-methanol (30 : 1)) to obtain 152 mg of 11-O-propionyl-8,9-anhydroerythromycin A 6,9-hemiketal (compound 51) as white powder.

Rf value : 0.21 ($CHCl_3$ : $CH_3OH$) = 10 : 1).

Example 47

300 mg of the compound 8 and 0.3 ml of butyric anhydride were reacted and after removal of the protection according to the process of Example 45, a crude product was obtained. This product was purified by silica gel column chromatography (Merck Art 7734 silica gel 20 g, eluting solvent : chloroform-methanol (30 : 1) to obtain 146 mg of 11-O-butyryl-8,9-anhydroerythromycin A 6,9-hemiketal (compound 52) as white powder.

Rf value : 0.21 ($CHCl_3$ : $CH_3OH$ = 10 : 1)

Example 48

300 mg of the compound 8 and 0.3 ml of benzoyl chloride were reacted and after removal of the protection according to the process of Example 45, a crude product was obtained. This product was purified by silica gel column chromatography (Merck Art 7734 silica gel 20 g, eluting solvent : chloroform-methanol (30 : 1)) to obtain 155 mg of 11-O-benzoyl-8,9-anhydroerythromycin A 6,9-hemiketal (compound 53) as white powder.

Rf value : 0.20 ($CHCl_3$ : $CH_3OH$ = 10 : 1)

Example 49

200 mg of erythromycin A was dissolved in 2 ml of $CHCl_3$, then added with 78 µl of 2-methoxypropene and 64 mg of pyridinium chloride and let to stand at room temperature. After 1 day, the reaction solution was diluted with 20 ml of $CHCl_3$, and washed with 20 ml of the saturated aqueous solution of sodium hydrogen carbonate and 20 ml of water. The $CHCl_3$ layer was dried with anhydrous sodium sulfated and

concentrated under a reduced pressure to obtain a colorless glass-like substance. The obtained glass-like substance was purified by silica gel column chromatography, utilizing a developing solvent system of $CHCl_3$ : $CH_3OH$ : conc. $NH_4OH$ = 30 : 1 : 0.01, to obtain 194 mg (94.0%) of 11,12-O-isopropylidene-8,9-anhydroerythromycin A 6,9-hemiketal (compound 54) as colorless powder.

Rf value : 0.14 ($CHCl_3$ : $CH_3OH$ : conc. $NH_4OH$ = 10 : 1 : 0.01), high mass : 755.4856 (calcd. for $C_{40}H_{69}NO_{12}$ : 755.4815).

The structure, specific rotatory power and NMR spectrum of the compounds obtained in Examples 38 - 49 are summarized in Tables 6 and 7.

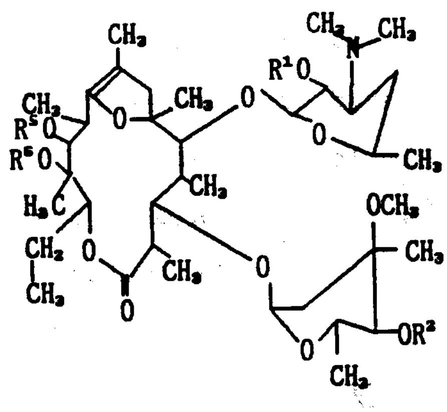

Table 6

| Compound No. | $R^1$ | $R^2$ | $R^5$ | $R^6$ | $[\alpha]_D^{23}$ (c1.0, $CHCl_3$) |
|---|---|---|---|---|---|
| 45 | $CH_3CO$ | $CH_3CO$ | $CH_3CO$ | H | −30.6° |
| 46 | $CH_3CO$ | $CH_3CO$ | $CH_3CO$ | $CH_3SCH_2$ | −31.6° |
| 47 | H | H | H | $CH_3SCH_2$ | −28.6° |
| 48 | $CH_3CO$ | CHO | $CH_3CO$ | H | −25.6° |
| 49 | H | CHO | $CH_3CO$ | H | −18.6° |
| 50 | H | H | $CH_3CO$ | H | −18.0° |
| 51 | H | H | $CH_3CH_2CO$ | H | −19.2° |
| 52 | H | H | $CH_3CH_2CH_2CO$ | H | −20.4° |
| 53 | H | H | PhCO | H | −38.0° |
| 54 | H | H | $(CH_3)_2C=$ | | −24.8° |

In the Table 6, Ph is phenyl.

The numbers of compounds correspond to those in Examples.

Table 7

$^1H$-NMR peak (δ value ppm, solvent $CDCl_3$)

| Compound No. | 8-Me(s,3H) | 3'-$NMe_2$(s,6H) | 3''-OMe(s,3H) | Others |
|---|---|---|---|---|
| 45 | 1.56 | 2.30 | 3.35 | 2'-$COCH_3$ 2.06(s,3H), 4''-$COCH_3$ 2.10(s,3H), 11-$COCH_3$, 2.12(s,3H) |
| 46 | 1.57 | 2.20 | 3.35 | 2'-$COCH_3$ 2.05(s,3H), 4''-$COCH_3$ 2.08(s,3H), 11-$COCH_3$, 2.10(s,3H), 12$CH_2SCH_3$, 2.10(s,3H) |
| 47 | 1.54 | 2.30 | 3.20 | 12-$CH_2SCH_3$ 2.10(s,3H) |
| 48 | 1.57 | 2.28 | 3.37 | 2'-OAc 2.05(s,3H), 11-OAc 2.12(s,3H), 4''-OCHO 8.20(s,1H) |
| 49 | 1.58 | 2.30 | 3.35 | 11-OAc 2.12(s,3H), 4''-OCHO 8.20(s,1H) |
| 50 | 1.58 | 2.31 | 3.36 | 11-OAc 2.13(s,3H) |
| 51 | 1.58 | 2.31 | 3.35 | |
| 52 | 1.58 | 2.31 | 3.35 | |
| 53 | 1.58 | 2.34 | 3.36 | 11-OBz 7.43 (m,3H), 8.05(m,2H) |

In Table 7, Ac is acetyl and Bz is benzoyl.

Example 50

100 mg of the compound 27 was dissolved in 1 ml of chloroform and stirred for 2 hours with addition of 40 µl of methyl iodide. After most of the solvent was distilled off, 5 ml of ether was added and the precipitate formed was filtered. The precipitate was washed with ether and dried to obtain 65 mg (yield 54%) of 8,9-anhydroerythromycin A 6,9-hemiketal methyl iodide (compound 55) in white powder.

Example 51

By using 30 mg of the compound 32 and 15 µl of methyl iodide, the same processing as in Example 50 was conducted to obtain 18 mg (yield 51%) of 11,12-di-O-acetyl-8,9-anhydroerythromycin A 6,9-hemiketal methyl iodide (compound 56) in white powder.

Example 52

By using 79 mg of 11-O-methanesulfonyl-8,9-anhydroerythromycin A 6,9-hemiketal (compound 57) and 29 µl of methyl iodide, the same processing as in Example 50 was conducted to obtain 55 mg (yield 58%) of 11-O-methanesulfonyl8,9-anhydroerythromycin A 6,9-hemiketal methyl iodide (compound 58), in white powder.

Example 53

By using 78 mg of the compound 25 and 59 µl of methyl iodide, the same processing as in Example 50 was conducted to obtain 67 mg (yield 74%) of 11-O-methanesulfonyl-4''-O-formyl-8,9-anhydroerythromycin A 6,9-hemiketal methyl iodide (compound 59) in pale yellow powder.

Example 54

200 mg of the compound 27 was dissolved in 4 ml of chloroform, then 0.5 ml of ethyl iodide was added thereto and the mixture was refluxed for 20 hours. After most of the solvent was distilled off under reduced pressure, 10 ml of ether was added and a precipitate formed was filtered. The precipitate was washed with ether and dried to obtain 145 mg (yield 60%) of 8,9-anhydroerythromycin A 6,9-hemiketal ethyl iodide (compound 60) in white powder.

Example 55

200 mg of the compound 27 was dissolved in 4 ml of chloroform, then 0.5 ml of propyl iodide was added thereto and the mixture was refluxed for 48 hours. After the same processing as in Example 54, 120 mg (yield 48%) of 8,9-anhydroerythromycin A 6,9-hemiketal propyl iodide (compound 61) was obtained in white powder.

Example 56

200 mg of the compound (1) and 0.2 ml of methyl iodide were employed to carry out the same processing as in Example 50. As the result, 154 mg (yield 65%) of 2'-O-acetyl-8,9-anhydroerythromycin A 6,9-hemiketal methyl iodide (compound 62) was obtained in white powder.

The structural formulae of the compounds obtained in Examples 50 to 56 and their physical properties are shown in Table 8 and Table 9, respectively.

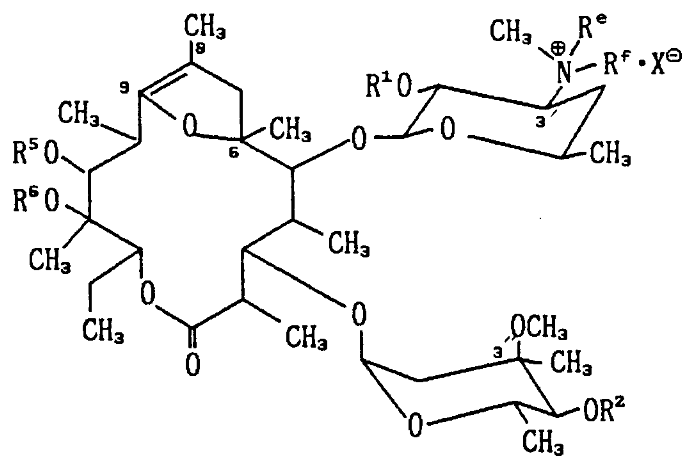

Table 8

| Compound No. | $R^1$ | $R^2$ | $R^5$ | $R^6$ | $R^e$ | $R^f$ | X |
|---|---|---|---|---|---|---|---|
| 55 | H | H | H | H | $CH_3$ | $CH_3$ | I |
| 56 | H | H | $CH_3CO$ | $CH_3CO$ | $CH_3$ | $CH_3$ | I |
| 58 | H | H | $CH_3SO_2$ | H | $CH_3$ | $CH_3$ | I |
| 59 | H | CHO | $CH_3SO_2$ | H | $CH_3$ | $CH_3$ | I |
| 60 | H | H | H | H | $CH_3CH_2$ | $CH_3$ | I |
| 61 | H | H | H | H | $CH_3CH_2CH_2$ | $CH_3$ | I |
| 62 | $CH_3CO$ | H | H | H | $CH_3$ | $CH_3$ | I |

Table 9

| Compound No. | specific rotary power | NMR spectrum δ value ppm | | | |
|---|---|---|---|---|---|
| | | 8-Me(s,3H) | 3′-NMe | 3″-OMe(s,3H) | others(solvent) |
| 55 | $[\alpha]^{23}_D$ -28.6° ($\underline{c}$=1.0, $CHCl_3$) | 1.58 | 3.64 (s,9H) | 3.49 | ($CDCl_3$) |
| 56 | $[\alpha]^{23}_D$ -25.4° ($\underline{c}$=1.0, $CHCl_3$) | 1.51 | 3.48 (s,9H) | 3.37 | 1.99(11-$COCH_3$,s,3H), 2.03(12-$COCH_3$,s,3H) ($CDCl_3$) |
| 58 | $[\alpha]^{23}_D$ -22.2° ($\underline{c}$=1.0, $CH_3OH$) | 1.59 | 3.35 (s,9H) | 3.43 | 3.18($SO_2CH_3$,s,3H) ($CDCl_3$) |
| 59 | $[\alpha]^{23}_D$ -24.8° ($\underline{c}$=1.0, $CHCl_3$) | 1.58 | 3.34 (s,9H) | 3.54 | 3.16($SO_2CH_3$,s,3H) 8.28(CHO,s,1H) ($CDCl_3$) |
| 60 | $[\alpha]^{23}_D$ -27.8° ($\underline{c}$=1.0, $CH_3OH$) | 1.59 | 3.19 (s,6H) | 3.38 | ($CD_3OD$) |
| 61 | $[\alpha]^{23}_D$ -28.4° ($\underline{c}$=1.0, $CH_3OH$) | 1.58 | 3.12 (s,6H) | 3.38 | ($CD_3OD$) |
| 62 | $[\alpha]^{23}_D$ -29.2° ($\underline{c}$=1.0, $CH_3OH$) | 1.58 | 3.22 (s,9H) | 3.40 | 2.19(2′-O-$COCH_3$,s,3H) ($CD_3OD$) |

Example 57

100 mg of the compound 27 was dissolved in 2 ml of dry ether and added with 73 µl of diisopropylethylamine and 33 µl of valeryl chloride at 0 °C. The mixture was warmed to room temperature, and stirred for 15 minutes at the ame treatment, followed by dilution with addition of 25 ml of ethyl acetate. This was washed with the saturated aqueous sodium hydrogen carbonate and saturated aqueous sodium chloride solution, followed by drying over anhydrous sodium sulfate. The crude produce obtained by evaporation of the solvent was purified by silica gel chromatography (developing solvent: chloroform-methanol-conc. aqueous ammonia (20 : 1 : 0.01)) to obtain 96 mg (yield 86%) of 2'-O-valeryl-8,9-anhydroerythromycin A 6,9-hemiketal (compound 63) in white powder.

Example 58

By using 50 mg of the compound 27, 37 µl of diisopropylethylamine and 20 µl of hexanoyl chloride, the same processing as in Example 57 was conducted to obtain 53 mg (yield 94%) of 2'-O-hexanoyl-8,9-anhydroerythromycin A 6,9-hemiketal (compound 64) in white powder.

Example 59

By using 100 mg of the compound 27, 73 µl of diisopropylethylamine and 93 mg of arachidonyl chloride, the same processing as in Example 57 was conducted to obtain 104 mg (yield 73%) of 2'-O-arachidonyl-8,9-anhydroerythromycin A 6,9-hemiketal (compound 65) in white powder.

Example 60

By using 100 mg of the compound 27, 73 µl of diisopropylethylamine and 34 µl of isovaleryl chloride, the same processing as in Example 57 was conducted to obtain 100 mg (yield 89%) of 2'-O-isovaleryl-8,9-anhydroerythromycin A 6,9-hemiketal (compound 66) in white powder.

Example 61

By using 100 mg of the compound 27, 73 µl of diisopropylethylamine and 27 µl of crotonyl chloride, the same processing as in Example 57 was conducted to obtain 87 mg (yield 79%) of 2'-O-crotonyl-8,9-anhydroerythromycin A 6,9-hemiketal (compound 67) in white powder.

Example 62

By using 100 mg of the compound 27, 73 µl of diisopropylethylamine and 33 µl of benzoyl chloride, the same processing as in Example 57 was conducted to obtain 86 mg (yield 75%) of 2'-O-benzoyl-8,9-anhydroerythromycin A 6,9-hemiketal (compound 68) in white powder.

Example 63

200 mg of the compound 27 was dissolved in 4 ml of chloroform and 150 µl of diisopropylethylamine was added thereto. After the mixture was heated to 50°C, 32 µl of methanesulfonyl chloride was added thereto and the mixture was stirred for 25 minutes, followed further by addition of 20 µl of methanesulfonyl chloride. After stirring for 15 minutes, the mixture was cooled to room temperature and diluted with 30 ml of ethyl acetate. This was washed with saturated aqueous sodium hydrogen carbonate and saturated aqueous sodium chloride solution, and dried over anhydrous sodium sulfate. The residue obtained by evaporation of the solvent was purified by silica gel chromatography (developing solvent: chloroform-methanol-conc. aqueous ammonia (60 : 1 : 0.01)) to obtain 53 mg of 2'-O-methanesulfonyl-8,9-anhydroerythromycin A 6,9-hemiketal (compound 69) (yield 24%) and 52 mg (yield 21%) of 11,2'-di-O-methanesulfonyl-8,9-anhydroerythromycin A 6,9-hemiketal (compound 70).

Example 64

100 mg of the compound 27 was dissolved in 1 ml of dry pyridine, added with 0.3 ml of diphenylchlorophosphate and the mixture was stirred overnight. The mixture was diluted with 20 ml of ethyl acetate and the solution washed with saturated aqueous sodium hydrogen carbonate and saturated aqueous sodium chloride solution, and was dired over anhydrous sodium sulfate and the solvent was evaporated. The crude product obtained was purified by silica gel chromatography (developing solvent: chloroform-methanol-conc. aqueous ammonia (10 : 1 : 0.01)) to obtain 43 mg (yield 33%) of 2'-O-diphenylphosphoryl-8,9-anhydroerythromycin A 6,9-hemiketal (compound 71) in white powder.

Example 65

Using 100 mg of the compound 27, 1 ml of pyridine and 0.2 ml of diethylchlorophosphate, the same processing as in Example 64 was conducted to obtain 25 mg (yield 21%) of 2'-O-diethylphosphoryl-8,9-anhydroerythromycin A 6,9-hemiketal (compound 72) in white powder.

Example 66

157 mg of the compound (8) was dissolved in 1 ml of dry pyridine, added with 0.2 ml of valeric anhydride and the mixture was stirred at 50 °C for 2 weeks. After the mixture was cooled to room temperature, it was diluted with 30 ml of ethyl acetate and washed with saturated aqueous sodium hydrogen carbonate and saturated aqueous sodium chloride solution and dried over anhydrous sodium sulfate. The solvent was evaporated and the residue obtained was dissolved in 6 ml of methanol, followed by stirring at 50 °C for 3 hours. After cooling to room temperature and addition of 0.4 ml of 5% aqueous sodium hydrogen carbonate solution, the mixture was further stirred for 6 hours. After concentration to a volume of about 2 ml, the concentrate was diluted with 30 ml of ethyl acetate and washed with saturated aqueous sodium chloride solution, followed by drying over anhydrous sodium sulfate. The crude product obtained by evaporation of the solvent was purified by silica gel chromatography (developing solvent: chloroform-methanol-conc. aqueous ammonia (10 : 1 : 0.1)) to obtain 91 mg (yield 57%) of 11-O-valeryl-8,9-anhydroerythromycin A 6,9-hemiketal (compound 73) in white powder.

Example 67

By using 157 mg of the compound 8, 1 ml of dry pyridine and 0.2 ml of hexanoic acid anhydride, the same processing as in Example 66 was conducted to obtain 98 mg (yield 60%) of 11-O-hexanoyl-8,9-anhydroerythromycin A 6,9-hemiketal (compound 74) in white powder.

The structural formulae, specific rotatory powers and NMR spectrum values of the compounds obtained in Examples 57-67 shown in Table 10.

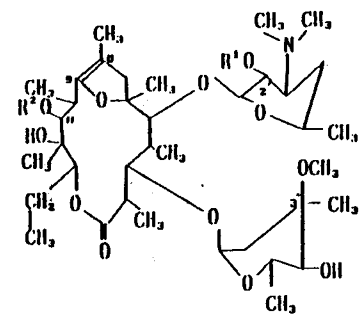

Table 10

| compound No. | $R^1$ | $R^2$ | $(\alpha)_D^{23}$ (c1.0, $CHCl_3$) | $^1H-NMR$ ($\delta$ value, $CDCl_3$) 8-Me(s, 3H) | 3'-$NMe_2$(s, 6H) | 3"-OMe(s, 3H) | others |
|---|---|---|---|---|---|---|---|
| 63 | $CO(CH_2)_3CH_3$ | H | -30.2° | 1.55 | 2.25 | 3.38 | |
| 64 | $CO(CH_2)_4CH_3$ | H | -39.8° | 1.56 | 2.23 | 3.38 | |
| 65 | $CO(CH_2)_{10}CH_3$ | H | -20.4° | 1.55 | 2.26 | 3.38 | |
| 66 | $COCH_2CH(CH_3)_2$ | H | -37.2° | 1.55 | 2.24 | 3.37 | 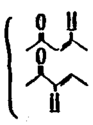 6.05(m, 1H) |
| 67 | $COCH=CHCH_3$ | H | -44.6° | 1.54 | 2.28 | 3.30 | 5.82(m, 1H) |
| 68 | COPh | H | -43.6° | 1.62 | 2.30 | 3.46 | Ph : 7.45(m, 3H), 8.00(m, 2H) |
| 69 | $SO_2CH_3$ | H | -20.2° | 1.57 | 2.30 | 3.35 | $SO_2CH_3$ : 3.17(s, 3H) |
| 70 | $SO_2CH_3$ | $SO_2CH_3$ | -24.0° | 1.58 | 2.30 | 3.34 | $SO_2CH_3$ : 3.17(s, 3H) |
| 71 | $PO(OPh)_2$ | H | -42.4° | 1.57 | 2.33 | 3.37 | Ph : 7.23(m, 10H) |
| 72 | $PO(OEt)_2$ | H | -42.4° | 1.56 | 2.28 | 3.32 | |
| 73 | H | $CO(CH_2)_3CH_3$ | -20.4° | 1.57 | 2.20 | 3.35 | |
| 74 | H | $CO(CH_2)_4CH_3$ | -17.8° | 1.57 | 2.30 | 3.35 | |

Example 68

1.00 g of de(N-methyl)erythromycin A (reference: Japanese Laid-open Patent Application No. 9129/1972) was dissolved in 5 ml of glacial acetic acid and the solution was stirred for 1 hour. The reaction

mixture was poured into 20 ml of ice-cooled conc. aqueous ammonia. The mixture was extracted 3 times with 10 ml of chloroform. The chloroform solution was dried over anhydrous sodium sulfate, and the solvent was evaporated under reduced pressure. The residue was purified by silica gel chromatography (developing solvent: chloroform-methanol-conc. aqueous ammonia (10 : 1 : 0.1)) to obtain 830 mg (yield 85%) of de(N-methyl)-8,9-anhydroerythromycin A 6,9-hemiketal (compound 75) in white powder.

Example 69

930 mg of bis-(de(N-methyl)) erythromycin A (reference: Japanese Laid-open Patent Application No. 9129/1972) was processed in the same manner as in Example 68 to obtain 770 mg (yield 85%) of bis-(de-(N-methyl))-8,9-anhydroerythromycin A 6,9-hemiketal (compound 76) in white powder.

Example 70

400 mg of ethyl-de(N-methyl)-erythromycin A (reference: R. K. Clark. Jr. et al. Antibiotics and Chemotherapy VII, 483, (1957)) was processed in the same manner as in Example 68 to obtain 327 mg (yield 84%) of ethyl-de(N-methyl)-8,9-anhydroerythromycin A 6,9-hemiketal (compound 77) in white powder.

Example 71

168 mg of butyl-de(N-methyl)-erythromycin A (reference: R. K. Clark, Jr. et al. Antibiotics and Chemotherapy VII, 483, (1957)) was processed in the same manner as in Example 68 to obtain 99 mg (yield 60%) of butyl-de(N-methyl)-8,9-anhydroerythromycin A 6,9-hemiketal (compound 78) in white powder.

Example 72

88 mg of the compound 77 was dissolved in 2 ml of chloroform, then 1 ml of ethyl iodide was added thereto and the mixture was stirred at 80 °C for 14 hours. After most of the solvent was evaporated under reduced pressure, 5 ml of ether was added and the precipitate formed was filtered. The precipitate was washed with ether and dried to obtain 72 mg (yield 67%) of ethyl-de(N-methyl)-8,9-anhydroerythromycin A 6,9-hemiketal ethyl iodide (compound 79) in white powder.

Example 73

376 mg of the compound 76 was dissolved in 5 ml of methanol. 138 mg of sodium hydrogen carbonate and 1.0 ml of 1,4-dibromobuthane were added, and the mixture was stirred at 50 °C for 8 hours. The reaction mixture was diluted with 30 ml of ethyl acetate, and washed with water and saturated aqueous sodium chloride solution. The ethyl acetate solution was dried over anhydrous sodium sulfate and the solvent was evaporated under reduced pressure. The residue was purified by silica gel column chromatography (eluant: chloroform-methanol-conc. aqueous ammonia (10 : 1 : 0.1)) to obtain 158 mg (yield 39%) of de(dimethylamino)-3'-pyrrolidino-8,9-anhydroerythromycin A 6,9-hemiketal (compound 80) in white powder.

Example 74

By using 63 mg of the compound 80 and 0.1 ml of methyl iodide, the same processing as in Example 50 was conducted to obtain 70 mg (yield 93%) of de(dimethylamino)-3'-pyrrolidino-8,9-anhydroerythromycin A 6,9-hemiketal methyl iodide (compound 81) in white powder.

Example 75

120 mg of the compound 27 was dissolved in 1 ml of chloroform, then 0.5 ml of 2-bromoethanol and 0.5 ml of diisopropylethylamine were added thereto and the mixture was stirred for 2 days. After evaporation of the solvent, 5 ml of ether was added and the precipitate formed was filtered. The precipitate was washed with 10 ml of ether and dried to obtain 119 mg (yield 84%) of 8,9-anhydroerythromycin A 6,9-hemiketal 2-hydroxyethyl bromide (compound 82) in white powder.

Example 76

150 mg of the compound 27 was dissolved in 1 ml of chloroform, then 0.5 ml of allylbromide and 0.25 ml of diisopropylethylamine were added thereto and the mixture was stirred for 1 day. After evaporation of the solvent, 5 ml of ether was added and a precipitate formed was filtered. The precipitate was washed with 10 ml of ether and dried to obtain 134 mg (yield 76%) of 8,9-anhydroerythromycin A 6,9-hemiketal allyl bromide (compound 83) in white powder.

The structural formulae, specific rotatory powers and NMR spectrum values of the compounds obtained in Examples 68 to 76 are shown in Table 11.

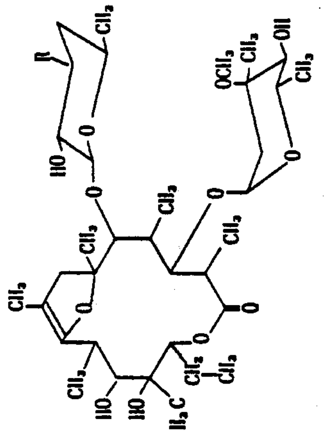

Table 11

| Compound No. | R | Specific rotary power | NMR spectrum δ value ppm 8-Me(s,3H) | 3'' -OMe(s,3H) | others (solvnt) |
|---|---|---|---|---|---|
| 75 | $N{<}^{H}_{CH_3}$ | $(\alpha)_D^{22}$ -29.2° ($\underline{c}$ 1.0, $CH_3OH$) | 1.57 | 3.35 | 2.42 ($NCH_3$, s, 3H) ($CDCl_3$) |
| 76 | $NH_2$ | $(\alpha)_D^{22}$ -43.2° ($\underline{c}$ 1.0, $CHCl_3$) | 1.57 | 3.31 | ($CDCl_3$) |
| 77 | $N{<}^{CH_3}_{C_2H_5}$ | $(\alpha)_D^{22}$ -36.4° ($\underline{c}$ 1.0, $CHCl_3$) | 1.56 | 3.32 | 2.23 ($NCH_3$, s, 3H) ($CDCl_3$) |
| 78 | $N{<}^{CH_3}_{C_4H_9}$ | $(\alpha)_D^{22}$ -34.0° ($\underline{c}$ 1.0, $CHCl_3$) | 1.57 | 3.36 | 2.23 ($NCH_3$, s, 3H) ($CDCl_3$) |
| 79 | $\overset{\oplus}{N}\begin{smallmatrix}CH_3\\C_2H_5\\C_2H_5\end{smallmatrix} \cdot I^{\ominus}$ | $(\alpha)_D^{22}$ -27.0° ($\underline{c}$ 1.0, $CH_3OH$) | 1.58 | 3.38 | 3.05 ($NCH_3$, s, 3H) ($CD_3OD$) |
| 80 | 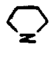 | $(\alpha)_D^{22}$ -30.8° ($\underline{c}$ 1.0, $CH_3OH$) | 1.57 | 3.36 | ($CDCl_3$) |
| 81 | 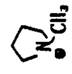 $I^{\ominus}$ | $(\alpha)_D^{22}$ -27.0° ($\underline{c}$ 1.0, $CH_3OH$) | 1.58 | 3.37 | 2.98 ($NCH_3$, s, 3H) ($CD_3OD$) |
| 82 | $\overset{\oplus}{N}\begin{smallmatrix}CH_3\\CH_3\\CH_2CH_2OH\end{smallmatrix}$ $Br^{\ominus}$ | $(\alpha)_D^{22}$ -26.4° ($\underline{c}$ 1.0, $CH_3OH$) | 1.54 | 3.34 | 3.34 ($NMe_2$, s, 6H) ($CD_3OD$) |
| 83 | $\overset{\oplus}{N}\begin{smallmatrix}CH_3\\CH_3\\CH_2CH{=}CH_2\end{smallmatrix}$ $Br^{\ominus}$ | $(\alpha)_D^{22}$ -25.8° ($\underline{c}$ 1.0, $CH_3OH$) | 1.58 | 3.37 | 3.19 ($NMe_2$, s, 6H) ($CD_3OD$) |

Example 77

100 mg of 9-dihydroerythromycin A 6,9-epoxide (compound 84) (reference: Japanese Laid-open Patent Publication No. 1588/1972) was dissolved in 1 ml of chloroform, then 0.6 ml of methyl iodide was added thereto and the mixture was heated under reflux for 1.5 hours. After evaporation of the solvent, 5 ml of ether was added and the precipitate formed was filtered. The precipitate was washed with 10 ml of ether and

dried to obtain 85 mg (yield 71%) 9-dihydroerythromycin A 6,9-epoxide methyl iodide (compound 85) in white powder.

Example 78

100 mg of the compound 84 was dissolved in 1 ml of chloroform, then 0.6 ml of ethyl iodide was added thereto and the mixture was heated under reflux for 2 days. After evaporation of the solvent, 5 ml of ether was added and the precipitate formed was filtered. The precipitate was washed with 10 ml of ether and dried to obtain 90 mg (yield 74%) of 9-dihydroerythromycin A 6,9-epoxide ethyl iodide (compound 86) in white powder.

Example 79

100 mg of the compound 84 was dissolved in 1 ml of chloroform, then 0.7 ml of propyl iodide was added thereto, and the mixture was heated under reflux for 2 days. After evaporation of the solvent, 5 ml of ether was added and the precipitate formed was filtered. The precipitate was washed with 10 ml of ether and dried to obtain 87 mg (yield 70%) of 9-dihydroerythromycin A 6,9-epoxide propyl iodide (compound 87) in white powder.

Example 80

100 mg of the compound 84 was dissolved in 1 ml chloroform, then 1.0 ml of butyl iodide was added thereto, and the mixture was heated under reflux for 1 day. After evaporation of the solvent, 5 ml of ether was added and the precipitate formed was filtered. The precipitate was washed with 10 ml of ether and dried to obtain 95 mg (yield 76%) of 9-dihydroerythromycin A 6,9-epoxide butyl iodide (compound 88) in white powder.

The structural formulae, specific rotatory powers and NMR spectrum values of the compound obtained in Examples 77 to 80 are shown in Table 12.

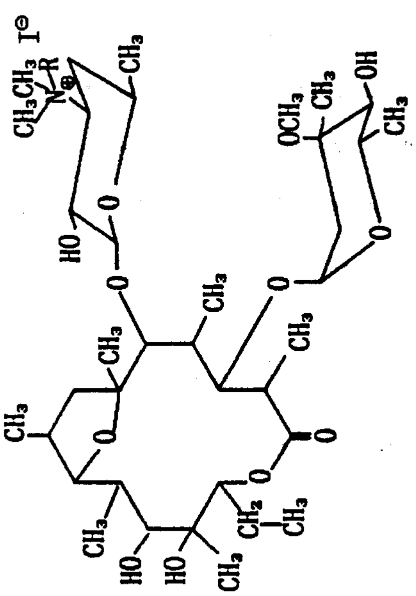

Table 12

| Compound No. | R | $[\alpha]_D^{23}$ (c 1.0, $CH_3OH$) | NMRspectrum δ value ppm ($CD_3OD$) | |
|---|---|---|---|---|
| | | | 3′ -NMe(s) | 3″ -OMe(s,3H) |
| 85 | $CH_3$ | −38.0° | 3.29(3′ -$NMe_3$,9H) | 3.37 |
| 86 | $C_2H_5$ | −35.2° | 3.19(3′ -$NMe_2$,6H) | 3.36 |
| 87 | $CH_2CH_2CH_3$ | −40.6° | 3.22(3′ -$NMe_2$,6H) | 3.36 |
| 88 | $CH_2CH_2CH_2CH_3$ | −40.6° | 3.20(3′ -$NMe_2$,6H) | 3.36 |

Example 81

200 mg of the compound 27 was dissolved in 4 ml of chloroform, then 0.3 ml of benzyl chloride was added thereto and the mixture was heated under reflux for 48 hours. Subsequently, the same processing as in Example 54 was conducted to obtain 122 mg (yield 52%) of 8,9-anhydroerythromycin A 6,9-hemiketal

benzyl chloride (compound 89) in white powder.

Example 82

200 mg of the compound 57 was dissolved in 4 ml of chloroform, then 0.5 ml of ethyl iodide was added thereto and the mixture was heated under reflux for 20 hours. Subsequently, the same processing as in Example 54 was conducted to obtain 134 mg (yield 56%) of 11-O-mesyl-8,9-anhydroerythromycin A 6,9-hemiketal ethyl iodide (compound 90) in pale yellow powder.

Example 83

200 mg of the compound 57 was dissolved in 4 ml of chloroform, then 0.5 ml of propyl iodide was added thereto and the mixture was heated under reflux for 20 hours. Subsequently, the same processing as in Example 54 was conducted to obtain 126 mg (yield 52%) of 11-O-mesyl-8,9-anhydroerythromycin A 6,9-hemiketal propyl iodide (compound 91) in pale yellow powder.

Example 84

200 mg of the compound 27 was dissolved in 4 ml of chloroform, then 0.5 ml of ethyl bromide was added thereto and the mixture was heated under reflux for 48 hours. Subsequently, the same processing as in Example 54 was conducted to obtain 189 mg (yield 82%) of 8,9-anhydroerythromycin A 6,9-hemiketal ethyl bromide (compound 92) in white powder.

The structural formulae, specific rotatory powers and NMR spectrum values of the compounds obtained in Examples 81-84 are shown in Table 13.

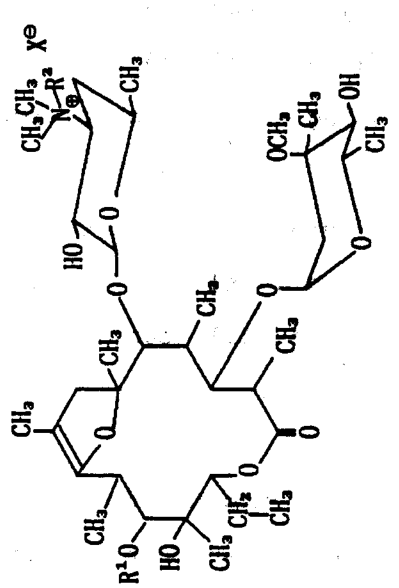

Table 13

| Compound No. | $R^1$ | $R^2$ | X | $[\alpha]_D^{22}$ (c1.0, $CH_3OH$) | NMR spectrum δ value ppm ($CD_3OD$) 8-Me(s,3H) | 3-$NMe_2$(s,6H) | 3″-OMe(s,3H) | others |
|---|---|---|---|---|---|---|---|---|
| 89 | H | $-CH_2Ph$ | Cl | −39.6° | 1.59 | 3.09 | 3.34 | 7.54 (Ph, broad s, 5H) |
| 90 | $-SO_2CH_3$ | $C_2H_5$ | I | −26.4° | 1.60 | 3.17 | 3.37 | 3.24 ($SO_2CH_3$, s, 3H) |
| 91 | $-SO_2CH_3$ | $CH_2CH_2CH_3$ | I | −27.8° | 1.60 | 3.18 | 3.37 | 3.24 ($SO_2CH_3$, s, 3H) |
| 92 | H | $C_2H_5$ | Br | −31.2° | 1.59 | 3.19 | 3.38 | |

## Example 85

206 mg of the compound 76 was dissolved in 3 ml of methanol, then 76 mg of sodium hydrogen carbonate and 0.5 ml of ethyl iodide were added thereto, and the mixture was stirred at 50 °C overnight.

This reaction mixture was diluted with 30 ml of ethyl acetate, and washed with a saturated aqueous sodium hydrogen carbonate and saturated aqueous sodium chloride solution. The ethyl acetate solution was dried over anhydrous sodium sulfate and the solvent was evaporated under reduced pressure. The residue was purified by silica gel column chromatography (developing solvent: chloroform-methanol-conc. aqueous ammonia (50 : 1 : 0.1)) to obtain 98 mg (yield 44%) of diethyl-bis-(de(N-methyl))-8,9-anhydroerythromycin A 6,9-hemiketal (compound 93) and 47 mg (yield 22%) of ethyl-bis-(de(N-methyl))-8,9-anhydroerythromycin A 6,9-hemiketal (compound 94).

Example 86

By using 550 mg of the compound 76, 1.6 ml of 1.5-dibromopentane and 202 mg of sodium hydrogen carbonate, the same processing as in Example 73 was conducted to obtain 327 mg (yield 54%) of de-(dimethylamino)-3'-piperidyl-8,9-anhydroerythromycin A 6,9-hemiketal (compound 95) in white powder.

Example 87

By using 78 mg of the compound 93 and 1 ml of ethyl iodide, the same processing as in Example 72 was conducted to obtain 15 mg (yield 16%) of diethyl-bis-(de(N-methyl))-8,9-anhydroerythromycin A 6,9-hemiketal ethyl iodide (compound 96) in pale yellow powder.

Example 88

By using 93 mg of the compound 80 and 1 ml of ethyl iodide, the same processing as in Example 72 was conducted to obtain 94 mg (yield 84%) of de(dimethylamino)-3'-pyrrolidino-8,9-anhydroerythromycin A 6,9-hemiketal ethyl iodide (compound 97) in pale yellow powder.

Example 89

83 mg of the compound 95 and 0.5 ml of methyl iodide were dissolved in 0.5 ml of chloroform, and stirred at 40 °C for 9 hours. Thereafter, the same processing as in Example 50 was conducted to obtain 84 mg (yield 85%) of de(dimethylamino)-3'-piperidino-8,9-anhydroerythromycin A 6,9-hemiketal methyl iodide (compound 98) in pale yellow powder.

Example 90

By using 94 mg of the compound 95 and 1 ml of ethyl iodide, the same processing as in Example 72 was conducted to obtain 33 mg (yield 29%) of de(dimethylamino)-3'-piperidino-8,9-anhydroerythromycin A 6,9-hemiketal ethyl iodide (compound 99) in pale yellow powder.

Example 91

By using 50 mg of the compound 27 and 0.6 ml of propargyl bromide, the same processing as in Example 50 was conducted to obtain 52 mg (yield 89%) of 8,9-anhydroerythromycin A 6,9-hemiketal propargyl bromide (compound 100) in white powder.

Example 92

By using 111 mg of the compound 32 and 0.12 ml of propargyl bromide, the same processing as in Example 50 was conducted to obtain 111 mg (yield 87%) of 11,12-di-O-acetyl-8,9-anhydroerythromycin A 6,9-hemiketal propargyl bromide (compound 101) in white powder.

The structural formulae, specific rotatory powers and NMR spectrum values of the compounds obtained in Examples 85-92 are shown in Table 14.

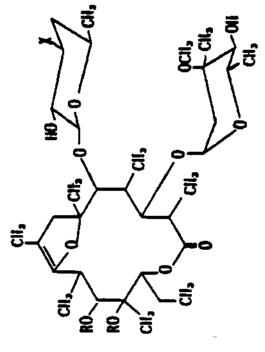

Table 14

| Compound No. | R | X | Specific rotatory power | NMR spectrum δ value ppm | | |
|---|---|---|---|---|---|---|
| | | | | 8-Me(s,3H) | 3″-OMe(s,3H) | Others (solvent) |
| 93 | H | $N(C_2H_5)_2$ | $(\alpha)_D^{22}$ -27.2° ($\underline{c}$ 1.0, $CHCl_3$) | 1.56 | 3.36 | ($CDCl_3$) |
| 94 | H | 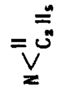 | $(\alpha)_D^{22}$ -34.8° ($\underline{c}$ 1.0, $CHCl_3$) | 1.56 | 3.35 | ($CDCl_3$) |
| 95 | H | 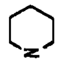 | $(\alpha)_D^{22}$ -33.8° ($\underline{c}$ 1.0, $CHCl_3$) | 1.56 | 3.35 | ($CDCl_3$) |
| 96 | H | $N^{\oplus}(C_2H_5)_3 \cdot I^{\ominus}$ | $(\alpha)_D^{22}$ -24.2° ($\underline{c}$ 1.0, $CH_3OH$) | 1.59 | 3.37 | ($CD_3OD$) |
| 97 | H | 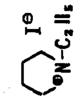 $I^{\ominus}$ | $(\alpha)_D^{22}$ -27.0° ($\underline{c}$ 1.0, $CH_3OH$) | 1.59 | 3.35 | ($CD_3OD$) |
| 98 | H | 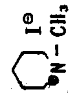 $I^{\ominus}$ | $(\alpha)_D^{22}$ -27.0° ($\underline{c}$ 1.0, $CH_3OH$) | 1.58 | 3.38 | 3.13 (3′-NMe, s, 3H) ($CD_3OD$) |
| 99 | H | 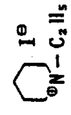 $I^{\ominus}$ | $(\alpha)_D^{22}$ -26.6° ($\underline{c}$ 1.0, $CH_3OH$) | 1.57 | 3.36 | ($CD_3OD$) |
| 100 | H | 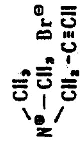 | $(\alpha)_D^{22}$ -31.0° ($\underline{c}$ 1.0, $CH_3OH$) | 1.58 | 3.39 | 3.26 (3′-$NMe_2$, s, 6H) ($CD_3OD$) |
| 101 | $COCH_3$ | 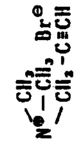 | $(\alpha)_D^{22}$ -20.0° ($\underline{c}$ 1.0, $CH_3OH$) | 1.51 | 3.39 | 2.01 (11-$COCH_3$, s, 3H) 2.04 (12-$COCH_3$, s, 3H) 3.27 (3′-$NMe_2$, s, 6H) ($CD_3OD$) |

Example 93

120 mg of 11-O-methylerythromycin A (reference: Japanese Laid-open Patent Publication No. 192294/1982) was dissolved in 6 ml of glacial acetic acid and the solution was stirred for one and a half hours. The reaction mixture was poured into 15 ml of ice-cooled conc. aqueous ammonia. This mixture was extracted 3 times with 10 ml of chloroform. This chloroform solution was dried over anhydrous sodium sulfate, and the solvent was evaporated under reduced pressure. The residue was purified by silica gel chromatography (developing solvent: chloroform-methanol-conc. aqueous ammonia (20 : 1 : 0.01)) to obtain

95 mg (yield 75%) of 11-O-methyl-8,9-anhydroerythromycin A 6,9-hemiketal (compound 102) in white powder.

Example 94

125 mg of 11-O-ethylerythromycin A (reference: Japanese Laid-open Patent Publication No. 192294/1982) was treated in the same manner as in Example 93 to obtain 102 mg (yield 84%) of 11-O-ethyl-8,9-anhydroerythromycin A 6,9-hemiketal (compound 103) in white powder.

Example 95

120 mg of the compound 48 was dissolved in 3.2 ml of chloroform, then added with 2 mg of 4-dimethylaminopyridine, 0.86 ml of triethylamine and 0.86 ml of propionic anhydride, and heated under reflux for 3 days. The reaction mixture was cooled to room temperature, and the same process as that for obtaining the compound 28 was conducted to obtain a pale yellow glass-like substance. This substance was dissolved, without purification, in 6 ml of methanol, and heated under reflux for 3 days. The solution was cooled to room temperature and concentrated under reduced pressure to obtain a pale yellow glass-like substance. This substance was purified by silica gel column chromatography, utilizing a developing solvent system of chloroform -methanol -conc. aqueous ammonia = 50 : 1 : 0.01, to obtain 65 mg (yield 55%) of 11-O-propionyl-12-O-acetyl-8,9-anhydroerythromycin A 6,9-hemiketal (compound 104) in white powder.

Rf value : 0.16 (chloroform : methanol : conc. aqueous ammonia = 10 : 1 : 0.01), low mass : $M^+$ 813, high mass : 813.486 (calcd. for $C_{42}H_{71}NO_{14}$ : 813.487).

Example 96

120 mg of the compound 48 was dissolved in 3.2 ml of chloroform, then added with 2 mg of 4-dimethylaminopyridine, 0.86 ml of triethylamine and 0.86 ml of butyric anhydride, and processed in the same manner as in the preparation of the compound 104 to obtain 75 mg (yield 63%) of 11-O-butyryl-12-O-acetyl-8,9-anhyroerythromycin A 6,9-hemiketal (compound 105) in white powder.

Rf value : 0.16 (chloroform -methanol -conc. aqueous ammonia = 10 : 1 : 0.01), low mass : $M^+$ 827, high mass : 827.502 (calcd. for $C_{43}H_{73}No_{14}$ : 827.502).

Example 97

100 mg of the compound 32 was dissolved in 1 ml of chloroform and heated under reflux for 2 days with addition of 0.5 ml of ethyl bromide. Thereafter, the same processing as in Example 50 was conducted to obtain 98 mg (yield 86%) of 11,12-di-O-acetyl-8,9-anhydroerythromycin A 6,9-hemiketal ethyl bromide (compound 106) in white powder.

Example 98

150 mg of the compound 27 was dissolved in 1 ml of chloroform, then 1 ml of methyl bromoacetate and 0.5 ml of diisopropylethylamine were added thereto and the mixture was stirred for 6 hours. After evaporation of the solvent, 5 ml of ether was added and the precipitate formed was filtered. The precipitate was washed with 10 ml of ether and dried to obtain 145 mg (yield 80%) of 8,9-anhydro-erythromycin A 6,9-hemiketal methoxycarbonyl methyl bromide (compound 107) in white powder.

Example 99

150 mg of the compound 27 was dissolved in 1 ml of chloroform, then 200 mg of bromoacetic acid and 0.5 ml of diisopropylethylamine were added thereto and the mixture was heated under reflux for 6 hours. After evaporation of the solvent, 5 ml of ether was added and the precipirate formed was filtered. The precipitate was washed with 10 ml of ether and dried to obtain 127 mg (yield 71%) of 8,9-anydroerythromycin A 6,9-hemiketal carboxymethyl bromide (compound 108) in white powder.

Example 100

150 mg of the compound 27 was dissolved in 1 ml of chloroform, then 0.5 ml of monofluoroethyl bromide was added thereto and the mixture was heated under reflux for 5 days. Subsequently, the same processing as in Example 75 was conducted to obtain 135 mg (yield 76%) of 8,9-anhydroerythromycin A 6,9-hemiketal 2-fluoroethyl bromide (compound 109) in white powder.

Example 101

150 mg of the compound 27 was dissolved in 1 ml of chloroform, then 0.5 ml of bromoacetonitrile was added thereto and the mixture was allowed to stand at room temperature for 5 hours. Subsequently, the same processing as in Example 75 was conducted to obtain 165 mg (yield 94%) of 8,9-anhydroerythromycin A 6,9-hemiketal cyanomethyl bromide (compound 110) in white powder.

The structural formulae, specific rotatory powers and NMR specturm values of the compounds obtained in Examples 93-101 are shown in Table 15.

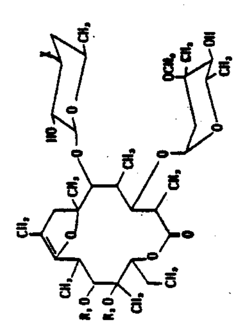

Table 15

| Compound No. | $R^1$ | $R^2$ | X | Specific rotatory power | NMR spectrum δ value ppm | | | |
|---|---|---|---|---|---|---|---|---|
| | | | | | 8-Me(s,3H) | 3'-$NMe_2$(s,6H) | 3'-OMe(s,3H) | Others (solvent) |
| 102 | $CH_3$ | H | $N(CH_3)_2$ | $[\alpha]_D^{24}$ −39.6° (c1.0, $CHCl_3$) | 1.56 | 2.29 | 3.36 | 11-OMe 3.49 (s,3H) ($CDCl_3$) |
| 103 | $C_2H_5$ | H | $N(CH_3)_2$ | $[\alpha]_D^{24}$ −29.8° (c1.0, $CHCl_3$) | 1.56 | 2.30 | 3.36 | ($CDCl_3$) |
| 104 | $COC_2H_5$ | $COCH_3$ | $N(CH_3)_2$ | $[\alpha]_D^{24}$ −20.2° (c1.0, $CHCl_3$) | 1.61 | 2.33 | 3.34 | 12-OAc 2.03 (s,3H) ($CDCl_3$) |
| 105 | $COC_3H_7$ | $COCH_3$ | $N(CH_3)_2$ | $[\alpha]_D^{24}$ −18.2° (c1.0, $CHCl_3$) | 1.58 | 2.29 | 3.32 | 12-OAc 2.00 (s,3H) ($CDCl_3$) |
| 106 | $COCH_3$ | $COCH_3$ | $N^{\oplus}(CH_3)_2-C_2H_5 \cdot Br^{\ominus}$ | $[\alpha]_D^{22}$ −26.4° (c1.0, $CH_3OH$) | 1.60 | 3.17 | 3.38 | 11-OAc 2.01 (s,3H) 12-OAc 2.05 ($CD_3OD$) |
| 107 | H | H | $N^{\oplus}(CH_3)_2-CH_2COOCH_3 \cdot Br^{\ominus}$ | $[\alpha]_D^{22}$ −30.0° (c1.0, $CH_3OH$) | 1.56 | 3.35 | 3.37 | ($CD_3OD$) |
| 108 | H | H | $N^{\oplus}(CH_3)_2-CH_2COOH \cdot Br^{\ominus}$ | $[\alpha]_D^{22}$ −31.8° (c1.0, $CH_3OH$) | 1.58 | 3.38 | 3.38 | ($CD_3OD$) |
| 109 | H | H | $N^{\oplus}(CH_3)_2-CH_2CH_2F \cdot Br^{\ominus}$ | $[\alpha]_D^{22}$ −26.0° (c1.0, $CH_3OH$) | 1.59 | 3.35 | 3.38 | ($CD_3OD$) |
| 110 | H | H | $N^{\oplus}(CH_3)_2-CH_2CN \cdot Br^{\ominus}$ | $[\alpha]_D^{22}$ −40.4° (c1.0, $CH_3OH$) | 1.58 | 3.35 | 3.38 | ($CD_3OD$) |

Example 102

By using 200 mg of 9-dihydroerythromycin A 6,9-epoxide (compound 84) (reference: Japanese Laid-open Patent Publication No. 1588/1972) and 0.5 ml of allyl bromide, the same processing as in Example 50 was conducted to obtain 190 mg of 9-dihydroerythromycin A 6,9-epoxide allyl bromide (compound 111) in

white powder.

Example 103

By using 200 mg of 9-dihydroerythromycin A 6,9-epoxide (compound 84) and 0.5 ml of propargyl bromide, the same processing as in Example 50 was conducted to obtain 195 mg (yield 84%) of 9-dihydroerythromycin A 6,9-epoxide propargyl bromide (compound 112) in white powder.

The structural formulae, specific rotatory powers and NMR spectrum values of the compounds obtained in Examples 102 and 103 are shown in Table 16.

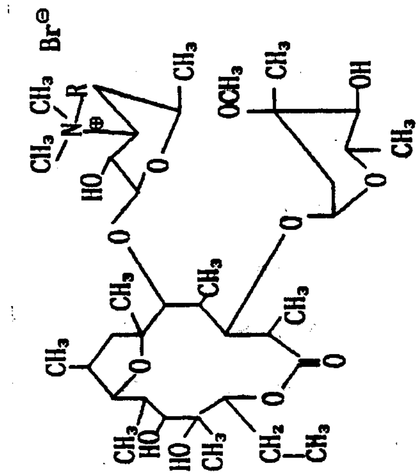

Table 16

| Compound No. | R | $[\alpha]_D^{22}$ (c 1.0, $CH_3OH$) | NMRspectrum δ value ppm ($CD_3OD$) 3′-NMe(s,6H) | 3″-OMe(s,3H) |
|---|---|---|---|---|
| 111 | $CH_2CH{=}CH_2$ | −38.4° | 3.16 | 3.36 |
| 112 | $CH_2C{\equiv}CH$ | −41.2° | 3.20 | 3.37 |

Example 104

505 mg of the compound 75 was dispersed in 5 ml of methanol, then 121 mg of sodium hydrogen carbonate and 68.5 µl of allyl bromide were added thereto, and the mixture thereof was stirred at 50 °C for

2 hours. This reaction mixture was diluted with 35 ml of ethyl acetate, and the solution was washed with a saturated aqueous sodium hydrogen carbonate and a saturated aqueous sodium chloride solution. The ethyl acetate solution was dried over anhydrous sodium sulfate and the solvent was evaporated under reduced pressure. The residue was purified by silica gel column chromatography (eluant chloroform-methanol-conc. aqueous ammonia (10 : 1 : 0.1)) to obtain 72 mg (yield 67%) of allyl-de(N-methyl)-8,9-anhydroerythromycin A 6,9-hemiketal (compound 113) in white powder.

Example 105

By using 105 mg of the compound 75, 25 mg of sodium hydrogen carbonate and 14.7 µl of propargyl bromide, the same processing as in Example 104 was conducted to obtain 66 mg (yield 60%) of propargyl-de(N-methyl)-8,9-anhydroerythromycin A 6,9-hemiketal (compound 114) in white powder.

Example 106

105 mg of the compound 75 was dispersed in 1 ml of methanol, then 0.29 ml of diisopropylethylamine and 0.29 ml of 1-iodopropane were added thereto, and the mixture thereof was stirred at 50 °C for 22 hours. This reaction mixture was diluted with 20 ml of ethyl acetate, and the solution was washed with a saturated aqueous sodium hydrogen carbonate and saturated aqueous sodium chloride solution. The ethyl acetate solution was dried over anhydrous sodium sulfate and the solvent was evaporated under reduced pressure. The residue was purified by silica gel column chromatography (eluant chloroform-methanol-conc. aqueous ammonia (50 : 1 : 0.1)) to obtain 84 mg (yield 75%) of propyl-de(N-methyl)-8,9-anhydroerythromycin A 6,9-hemiketal (compound 115) in white powder.

Example 107

By using 105 mg of the compound 75, 0.26 ml of diisopropylethylamine and 0.21 ml of bromoethanol, the same processing as in Example 106 was conducted to obtain 94 mg (yield 84%) of 2-hydroxyethyl-de-(N-methyl)-8,9-anhydroerythromycin A 6,9-hemiketal (compound 116) in white powder.

Example 108

By using 351 mg of the compound 75, 0.87 ml of diisopropylethylamine and 2 ml of 2-iodopropane, the same processing as in Example 106 was conducted to obtain 101 mg (yield 27%) of isopropyl-de(N-methyl)-8,9-anhydroerythromycin A 6,9-hemiketal (compound 117) in white powder.

Example 109

By using 351 mg of the compound 75, 0.87 ml of diisopropylethylamine and 2.2 ml of isobutyl bromide, the same processing as in Example 106 was conducted to obtain 52 mg (yield 14%) of isobutyl-de(N-methyl)-8,9-anhydroerythromycin A 6,9-hemiketal (compound 118) in white powder.

Example 110

1.0 g of the compound 76 was dissolved in 10 ml of methanol. To this, 2.5 ml of diisopropylethylamine and 1.3 ml of allyl bromide were added, and the mixture was stirred at 50 °C for 40 minutes. The solvent was removed under reduced pressure, and the residue was purified by silica gel column chromatography (eluant chloroform-methanol-conc. aqueous ammonia (50 : 1 : 0.01)) to obtain 337 mg (yield 30%) of dialkyl-bis-(de(N-methyl))-8,9-anhydroerythromycin A 6,9-hemiketal (compound 119) in white powder and 256 mg (yield 24%) of allyl-bis-(de(N-methyl))-8,9-anhydroerythromycin A 6,9-hemiketal (compound 120) in white powder.

Example 111

500 mg of the compound 76 was dissolved in 5 ml of methanol. To this were added 0.64 ml of diisopropylethylamine and 0.33 ml of propargyl bromide, and the mixture was stirred at 50 °C for 1 hour. The solvent was removed under reduced pressure, and the residue was purified by silica gel column chromatography (eluant chloroform-methanol-conc. aqueous ammonia (100 : 1 : 0.01)) to obtain 114 mg

(yield 21%) of dipropargyl-bis-(de(N-methyl))-8,9-anhydroerythromycin A 6,9-hemiketal (compound 121) in white powder and 252 mg (yield 45%) of propargyl-bis-(de(N-methyl))-8,9-anhydroerythromycin A 6,9-hemiketal (compound 122) in white powder.

Example 112

By using 256 mg of the compound 120, 0.61 ml of diisopropylethylamine and 0.31 ml of propargyl bromide, the same processing as in Example 106 was conducted to obtain 207 mg (yield 77%) of N-allyl-N-propargyl-bis-(de(N-methyl))-8,9-anhydroerythromycin A 6,9-hemiketal (compound 123) in white powder.

Example 113

By using 100 mg of the compound 113 and 0.1 ml of allyl bromide, the same processing as in Example 50 was conducted to obtain 110 mg (yield 94%) of allyl-de(N-methyl)-8,9-anhydroerythromycin A 6,9-hemiketal allyl bromide (compound 124) in white powder.

Example 114

By using 100 mg of the compound 113 and 0.1 ml of propargyl bromide, the same processing as in Example 50 was conducted to obtain 102 mg (yield 85%) of allyl-de(N-methyl)-8,9-anhydroerythromycin A 6,9-hemiketal propargyl bromide (compound 125) in white powder.

Example 115

By using 61 mg of the compound 114 and 0.1 ml of propargyl bromide, the same processing as in Example 50 was conducted to obtain 51 mg (yield 72%) of propargyl-de(N-methyl)-8,9-anhydroerythromycin A 6,9-hemiketal propargyl bromide (compound 126) in white powder.

Example 116

By using 99 mg of the compound 119 and 0.1 ml of allyl bromide, the same processing as in Example 50 was conducted to obtain 16 mg (yield 14%) of diallyl-bis-(de(N-methyl))-8,9-anhydroerythromycin A 6,9-hemiketal allyl bromide (compound 127) in white powder.

Example 117

61 mg of the compound 119 was dissolved 1 ml of methanol, then 12 mg of sodium hydrogen carbonate and 81.9 µl of propargyl bromide were added thereto, and the mixture was stirred at room temperature for 3 days. The same processing as in Example 50 was hereinafter conducted to obtain 32 mg (yield 39%) of diallyl-bis-(de(N-methyl))-8,9-anhydroerythromycin A 6,9-hemiketal propargyl bromide (compound 128) in white powder.

Example 118

By using 101 mg of the compound 122, 24 mg of sodium hydrogen carbonate and 0.1 ml of propargyl bromide, the same processing as in Example 117 was conducted to obtain 38 mg (yield 30%) of dipropargyl-bis-(de(N-methyl))-8,9-anhydroerythromycin A 6,9-hemiketal propargyl bromide (compound 129) in white powder.

Example 119

By using 50 mg of the compound 117 and 0.1 ml of iodomethane, the same processing as in Example 50 was conducted to obtain 52 mg (yield 86%) of 8,9-anhydroerythromycin A 6,9-hemiketal isopropyl iodide (compound 130) in white powder.

Example 120

By using 29 mg of the compound 118 and 0.4 ml of iodomethane, the same processing as in Example 50 was conducted to obtain 30 mg (yield 86%) of 8,9-anhydroerythromycin A 6,9-hemiketal isobutyl iodide (compound 131) in white powder.

Example 121

150 mg of the compound 27 was dissolved in 3 ml of chloroform, then 1 ml of butyl iodide was added thereto and the mixture was heated under reflux for 3 days. The same processing as in Example 50 was hereinafter conducted to obtain 121 mg (yield 64%) of 8,9-anhydroerythromycin A 6,9-hemiketal butyl iodide (compound 132) in white powder.

Example 122

150 mg of the compound 27 was dissolved in 2 ml of chloroform, then 0.3 ml of cyclopropylmethyl bromide was added thereto and the mixture was heated under reflux for 2 days. The same processing as in Example 50 was hereinafter conducted to obtain 145 mg (yield 81%) of 8,9-anhydroerythromycin A 6,9-hemiketal cyclopropylmethyl bromide (compound 133) in white powder.

Example 123

150 mg of the compound 27 was dissolved in 2 ml of chloroform, then 0.5 ml of crotyl bromide was added thereto and the mixture was allowed to stand at room temperature for 6 hours. The same processing as in Example 50 was hereinafter conducted to obtain 175 mg (yield 98%) of 8,9-anhydroerythromycin A 6,9-hemiketal crotyl bromide (compound 134) in white powder.

Example 124

150 mg of the compound 27 was dissolved in 1.5 ml of chloroform, then 0.5 ml of 2,3-dibromopropene was added thereto and the mixture was allowed to stand at room temperature for 1 day. The same processing as in Example 50 was hereinafter conducted to obtain 111 mg (yield 58%) of 8,9-anhydroerythromycin A 6,9-hemiketal 2-bromo allyl bromide (compound 135) in white powder.

Example 125

150 mg of the compound 27 was dissolved in 3 ml of chloroform, then 0.5 ml of propargyl chloride was added thereto and the mixture thereof was heated under reflux for 1 day. The same processing as in Example 50 was conducted to obtain 156 mg (yield 94%) of 8,9-anhydroerythromycin A 6,9-hemiketal propargyl chloride (compound 136) in white powder.

The structural formulae, specific rotatory powers and NMR spectrum values of the compounds obtained in Examples 104 - 125 are shown in Table 17.

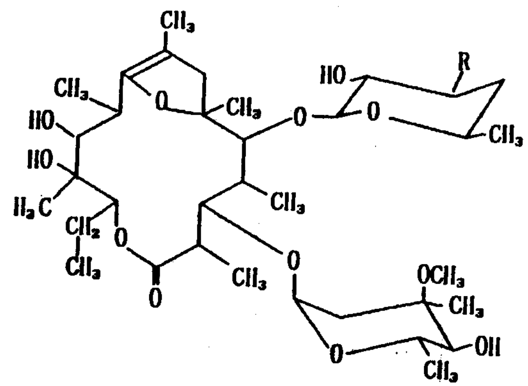

Table 17

| Compound No. | R | $[\alpha]_D^{24}$ (c 1.0) | NMR spectrum δ value ppm | | |
|---|---|---|---|---|---|
| | | | 8-Me (s, 3H) | 3"-OMe (s, 3H) | Others (solvent) |
| 113 | $CH_3$<br>N<br>$CH_2CH=CH_2$ | $-40.2^{\circ}$ ($CHCl_3$) | 1.56 | 3.33 | 2.19 (3'-NMe, s, 3H) ($CDCl_3$) |
| 114 | $CH_3$<br>N<br>$CH_2C\equiv CH$ | $-40.2^{\circ}$ ($CHCl_3$) | 1.57 | 3.36 | 2.35 (3'-NMe, s, 3H) ($CDCl_3$) |
| 115 | $CH_3$<br>N<br>$CH_2CH_2CH_3$ | $-36.2^{\circ}$ ($CHCl_3$) | 1.57 | 3.36 | 2.23 (3'-NMe, s, 3H) ($CDCl_3$) |
| 116 | $CH_3$<br>N<br>$CH_2CH_2OH$ | $-32.4^{\circ}$ ($CHCl_3$) | 1.57 | 3.35 | 2.34 (3'-NMe, s, 3H) ($CDCl_3$) |

| Compound No. | R | $[\alpha]_D^{24}$ (c 1.0) | NMR spectrum δ value ppm | | |
|---|---|---|---|---|---|
| | | | 8-Me (s, 3H) | 3"-OMe (s, 3H) | Others (solvent) |
| 117 | 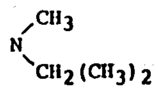 | -36.8° ($CHCl_3$) | 1.56 | 3.36 | 2.21 (3'-NMe, s, 3H) ($CDCl_3$) |
| 118 | 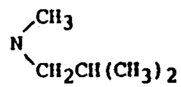 | -36.6° ($CHCl_3$) | 1.57 | 3.36 | 2.22 (3'-NMe, s, 3H) ($CDCl_3$) |
| 119 | $N-(CH_2CH=CH_2)_2$ | -39.6° ($CHCl_3$) | 1.56 | 3.32 | ($CDCl_3$) |
| 120 | 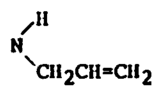 | -31.4° ($CHCl_3$) | 1.57 | 3.35 | ($CDCl_3$) |
| 121 | $N-(CH_2C\equiv CH)_2$ | -28.4° ($CHCl_3$) | 1.57 | 3.36 | ($CDCl_3$) |
| 122 | 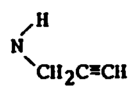 | -32.2° ($CHCl_3$) | 1.57 | 3.36 | ($CDCl_3$) |
| 123 | 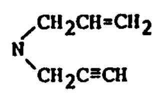 | -31.2° ($CHCl_3$) | 1.57 | 3.36 | ($CDCl_3$) |
| 124 | 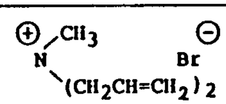 | -24.4° ($CH_3OH$) | 1.59 | 3.36 | 3.07 (3'-NMe, s, 3H) ($CD_3OD$) |

| Compound No. | R | $[\alpha]_D^{24}$ (c 1.0) | NMR spectrum δ value ppm | | |
|---|---|---|---|---|---|
| | | | 8-Me (s, 3H) | 3"-OMe (s, 3H) | Others (solvent) |
| 125 | $\oplus N(CH_3)(CH_2CH{=}CH_2)(CH_2C{\equiv}CH)\ Br^{\ominus}$ | -23.8° ($CH_3OH$) | 1.59 | 3.39 | 3.17 (3'-NMe, s, 3H) ($CD_3OD$) |
| 126 | $\oplus N(CH_3)(CH_2C{\equiv}CH)_2\ Br^{\ominus}$ | -20.5° ($CH_3OH$) | 1.59 | 3.40 | 3.29 (3'-NMe, s, 3H) ($CD_3OD$) |
| 127 | $\oplus N(CH_2CH{=}CH_2)_3Br^{\ominus}$ | -13.3° ($CH_3OH$) | 1.58 | 3.34 | ($CD_3OD$) |
| 128 | $\oplus N(CH_2C{\equiv}CH)(CH_2CH{=}CH_2)_2\ Br^{\ominus}$ | -18.0° ($CH_3OH$) | 1.59 | 3.36 | ($CD_3OD$) |
| 129 | $\oplus N(CH_2C{\equiv}CH)_3\ Br^{\ominus}$ | -18.4° ($CH_3OH$) | 1.58 | 3.39 | ($CD_3OD$) |
| 130 | $\oplus N(CH_3)_2(CH(CH_3)_2)\ I^{\ominus}$ | -26.4° ($CH_3OH$) | 1.58 | 3.36 | 2.90 (3'-$NMe_2$, s, 6H) ($CD_3OD$) |
| 131 | $\oplus N(CH_3)_2(CH_2CH(CH_3)_2)\ I^{\ominus}$ | -26.0° ($CH_3OH$) | 1.59 | 3.38 | 3.19 (3'-$NMe_2$, s, 6H) ($CD_3OD$) |

| Compound No. | R | $[\alpha]_D^{24}$ (c 1.0) | NMR spectrum δ value ppm | | |
|---|---|---|---|---|---|
| | | | 8-Me (s, 3H) | 3"-OMe (s, 3H) | Others (solvent) |
| 132 | $\oplus N(CH_3)_2(CH_2CH_2CH_2CH_3)$ $I^{\ominus}$ | -29.4° ($CH_3OH$) | 1.59 | 3.39 | 3.22 (3'-$NMe_2$, s, 6H) ($CD_3OD$) |
| 133 | $\oplus N(CH_3)_2(CH_2$-◁$)$ $Br^{\ominus}$ | -24.4° ($CH_3OH$) | 1.58 | 3.37 | 3.24 (3'-$NMe_2$, s, 6H) ($CD_3OD$) |
| 134 | $\oplus N(CH_3)_2(CH_2CH{=}CHCH_3)$ $Br^{\ominus}$ | -29.6° ($CH_3OH$) | 1.58 | 3.38 | 3.13 (3'-$NMe_2$, s, 6H) ($CD_3OD$) |
| 135 | $\oplus N(CH_3)_2(CH_2CBr{=}CH_2)$ $Br^{\ominus}$ | -26.2° ($CH_3OH$) | 1.58 | 3.34 | 3.34 (3'-$NMe_2$, s, 6H) ($CD_3OD$) |
| 136 | $\oplus N(CH_3)_2(CH_2C{\equiv}CH)$ $Cl^{\ominus}$ | -27.8° ($CH_3OH$) | 1.58 | 3.39 | 3.26 (3'-$NMe_2$, s, 6H) ($CD_3OD$) |

Example 126

73.5 mg of the compound 32 was dissolved in 0.8 ml of methanol, and 0.2 ml of water was added thereto, followed by addition of 66.4 mg of $CH_3COONa \cdot 3H_2O$. The reaction mixture was heated at 50°C, and stirred after 26 mg of iodine was added thereto. In order to maintain the pH of the reaction mixture at 8 to 9, 0.4 ml portions of 1N aqueous sodium hydroxide solution were added thereto after 10 minutes, 30

minutes and 1 hour, respectively, and the stirring was further continued for 1 hour. The solution was thereafter poured into 100 ml of dilute aqueous ammonia and the resultant product was extracted with chloroform. The extract was washed with dilute aqueous ammonia and dried with anhydrous sodium sulfate. Thereafter, the solvent was distilled off under reduced pressure. The residue was purified by silica gel column chromatography (eluant: chloroform-methanol-conc. aqueous ammonia (15 : 1 : 0.1)) to obtain 51 mg (yield 70%) of 11,12-di-O-acetyl-de(N-methyl)-8,9-anhydroerythromycin A 6,9-hemiketal (compound 137) in white powder.

Example 127

By using 79 mg of the compound 137, 0.17 ml of diisopropylethylamine and 0.16 ml of iodomethane, the same processing as in Example 106 was conducted to obtain 30 mg (yield 37%) of 11,12-di-O-acetyl-N-ethyl-de(N-methyl)-8,9-anhydroerythromycin A 6,9-hemiketal (compound 138) in white powder.

Example 128

By using 500 mg of the compound 20, 468 mg of $CH_3COONa\cdot3H_2O$ and 170 mg of iodine, the same processing as in Example 126 was conducted to obtain 413 mg (yield 84%) of de(N-methyl)-8,9-anhydroerythromycin A 6,9-hemiketal cyclic 11,12-carbonate (compound 139) in white powder.

Example 129

By using 350 mg of the compound 139, 0.84 ml of diisopropylethylamine and 0.77 ml of iodoethane, the same processing as in Example 106 was conducted to obtain 254 mg (yield 69%) of N-ethyl-de(N-methyl)-8,9-anhydroerythromycin A 6,9-hemiketal cyclic 11,12-carbonate (compound 140) in white powder.

Example 130

By using 24.8 mg of 8,9-anhydroerythromycin B 6,9-hemiketal (reference: P. Kurath, et al., Experientia, 27, 362, 1971) and 0.2 ml of bromoethane, the same processing as in Example 97 was conducted to obtain 20 mg (yield 69%) of 8,9-anhydroerythromycin B 6,9-hemiketal ethyl bromide (compound 141) in white powder.

Example 131

By using 24.7 mg of 8,9-anhydroerythromycin B 6,9-hemiketal and 0.05 ml of propargyl bromide, the same processing as in Example 50 was conducted to obtain 24 mg (yield 83%) of 8,9-anhydroerythromycin B 6,9-hemiketal propargyl bromide (compound 142) in white powder.

Example 132

By using 50 mg of the compound 54 and 0.3 ml of propargyl bromide, the same processing as in Example 50 was conducted to obtain 54 mg (yield 93%) of 11,12-O-isopropylidene-8,9-anhydroerythromycin A 6,9-hemiketal propargyl bromide (compound 143) in white powder.

Example 133

By using 50 mg of the compound 39 and 0.3 ml of propargyl bromide, the same processing as in Example 50 was conducted to obtain 55 mg (yield 96%) of 8,9-anhydroerythromycin A 6,9-hemiketal 11,12-phenylboronate propargyl bromide (compound 144) in white powder.

Example 134

By using 100 mg of the compound 20 and 0.3 ml of propargyl bromide, the same processing as in Example 50 was conducted to obtain 108 mg (yield 93%) of 8,9-anhydroerythromycin A 6,9-hemiketal 11,12-cyclic carbonate propargyl bromide (compound 145) in white powder.

Example 135

By using 100 mg of the compound 37 and 0.3 ml of propargyl bromide, the same processing as in Example 50 was conducted to obtain 107 mg (yield 93%) of 8,9-anhydroerythromycin A 6,9-hemiketal 11,12-sulfite propargyl bromide (compound 146) in white powder.

Example 136

100 mg of the compound 8 was dissolved in 2 ml of dry dimethyl sulfoxide, and to this, were added with 1 ml of acetic anhydride and 0.3 ml of acetic acid. The reaction mixture was allowed to stand for 1 day at room temperature. Thereafter, the same processing as in Example 39 was conducted to obtain 65 mg (yield 56%) of 2'-O-acetyl-4''-O-formyl-11,12-di-O-methylthiomethyl-8,9-anhydroerythromycin A 6,9-hemiketal (compound 147) in white powder.

Example 137

150 mg of the compound 147 was dissolved in 6 ml of methanol, and to this, was added with 1 ml of conc. aqueous ammonia. The reaction mixture was heated, for 2 days under reflux. Thereafter, the same processing as in Example 40 was conducted to obtain 105 mg (yield 76%) of 11,12-di-O-methylthiomethyl-8,9-anhydroerythromycin A 6,9-hemiketal (compound 148) in white powder.

Example 138

By using 100 mg of the compound 148 and 0.2 ml of propargyl bromide, the same processing as in Example 50 was conducted to obtain 98 mg (yield 86%) of 11,12-di-O-methylthiomethyl-8,9-anhydroerythromycin A 6,9-hemiketal propargyl bromide (compound 149) in white powder.

Example 139

99 mg of the compound 1 was dissolved in 3 ml of chloroform, then 0.5 ml of propargyl bromide was added thereto and the mixture was allowed to stand at room temperature for 3 hours. The same processing as in Example 50 was hereinafter conducted to obtain 76 mg (yield 66%) of 2'-O-acetyl-8,9-anhydroerythromycin A 6,9-hemiketal propargyl bromide (compound 150) in white powder.

The structural formulae, specific rotatory powers and NMR spectrum values of the compounds obtained in Examples 126 - 139 are shown in Table 18.

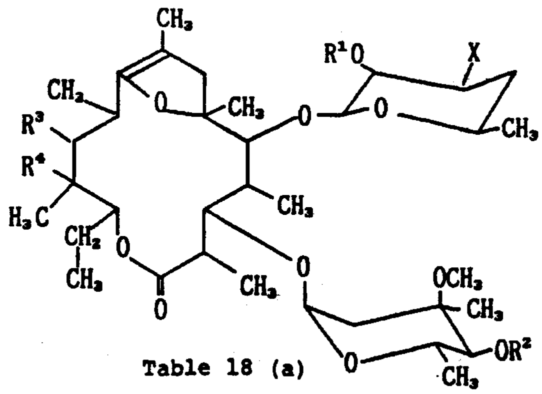

Table 18 (a)

| No. | $R^1$ | $R^2$ | $R^3$ | $R^4$ | X | $[\alpha]_D^{24}$ (c 1.0) |
|---|---|---|---|---|---|---|
| 137 | H | H | OAc | OAc | 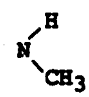 | -17.0° ($CHCl_3$) |
| 138 | H | H | OAc | OAc | 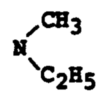 | -11.8° ($CHCl_3$) |
| 139 | H | H | 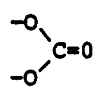 | | 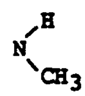 | -30.0° ($CHCl_3$) |
| 140 | H | H | 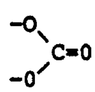 | | 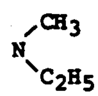 | -30.8° ($CHCl_3$) |
| 141 | H | H | OH | H | 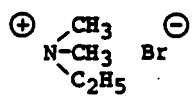 | -18.8° ($CH_3OH$) |
| 142 | H | H | OH | H | 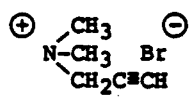 | -23.6° ($CH_3OH$) |
| 143 | H | H | 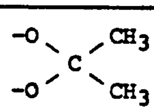 | | 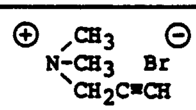 | -23.2° ($CH_3OH$) |
| 144 | H | H | 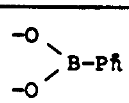 | | 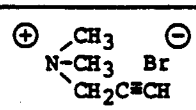 | -55.4° ($CH_3OH$) |
| 145 | H | H | 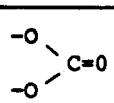 | | 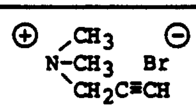 | -29.6° ($CH_3OH$) |

Table 18 (a)

| No. | $R^1$ | $R^2$ | $R^3$ | $R^4$ | X | $[\alpha]_D^{24}$ (c 1.0) |
|---|---|---|---|---|---|---|
| 146 | H | H | 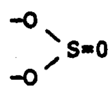 | | 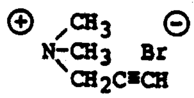 | -31.4°C ($CH_3OH$) |
| 147 | Ac | CHO | $OCH_2SCH_3$ | $OCH_2SCH_3$ | $N(CH_3)_2$ | -37.2° ($CHCl_3$) |
| 148 | H | H | $OCH_2SCH_3$ | $OCH_2SCH_3$ | $N(CH_3)_2$ | -34.6° ($CHCl_3$) |
| 149 | H | H | $OCH_2SCH_3$ | $OCH_2SCH_3$ | 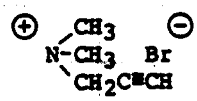 | -32.2° ($CH_3OH$) |
| 150 | Ac | H | OH | OH | 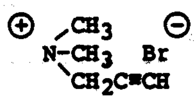 | -41.2° ($CH_3OH$) |

Table 18 (b)

| Compound No. | NMR spectrum δ value ppm | | |
|---|---|---|---|
| | 8-Me (s, 3H) | 3"-OMe (s, 3H) | Others (solvent) |
| 137 | 1.59 | 3.34 | 1.99 (OAc, s, 3H), 2.03 (OAc, s, 3H)<br>2.42 (3'-NMe, s, 3H) ($CDCl_3$) |
| 138 | 1.60 | 3.34 | 1.99 (OAc, s, 3H), 2.03 (OAc, s, 3H)<br>2.23 (3'-NMe , s, 3H) ($CDCl_3$) |
| 139 | 1.62 | 3.35 | 2.42 (3'-NMe, s, 3H) ($CDCl_3$) |
| 140 | 1.61 | 3.35 | 2.23 (3'-NMe , s, 3H) ($CDCl_3$) |
| 141 | 1.58 | 3.38 | 3.14 (3'-$NMe_2$, s, 6H) ($CD_3OD$) |
| 142 | 1.58 | 3.39 | 3.25 (3'-$NMe_2$, s, 6H) ($CD_3OD$) |
| 143 | 1.62 | 3.39 | 1.38 (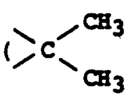 , s, 6H) ($CD_3OD$)<br>3.27 (3'-$NMe_2$, s, 6H) |
| 144 | 1.62 | 3.39 | 3.28 (3'-$NMe_2$, s, 6H)<br>($CD_3OD$)<br>7.3 - 7.8 (Ph, m, 5H) |
| 145 | 1.61 | 3.53 | 3.37 (3'-$NMe_2$, s, 6H) ($CDCl_3$) |
| 146 | 1.57 | 3.39 | 3.39 (3'-$NMe_2$, s, 6H) ($CD_3OD$) |
| 147 | 1.58 | 3.36 | 2.04 (2'-OAc, s, 3H),<br>2.27 (3'-$NMe_2$, s, 6H), ($CDCl_3$)<br>8.19 (4"-CHO, s, 1H) |
| 148 | 1.58 | 3.35 | 2.22 (11-$SCH_3$, s, 3H),<br>2.24 (12-$SCH_3$, s, 3H), ($CDCl_3$)<br>2.29 (3'-$NMe_2$, s, 6H) |

Table 18 (b)

| Compound No. | NMR spectrum δ value ppm | | |
|---|---|---|---|
| | 8-Me (s, 3H) | 3"-OMe (s, 3H) | Others (solvent) |
| 149 | 1.58 | 3.39 | 2.22 ($SCH_3$, s, 6H), 3.25 (3'-$NMe_2$, s, 6H) ($CD_3OD$) |
| 150 | 1.56 | 3.38 | 2.20 (2'-OAc, s, 3H), 3.32 (3'-$NMe_2$, s, 6H) ($CD_3OD$) |

Example 140

150 mg of the compound 84 was dissolved in 3 ml of chloroform, then 0.5 ml of propargyl chloride was added thereto and the mixture thereof was heated under reflux for 1 day. Thereafter, the same processing as in Example 50 was conducted to obtain 142 mg (yield 86%) of 9-dihydroerythromycin A 6,9-epoxide propargyl chloride (compound 151) in white powder.

Example 141

By using 143 mg of the compound 84, 27 µl of acetic anhydride and 31 µl of pyridine, the same processing as in Example 23 was conducted to obtain 125 mg (yield 83%) of 2'-O-acetyl-9-dihydroerythromycin A 6,9-epoxide (compound 152) in white powder.

Example 142

150 mg of the compound 84 was dissolved in 3 ml of chloroform, then 0.5 ml of benzyl chloride was added thereto and the mixture was heated under reflux for 38 hours. Thereafter, the same processing as in Example 50 was conducted to obtain 155 mg (yield 81%) of 9-dihydroerythromycin A 6,9-epoxide benzyl chloride (compound 153) in white powder.

Example 143

150 mg of the compound 84 was dissolved in 3 ml of chloroform, then 0.5 ml of 1-bromo-2-fluoroethane was added thereto, and the mixture was heated under reflux for 7 days. Thereafter, the same processing as in Example 50 was conducted to obtain 66 mg (yield 37%) of 9-dihydroerythromycin A 6,9-epoxide 2-fluoroethyl bromide (compound 154) in pale yellow powder.

Example 144

150 mg of the compound 84 was dissolved in 3 ml of chloroform, then 0.5 ml of cyclopropylmethyl bromide was added thereto and the mixture was heated under reflux for 38 hours. Thereafter, the same processing as in Example 50 was conducted to obtain 153 mg (yield 86%) of 9-dihydroerythromycin A 6,9-epoxide cyclopropylmethyl bromide (compound 155) in white powder.

Example 145

150 mg of the compound 84 was dissolved in 3 ml of chloroform, then 0.5 ml of 3-butenyl bromide was added thereto, and the mixture was heated under reflux for 38 hours. Thereafter, the same processing as in Example 50 was conducted to obtain 113 mg (yield 63%) of 9-dihydroerythromycin A 6,9-epoxide 3-butenyl bromide (compound 156) in white powder.

Example 146

125 mg of the compound 152 was dissolved in 3 ml of chloroform, then 0.5 ml of propargyl bromide was added thereto and the mixture thereof was allowed to stand at room temperature for 3 hours. Thereafter, the same processing as in Example 50 was conducted to obtain 114 mg (yield 79%) of 2'-O-acetyl-9-dihydroerythromycin A 6,9-epoxide propargyl bromide (compound 157) in white powder.

The structural formulae, specific rotatory powers and NMR spectrum values of the compounds obtained in Examples 140 - 146 are shown in Table 19.

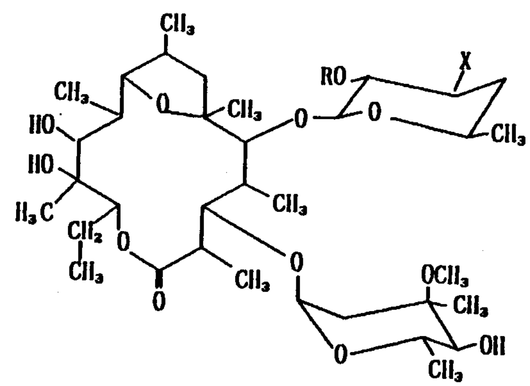

Table 19

| Compound No. | R | X | $[\alpha]_D^{24}$ (c 1.0, $CH_3OH$) | NMR spectrum δ value ppm ($CD_3OD$) | | |
|---|---|---|---|---|---|---|
| | | | | 3'-$NMe_2$ (s, 6H) | 3"-OMe (s, 3H) | Others |
| 151 | H | $\oplus N(CH_3)_2CH_2C\equiv CH\ Cl^{\ominus}$ | -44.4° | 3.20 | 3.37 | |
| 152 | Ac | $N(CH_3)_2$ | -53.6° | 2.28 | 3.35 | 2.07 (2'-OAc, s, 3H) ($CDCl_3$) |
| 153 | H | $\oplus N(CH_3)_2CH_2Ph\ Cl^{\ominus}$ | -47.4° | 3.12 | 3.33 | |
| 154 | H | $\oplus N(CH_3)_2CH_2CH_2F\ Br^{\ominus}$ | -38.4° | 3.25 | 3.36 | |

| Compound No. | R | X | $[\alpha]_D^{24}$ ($\underline{c}$ 1.0, $CH_3OH$) | NMR spectrum δ value ppm ($CD_3OD$) 3'-$NMe_2$ (s, 6H) | 3"-OMe (s, 3H) | Others |
|---|---|---|---|---|---|---|
| 155 | H | 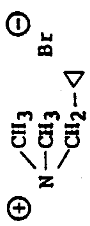 | -37.0° | 3.23 | 3.36 | |
| 156 | H | 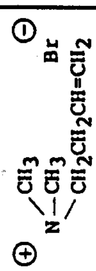 | -37.6° | 3.13 | 3.37 | |
| 157 | Ac | 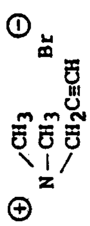 | -52.0° | 3.23 | 3.36 | 2.21 (2'-OAc, s, 3H) |

## Example 147

By using 64 mg of 6-O-methylerythromycin A (reference: S. Morimoto et al., J. Antibiotics, 37, 187, 1984) and 0.1 ml of propargyl bromide, the same processing as in Example 50 was conducted to obtain 73

mg (yield 98%) of 6-O-methylerythromycinA propargyl bromide (compound 158) in white powder.

Example 148

200 mg of erythromycin A was dissolved in 3 ml of chloroform, then 0.3 ml of ethyl iodide was added thereto and the mixture was heated under reflux for 20 hours. Thereafter, the same processing as in Example 54 was conducted to obtain 150 mg (yield 62%) of erythromycin A ethyl iodide (compound 159) in pale yellow powder.

Example 149

100 mg of erythromycin A was dissolved in 2 ml of chloroform, then 0.2 ml of allyl bromide was added thereto, and the mixture was stirred at room temperature for 5 hours. Thereafter, the same processing as in Example 50 was conducted to obtain 97 mg (yield 83%) of erythromycin A allyl bromide (compound 160) in white powder.

Example 150

200 mg of erythromycin A was dissolved in 3 ml of chloroform, then 0.2 ml of propargyl bromide was added thereto and the mixture was stirred at room temperature for 3 hours. Thereafter, the same processing as in Example 54 was conducted to obtain 202 mg (yield 87%) of erythromycin A propargyl bromide (compound 161) in white powder.

The structural formulae, specific rotatory powers and NMR spectrum values of the compounds obtained in Examples 147 - 150 are shown in Table 20.

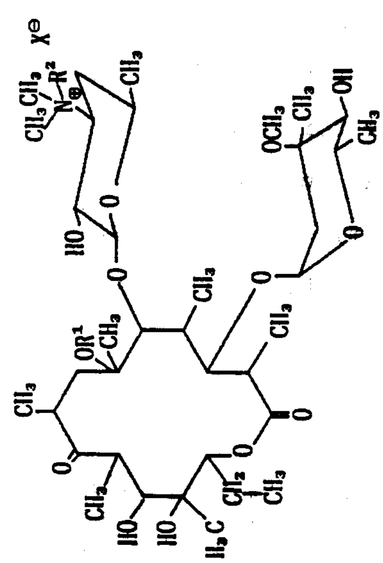

Table 20

| Compound No. | $R_1$ | $R_2$ | X | $[\alpha]^{24}_D$ (c 1.0, $CH_3OH$) | NMR spectrum δ value ppm ($CD_3OD$) 3'-$NMe_2$ (s, 6H) | 3"-OMe (s, 3H) | Others |
|---|---|---|---|---|---|---|---|
| 158 | $CH_3$ | $CH_2C{\equiv}CH$ | Br | -77.4° | 3.26 | 3.36 | 3.04 (6-OMe, s, 3H) |
| 159 | H | $C_2H_5$ | I | -43.6° | 3.20 | 3.35 | |
| 160 | H | $CH_2CH{=}CH_2$ | Br | -50.4° | 3.12 | 3.35 | |
| 161 | H | $CH_2C{\equiv}CH$ | Br | -54.6° | 3.27 | 3.36 | |

Example 151

50 mg of the compound 9 was dissolved in 1 ml of chloroform, then 0.2 ml of methyl iodide was added thereto and the mixture was stirred at room temperature for 3 hours. Thereafter, the same processing as in

Example 50 was conducted to obtain 49 mg (yield 83%) of 4''-O-formyl-8,9-anhydroerythromycin A 6,9-hemiketal methyl iodide (compound 162) in pale yellow powder.

Example 152

50 mg of the compound 9 was dissolved in 2 ml of chloroform, then 0.5 ml of ethyl iodide was added thereto and the mixture was heated under reflux for 20 hours. Subsequently, the same processing as in Example 50 was conducted to obtain 38 mg (yield 13%) of 4''-O-formyl-8,9-anhydroerythromycinA 6,9-hemiketal ethyl iodide (compound 163) in pale yellow powder.

Example 153

50 mg of the compound 9 was dissolved in 2 ml of chloroform, then 0.5 ml of propyl iodide was added thereto and the mixture was heated under reflux for 48 hours. Subsequently, the same processing as in Example 50 was conducted to obtain 34 mg (yield 56%) of 4''-O-formyl-8,9-anhydroerythromycinA 6,9-hemiketal propyl iodide (compound 164) in pale yellow powder.

Example 154

50 mg of the compound 9 was dissolved in 1 ml of chloroform, then 0.2 ml of propargyl bromide was added thereto and the mixture was stirred at room temperature for 3 hours. Subsequently, the same processing as in Example 50 was conducted to obtain 51 mg (yield 87%) of 4''-O-formyl-8,9-anhydroerythromycinA 6,9-hemiketal propargyl bromide (compound 165) in white powder.

Example 155

50 mg of the compound 9 was dissolved in 1 ml of choloroform, then 0.2 ml of allyl bromide was added thereto and the mixture was stirred at room temperature for 5 hours. Subsequently, the same processing as in Example 50 was conducted to obtain 47 mg (yield 80%) of 4''-O-formyl-8,9-anhydroerythromycinA 6,9-hemiketal allyl bromide (compound 166) in white powder.

Example 156

50 mg of the compound 50 was processed in the same manner as in Example 151 to obtain 50 mg (yield 84%) of 11-O-acetyl-8,9-anhydroerythromycin A 6,9-hemiketal methyl iodide (compound 167) in pale yellow powder.

Example 157

50 mg of the compound 50 was processed in the same manner as in Example 152 to obtain 39 mg (yield 65%) of 11-O-acetyl-8,9-anhydroerythromycin A 6,9-hemiketal ethyl iodide (compound 168) in pale yellow powder.

Example 158

50 mg of the compound 50 was processed in the same manner as in Example 153 to obtain 33 mg (yield 54%) of 11-O-acetyl-8,9-anhydroerythromycin A 6,9-hemiketal propyl iodide (compound 169) in pale yellow powder.

Example 159

50 mg of the compound 50 was processed in the same manner as in Example 154 to obtain 49 mg (yield 84%) of 11-O-acetyl-8,9-anhydroerythromycin A 6,9-hemiketal propargyl bromide (compound 170) in white powder.

Example 160

50 mg of the compound 50 was processed in the same manner as in Example 155 to obtain 46 mg (yield 79%) of 11-O-acetyl-8,9-anhydroerythromycin A 6,9-hemiketal allyl bromide (compound 171) in white powder.

Example 161

50 mg of the compound 25 was dissolved in 1 ml of chloroform, then 0.2 ml of propargyl bromide was added thereto and the mixture was stirred at room temperature for 3 hours. Thereafter, the same processing as in Example 50 was conducted to obtain 44 mg (yield 77%) of 4''-O-formyl-11-O-mesyl-8,9-anhydroerythromycin A 6,9-hemiketal propargyl bromide (compound 172) in white powder.

Example 162

50 mg of the compound 57 was dissolved in 2 ml of chloroform, then 0.3 ml of ethyl iodide was added thereto and the mixture thereof was heated under reflux for 20 hours. Subsequently, the same processing as in Example 50 was conducted to obtain 39 mg (yield 66%) of 11-O-mesyl-8,9-anhydroerythromycin A 6,9-hemiketal ethyl iodide (compound 173) in pale yellow powder.

Example 163

50 mg of the compound 57 was dissolved in 2 ml of chloroform, then 0.3 ml of propyl iodide was added thereto and the mixture was heated under reflux for 48 hours. Subsequently, the same processing as in Example 54 was conducted to obtain 34 mg (yield 56%) of 11-O-mesyl-8,9-anhydroerythromycin A 6,9-hemiketal propyl iodide (compound 174) in pale yellow powder.

The structural formulae, specific rotatory powers and NMR spectrum values of the compounds obtained in Examples 151 - 163 are shown in Table 21.

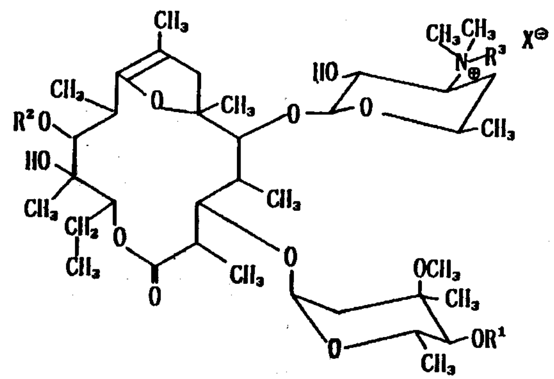

Table 21

| Compound No. | $R_1$ | $R_2$ | $R_3$ | X | $[\alpha]_D^{24}$ (c 1.0, $CH_3OH$) | NMR spectrum δ value ppm ($CD_3OD$) 8-$CH_3$(s, 3H) | 3'-$NMe_2$(s, 6H) | 3"-OMe (s, 3H) | Others |
|---|---|---|---|---|---|---|---|---|---|
| 162 | CHO | H | $CH_3$ | I | -31.4° | 1.59 | 3.31 | 3.41 | 8.33 (4"-OCHO, s, 1H) |
| 163 | CHO | H | $C_2H_5$ | I | -31.8° | 1.59 | 3.19 | 3.41 | 8.31 (4"-OCHO, s, 1H) |
| 164 | CHO | H | $C_3H_7$ | I | -30.4° | 1.59 | 3.20 | 3.40 | 8.31 (4"-OCHO, s, 1H) |
| 165 | CHO | H | $CH_2C{\equiv}CH$ | Br | -34.4° | 1.59 | 3.31 | 3.41 | 8.31 (4"-OCHO, s, 1H) |
| 166 | CHO | H | $CH_2CH{=}CH_2$ | Br | -32.8° | 1.59 | 3.18 | 3.38 | 8.29 (4"-OCHO, s, 1H) |

| Compound No. | $R_1$ | $R_2$ | $R_3$ | X | $[\alpha]_D^{24}$ (c 1.0, $CH_3OH$) | NMR spectrum δ value ppm ($CD_3OD$) | | | |
|---|---|---|---|---|---|---|---|---|---|
| | | | | | | 8-$CH_3$(s, 3H) | 3'-$NMe_2$(s, 6H) | 3"-OMe (s, 3H) | Others |
| 167 | H | $COCH_3$ | $CH_3$ | I | -12.0° | 1.60 | 3.29 | 3.38 | 2.11 (11-OAc, s, 3H) |
| 168 | H | $COCH_3$ | $C_2H_5$ | I | -10.0° | 1.60 | 3.15 | 3.38 | 2.10 (11-OAc, s, 3H) |
| 169 | H | $COCH_3$ | $C_3H_7$ | I | -13.4° | 1.60 | 3.19 | 3.38 | 2.10 (11-OAc, s, 3H) |
| 170 | H | $COCH_3$ | $CH_2C{\equiv}CH$ | Br | -14.6° | 1.60 | 3.27 | 3.39 | 2.11 (11-OAc, s, 3H) |
| 171 | H | $COCH_3$ | $CH_2CH{=}CH_2$ | Br | -12.4° | 1.60 | 3.14 | 3.37 | 2.10 (11-OAc, s, 3H) |
| 172 | CHO | $SO_2CH_3$ | $CH_2C{=}CH$ | Br | -28.8° | 1.61 | 3.25 | 3.41 | 3.30 (10-OMs, s, 3H)<br>8.30 (4"-OCHO, s, 1H) |
| 173 | H | $SO_2CH_3$ | $C_2H_5$ | I | -26.4° | 1.60 | 3.17 | 3.37 | 3.24 (11-OMs, s, 3H) |
| 174 | H | $SO_2CH_3$ | $C_3H_7$ | I | -27.8° | 1.60 | 3.18 | 3.37 | 3.24 (11-OMs, s, 3H) |

As hereinbefore described, the compound (1) of the present invention has an excellent effect of stimulating the gastrointestinal contractive motion, and the preparation containing this compound can be advantageously used as a digestive tract contractive motion stimulant.

**Claims**

**Claims for the following Contracting States : BE, DE, FR, IT, LU, NL, SE, CH, GB, LI**

**1.** A compound of the formula:

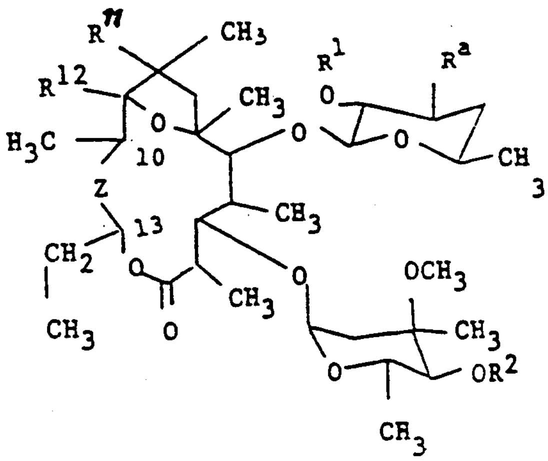

[I]

wherein $R^1$ is hydrogen;
$R^2$ is hydrogen;
Z is a group of the formula

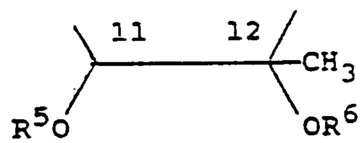

wherein $R^5$ is hydrogen and $R^6$ is hydrogen;
$R^a$ is a group of the formula

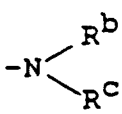

in which $R^b$ is methyl, and $R^c$ is ethyl or isopropyl,
or $R^a$ is a group of the formula

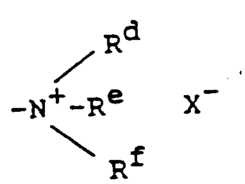

in which $R^d$ is methyl, and $R^e$ and $R^f$ which may be the same or different, are (1) a methyl, ethyl or isopropyl radical which may be substituted by hydroxyl, cyano, halogen or cylopropyl, or (2) a propargyl radical,
or together $R^e$ and $R^f$ form pyrrolidino or piperidino with the adjacent nitrogen atom;
X is a halogen anion;
and
$R^{11}$ and $R^{12}$ both taken together form a chemical bond,
or a salt thereof.

2. A compound according to claim 1, wherein $R^a$ is N-methyl-N-ethylamino radical.

3. A compound according to claim 1, wherein $R^a$ is a group of the formula:

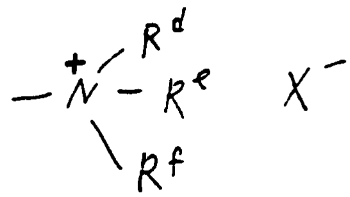

wherein $R^d$ is methyl, and $R^e$ and $R^f$ which may be the same or different, are (1) a methyl, ethyl or isopropyl radical which may be substituted by hydroxyl, cyano, halogen or cylopropyl, or (2) a propargyl radical,
or together $R^e$ and $R^f$ form pyrrolidino or piperidino with the adjacent nitrogen atom; and
X is a halogen anion.

4. A compound according to claim 1, wherein together $R^e$ and $R^f$ form pyrrolidino or piperidino with the adjacent nitrogen atom.

5. A compound according to claim 1, selected from N-ethyl-de(N-methyl)-8,9-anhydroerythromycin A 6,9-hemiketai, 8,9-anhydroerythromycin A 6,9-hemiketai propargyl bromide, 8,9-anhydroerythromycin A 6,9-hemiketal propargyl chloride, 8,9-anhydroerythromycin A 6,9-hemiketal ethyl bromide, 8,9-anhydroerythromycin A 6,9-hemiketal 2-hydroxyethyl bromide and N-isopropyl-de(N-methyl)-8,9-anhydroerythromycin A 6,9-hemiketal.

6. A compound according to claim 1, selected from dipropargyl-bis-(de(N-methyl))-8,9-anhydroerythromycin A 6,9-hemiketal propargyl bromide and 8,9-anhydroerythromycin A 6,9-hemiketal allyl bromide.

7. A process for preparing a compound of the formula [4]

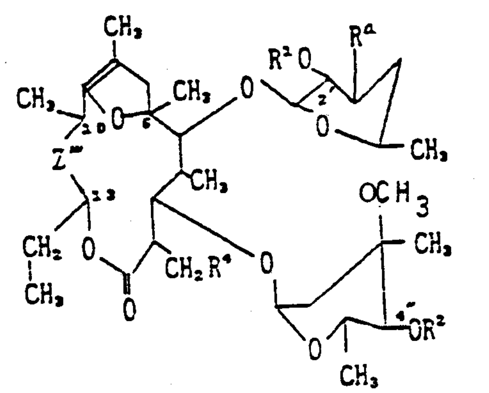

[4]

wherein Z''' Is as defined for Z, $R^4$ is hydrogen and the other symbols are as defined in claim 1, or a salt thereof, which comprises treating a compound of the following formula or a salt thereof under an acidic condition:

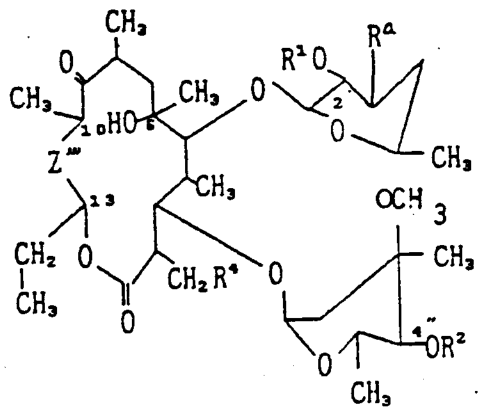

[3]

wherein Z''' is as defined for Z, $R^4$ is hydrogen and the other symbols are as defined in claim 1.

**8.** A process for preparing a compound of the formula [I]

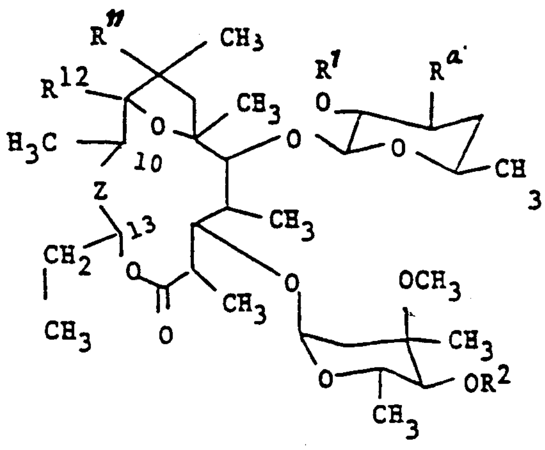

[I]

wherein the symbols are as defined in claim 1, or a salt thereof, which comprises subjecting a compound of the following formula to N-alkylation or N-alkynylation reaction:

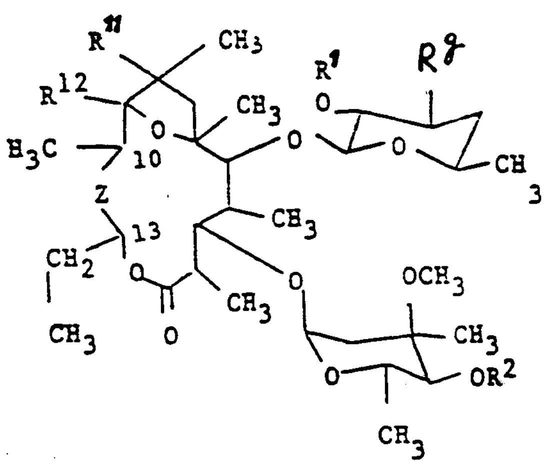

[5]

wherein $R^g$ is (1) a group of the formula -NH-$R^b$
or

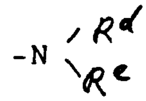

in which $R^b$, $R^d$ and $R^e$ are as defined in claim 1, (2)pyrrolidino or (3)piperidino, and the other symbols are as defined in claim 1.

**Claims for the following Contracting State : AT**

1. A process for preparing a compound of the formula:

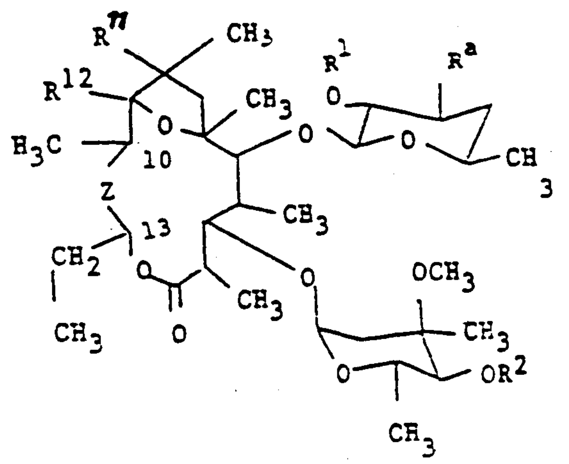

[I]

wherein $R^1$ is hydrogen;
$R^2$ is hydrogen;
Z is a group of the formula

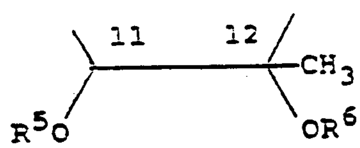

wherein $R^5$ is hydrogen and $R^6$ is hydrogen;
$R^a$ is a group of the formula

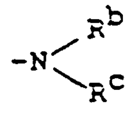

in which $R^b$ is methyl, and $R^c$ is ethyl or isopropyl,
or $R^a$ is a group of the formula

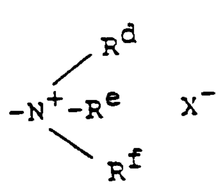

in which $R^d$ is methyl, and $R^e$ and $R^f$ which may be the same or different, are (1) a methyl, ethyl or isopropyl radical which may be substituted by hydroxyl, cyano, halogen or cylopropyl, or (2) a propargyl radical,
or together $R^e$ and $R^f$ form pyrrolidino or piperidino with the adjacent nitrogen atom;
X is a halogen anion;
and
$R^{11}$ end $R^{12}$ both taken together form a chemical bond,
or a salt thereof, which comprises

(a) treating a compound of the following formula or a salt thereof under an acidic condition:

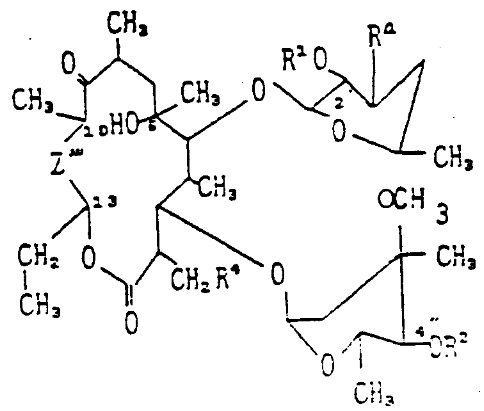

[3]

wherein $R^4$ is hydrogen, Z''' is as defined for Z, and Z and the other symbols are as defined above;
or

(b) subjecting a compound of the following formula to N-alkylation or N-alkynylation reaction:

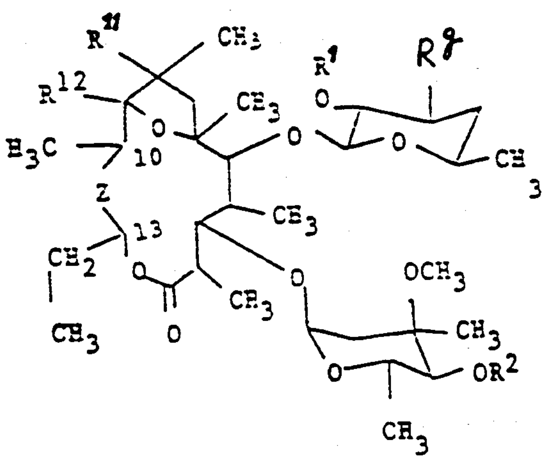

{5}

wherein $R^g$ is (1)a group of the formula -NH-$R^b$
or

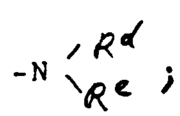

(2) pyrrolidino or
(3)piperidino, in which $R^b$, $R^d$ and $R^e$ and the other symbols are as defined above.

**2.** A process according to claim 1, wherein $R^a$ is N-methyl-N-ethylamino radical.

3. A process according to claim 1, wherein $R^a$ is a group of the formula:

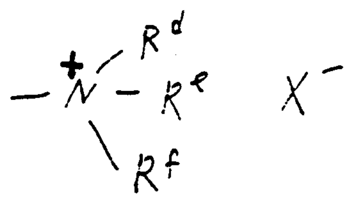

wherein $R^d$ is methyl, and $R^e$ and $R^f$ which may be the same or different, are (1) a methyl, ethyl or isopropyl radical which may be substituted by hydroxyl, cyano, halogen or cylopropyl, or (2) a propargyl radical,
or together $R^e$ and $R^f$ form pyrrolidino or piperidino with the adjacent nitrogen atom; and
X is a halogen anion.

4. A process according to claim 1, wherein together $R^e$ and $R^f$ form pyrrolidino or piperidino with the adjacent nitrogen atom.

5. A process according to claim 1, wherein the prepared compound is selected from N-ethyl-de(N-methyl)-8,9-anhydroerythromycin A 6,9-hemiketal, 8,9-anhydroerythromycin A 6,9-hemiketal propargyl bromide, 8,9-anhydroerythromycin A 6,9-hemiketal propargyl chloride, 8,9-anhydroerythromycin A 6,9-hemiketal ethyl bromide, 8,9-anhydroerythromycin A 6,9-hemiketal 2-hydroxyethyl bromide and N-isopropyl-de(N-methyl)-8,9-anhydroerythromycin A 6,9-hemiketal.

6. A process according to claim 1, wherein the prepared compound is selected from dipropargyl-bis-(de-(N-methyl))-8,9-anhydroerythromycinA 6,9-hemiketal propargyl bromide and 8,9-anhydroerythromycin A 6,9-hemiketal allyl bromide.

**Patentansprüche**
**Patentansprüche für folgende Vertragsstaaten : BE, DE, FR, IT, LU, NL, SE, CH, GB, LI**

1. Verbindung der Formel:

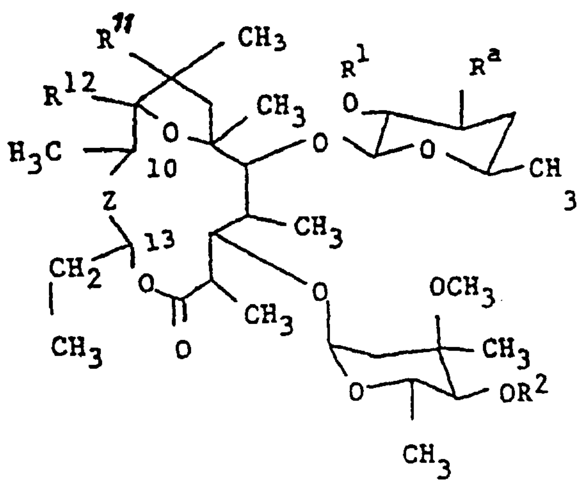

[I]

worin
$R^1$ Wasserstoff ist;
$R^2$ Wasserstoff ist;
Z eine Gruppe der Formel

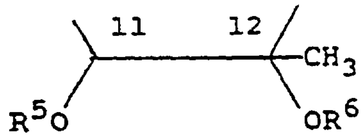

ist, worin $R^5$ Wasserstoff und $R^6$ Wasserstoff ist;

$R^a$ eine Gruppe der Formel

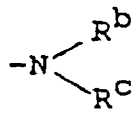

ist, in welcher $R^b$ Methyl ist und $R^c$ Ethyl oder Isopropyl ist, oder

$R^a$ eine Gruppe der Formel

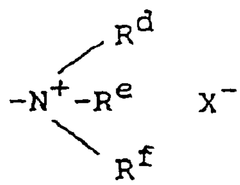

ist, in welcher $R^d$ Methyl ist und $R^e$ und $R^f$, welche gleich oder verschieden sein können, (1) ein Methyl-, Ethyl- oder Isopropylrest sind, der durch Hydroxyl, Cyano, Halogen oder Cyclopropyl substituiert sein kann, oder (2) ein Propargylrest ist, oder $R^e$ und $R^f$ zusammen mit dem benachbarten Stickstoffatom Pyrrolidino oder Piperidino bilden;

X ein Halogenanion ist und

$R^{11}$ und $R^{12}$ beide zusammengenommen eine chemische Bindung bilden,

oder ein Salz derselben.

**2.** Verbindung gemäß Anspruch 1, worin $R^a$ ein N-Methyl-N-ethylaminorest ist.

**3.** Verbindung gemäß Anspruch 1, worin $R^a$ eine Gruppe der Formel

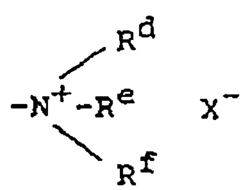

ist, in welcher $R^d$ Methyl ist und $R^e$ und $R^f$, welche gleich oder verschieden sein können, (1) ein Methyl-, Ethyl- oder Isopropylrest sind, der durch Hydroxyl, Cyano, Halogen oder Cyclopropyl substituiert sein kann, oder (2) ein Propargylrest ist, oder $R^e$ und $R^f$ zusammen mit dem benachbarten Stickstoffatom Pyrrolidino oder Piperidino bilden und X ein Halogenanion ist.

**4.** Verbindung gemäß Anspruch 1, worin $R^e$ und $R^f$ zusammen mit dem benachbarten Stickstoffatom Pyrrolidino oder Piperidino bilden.

**5.** Verbindung gemäß Anspruch 1, die aus N-Ethyl-des(N-methyl)-8,9-anhydroerythromycin A-6,9-hemiketal,

8,9-Anhydroerythromycin A-6,9-hemiketal-propargylbromid,

8,9-Anhydroerythromycin A-6,9-hemiketal-propargylchlorid,
8,9-Anhydroerythromycin A-6,9-hemiketal-ethylbromid,
8,9-Anhydroeryhromycin A-6,9-hemiketal-2-hydroxyethylbromid und N-Isopropyl-des(N-methyl)-8,9-anhydroerythromycin A-6,9-hemike-tal ausgewählt ist.

6. Verbindung gemäß Anspruch 1, die aus Dipropargyl-bis-(des(N-methyl))-8,9-anhydroerythromycin A-6,9-hemiketal-propargylbromid und 8,9-Anhydroerythromycin A-6,9-hemiketal-allylbromid ausgewählt ist.

7. Verfahren zur Herstellung einer Verbindung der Formel [4]

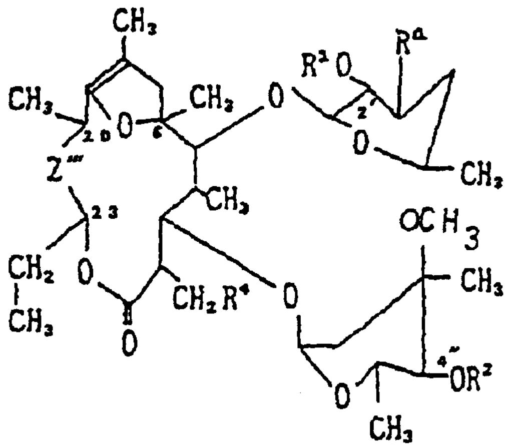

[4]

worin Z''' wie für Z definiert ist, $R^4$ Wasserstoff ist und die anderen Symbole wie in Anspruch 1 definiert sind, oder eines Salzes derselben, welches das Behandeln einer Verbindung der folgenden Formel oder eines Salzes derselben

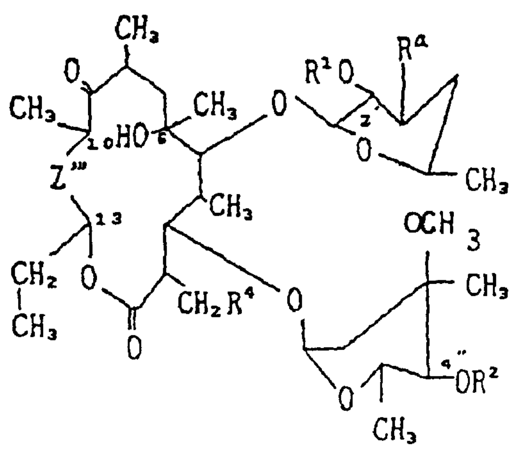

[3]

worin Z''' wie für Z definiert ist, $R^4$ Wasserstoff ist und die anderen Symbole wie in Anspruch 1 definiert sind, unter sauren Bedingungen umfaßt.

**8.** Verfahren zur Herstellung einer Verbindung der Formel [I]

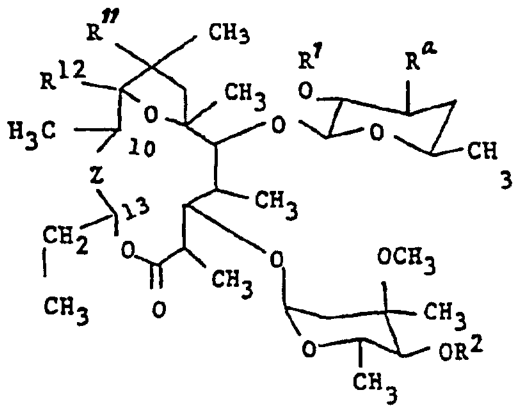

[I]

worin die Symbole wie in Anspruch 1 definiert sind, oder eines Salzes derselben, welches das Unterziehen einer Verbindung der folgenden Formel

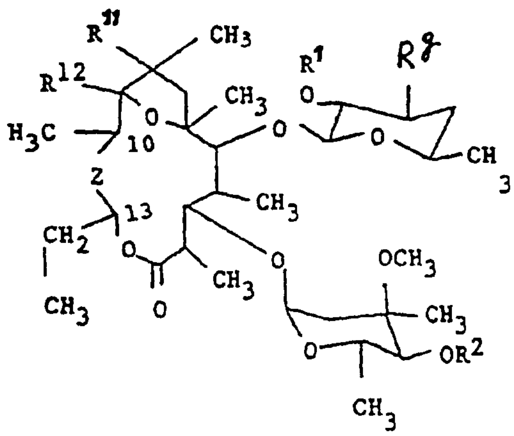

[5]

worin $R^g$ (1) eine Gruppe der Formel -NH-$R^b$ oder

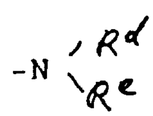

,

in welchen $R^b$, $R^d$ und $R^e$ wie in Anspruch 1 definiert sind, (2) Pyrrolidino oder (3) Piperidino ist und die anderen Symbole wie in Anspruch 1 definiert sind, einer N-Alkylierungs- oder N-Alkinylierungsreaktionumfaßt.

**Patentansprüche für folgenden Vertragsstaat : AT**

**1.** Verfahren zur Herstellung einer Verbindung der Formel:

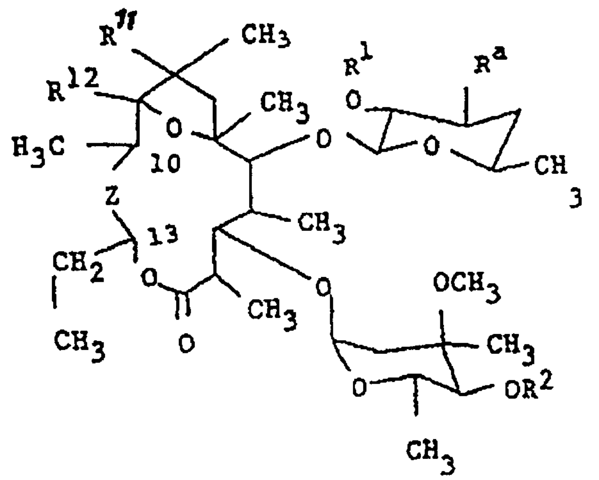

[I]

worin

| | |
|---|---|
| $R^1$ | Wasserstoff ist; |
| $R^2$ | Wasserstoff ist; |
| Z | eine Gruppe der Formel |

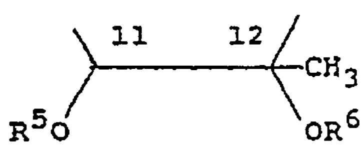

ist, worin $R^5$ Wasserstoff und $R^6$ Wasserstoff ist;

$R^a$ eine Gruppe der Formel

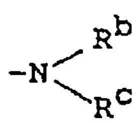

ist, in welcher $R^b$ Methyl ist und $R^c$ Ethyl oder Isopropyl ist, oder

$R^a$ eine Gruppe der Formel

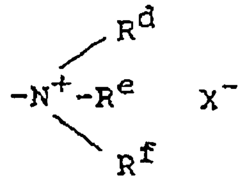

ist, in welcher $R^d$ Methyl ist und $R^e$ und $R^f$, welche gleich oder verschieden sein können, (1) ein Methyl-, Ethyl- oder Isopropylrest sind, der durch Hydroxyl, Cyano, Halogen oder Cyclopropyl substituiert sein kann, oder (2) ein Propargylrest ist, oder $R^e$ und $R^f$ zusammen mit dem benachbarten Stickstoffatom Pyrrolidino oder Piperidino bilden;

X ein Halogenanion ist und

$R^{11}$ und $R^{12}$ beide zusammengenommen eine chemische Bindung bilden,

oder eines Salzes derselben, welches

(a) das Behandeln einer Verbindung der folgenden Formel oder eines Salzes derselben R

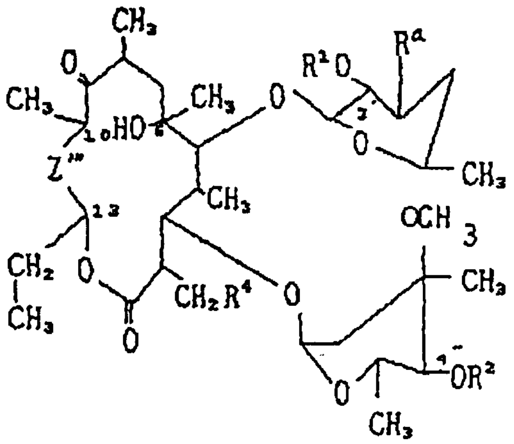

[3]

worin Z''' wie für Z definiert ist, $R^4$ Wasserstoff ist und die anderen Symbole vorstehend 1 definiert sind, unter sauren Bedingungen oder

(b) das Unterziehen einer Verbindung der folgenden Formel

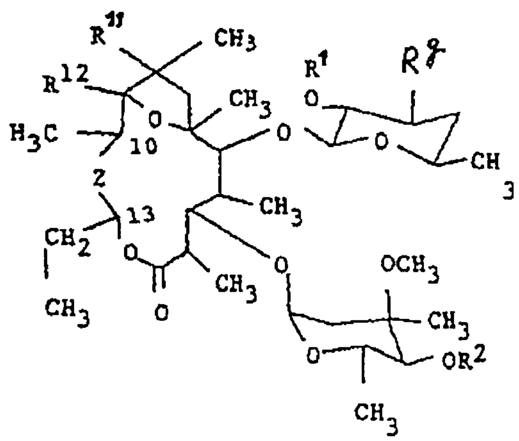

[5]

worin $R^g$ (1) eine Gruppe der Formel -NH-$R^b$ oder

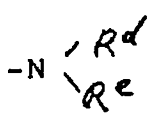 ,

(2) Pyrrolidino oder (3) Piperidino ist, in welchen $R^b$, $R^d$ und $R^e$ und die anderen Symbole wie vorstehend definiert sind, einer N-Alkylierungs- oder N-Alkinylierungsreaktion umfaßt.

**2.** Verfahren gemäß Anspruch 1, in welchem $R^a$ ein N-Methyl-N-ethylaminorest ist.

3. Verfahren gemäß Anspruch 1, in welchem $R^a$ eine Gruppe der Formel

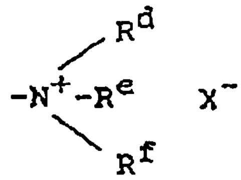

ist, in welcher $R^d$ Methyl ist und $R^e$ und $R^f$, welche gleich oder verschieden sein können, (1) ein Methyl-, Ethyl- oder Isopropylrest sind, der durch Hydroxyl, Cyano, Halogen oder Cyclopropyl substituiert sein kann, oder (2) ein Propargylrest ist, oder $R^e$ und $R^f$ zusammen mit dem benachbarten Stickstoffatom Pyrrolidino oder Piperidino bilden und X ein Halogenanion ist.

4. Verfahren gemäß Anspruch 1, in welchem $R^e$ und $R^f$ zusammen mit dem benachbarten Stickstoffatom Pyrrolidino oder Piperidino bilden.

5. Verfahren gemäß Anspruch 1, in welchem die hergestellte Verbindung aus N-Ethyl-des(N-methyl)-8,9-anhydroerythromycin A-6,9-hemiketal,
8,9-Anhydroerythromycin A-6,9-hemiketal-propargylbromid,
8,9-Anhydroerythromycin A-6,9-hemiketal-propargylchlorid,
8,9-Anhydroerythromycin A-6,9-hemiketal-ethylbromid,
8,9-Anhydroerythromycin A-6,9-hemiketal-2-hydroxyethylbromid und N-Isopropyl-des(N-methyl)-8,9-anhydroerythromycin A-6,9-hemiketal ausgewählt ist.

6. Verfahren gemäß Anspruch 1, in welchem die hergestellte Verbindung aus Dipropargyl-bis-(des(N-methyl))-8,9-anhydroerythromycin A-6,9-hemiketal-propargylbromid und 8,9-Anhydroerythromycin A-6,9-hemiketal-allylbromid ausgewählt ist.

**Revendications**
**Revendications pour les Etats contractants suivants : BE, DE, FR, IT, LU, NL, SE, CH, GB, LI**

1. Composé de formule :

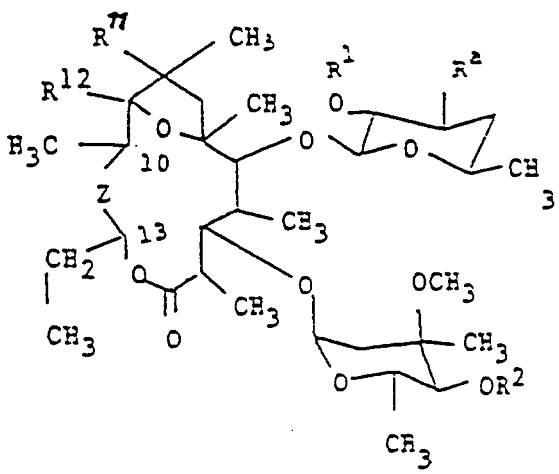

(I)

dans laquelle
$R^1$ représente un atome d'hydrogène ;
$R^2$ représente un atome d'hydrogène ;
Z représente un groupe de formule

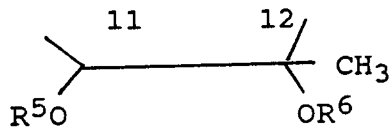

dans laquelle $R^5$ et $R^6$ représentent chacun un atome d'hydrogène ;
$R^a$ représente un groupe de formule

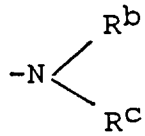

dans laquelle $R^b$ représente un groupe méthyle et $R^c$ représente un groupe éthyle ou isopropyle, ou bien $R^a$ représente un groupe de formule

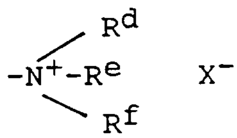

dans laquelle $R^d$ représente un groupe méthyle et $R^e$ et $R^f$,qui peuvent être identiques ou différents, représentent (1) un radical méthyle, éthyle ou isopropyle, qui peut être substitué par hydroxyle, cyano, halogéno ou cyclopropyle, ou bien (2) un radical propargyle, ou encore, pris conjointement, $R^e$ et $R^f$ forment un groupe pyrrolidino ou pipéridino avec l'atome d'azote adjacent, et X représente un anion halogénure ;
et $R^{11}$ et $R^{12}$, pris ensemble, forment une liaison chimique ;
ou un sel d'un tel composé.

**2.** Composé conforme à la revendication 1, dans lequel $R^a$ représente un radical N-méthyl-N-éthyl-amino.

**3.** Composé conforme à la revendication 1, dans lequel $R^a$ représente un groupe de formule

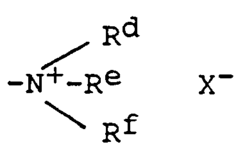

dans laquelle $R^d$ représente un groupe méthyle et $R^e$ et $R^f$,qui peuvent être identiques ou différents, représentent (1) un radical méthyle, éthyle ou isopropyle, qui peut être substitué par hydroxyle, cyano, halogéno ou cyclopropyle, ou bien (2) un radical propargyle, ou encore, pris conjointement, $R^e$ et $R^f$ forment un groupe pyrrolidino ou pipéridino avec l'atome d'azote adjacent, et X représente un anion halogénure.

**4.** Composé conforme à la revendication 1, dans lequel $R^e$ et $R^f$ forment ensemble un cycle pyrrolidine ou pipéridine avec l'atome d'azote adjacent.

**5.** Composé conforme à la revendication 1, choisi parmi le 6,9-hémicétal de N-éthyl-dé(N-méthyl)-8,9-anhydroérythromycine A, le 6,9-hémicétal de 8,9-anhydroérythromycine A-bromure de propargyle, le 6,9-hémicétal de 8,9-anhydroérythromycine A-chlorure de propargyle, le 6,9-hémicétal de 8,9-anhydroérythromycine A-bromure d'éthyle, le 6,9-hémicétal de 8,9-anhydroérythromycine A-bromure de 2-hydroxyéthyle, et le 6,9-hémicétal de N-isopropyl-dé(N-méthyl)-8,9-anhydroérythromycine A.

6. Composé conforme à la revendication 1, choisi parmi le 6,9-hémicétal de dipropargyl-bis-(dé(N-méthyl))-8,9-anhydroérythromycine A-bromure de propargyle et le 6,9-hémicétal de 8,9-anhydroérythromycine A-bromure d'allyle.

7. Procédé de préparation d'un composé de formule (4)

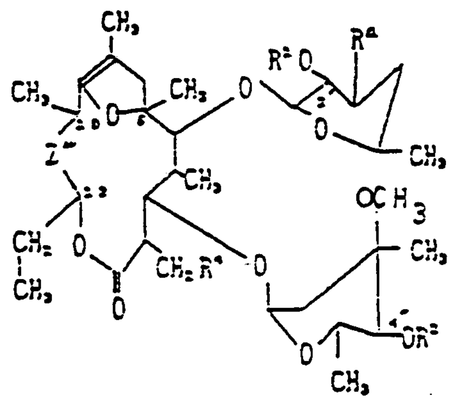

(4)

dans laquelle Z''' possède la même définition que Z, $R^4$ représente un atome d'hydrogène et les autres symboles ont les définitions indiquées dans la revendication 1, ou d'un sel d'un tel composé, qui comprend le traitement, dans des conditions acides, d'un composé de formule suivante ou de l'un de ses sels :

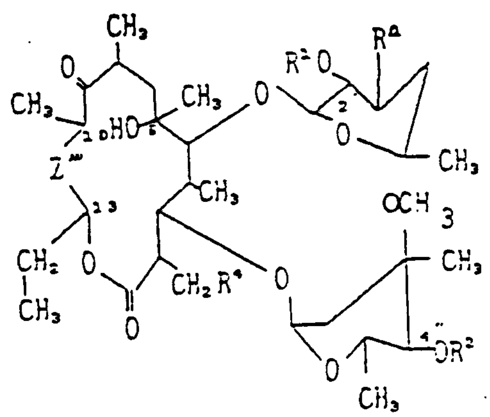

(3)

dans laquelle Z''' a la même définition que Z, $R^4$ représente un atome d'hydrogène et les autres symboles ont les définitions indiquées dans la revendication 1.

**8.** Procédé de préparation d'un composé de formule [I]

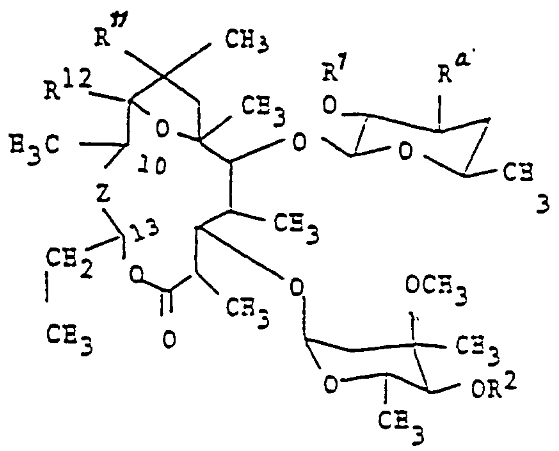

[I]

dans laquelle les symboles ont les définitions indiquées dans la revendication 1, ou d'un sel d'un tel composé, qui comprend le fait de soumettre à une réaction de N-alkylation ou de N-alcynylation un composé de formule suivante :

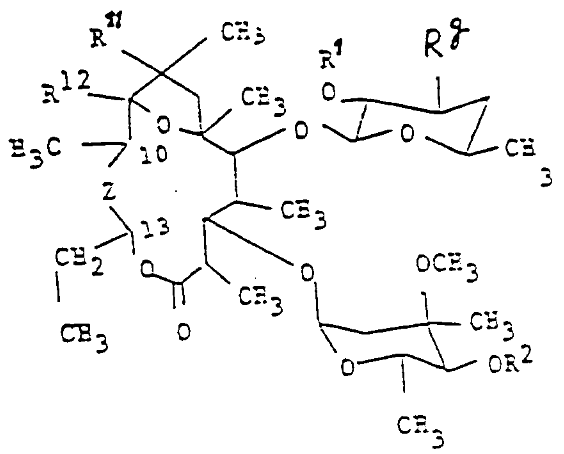

[5]

dans laquelle $R^g$ représente (1) un groupe de formule -NH-$R^b$ ou -N($R^d$)$R^e$, où $R^b$, $R^d$ et $R^e$ ont les définitions indiquées dans la revendication 1, (2) un groupe pyrrolidino ou (3) un groupe pipéridino, et les autres symboles ont les définitions indiquées dans la revendication 1.

**Revendications pour l'Etat contractant suivant : AT**

**1.** Procédé de préparation d'un composé de formule :

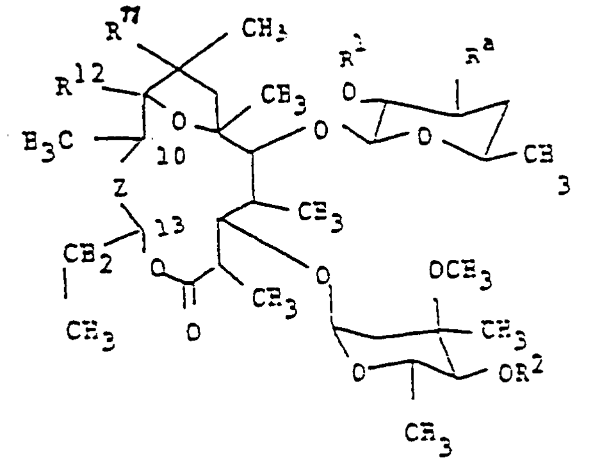

(I)

dans laquelle
$R^1$ représente un atome d'hydrogène ;
$R^2$ représente un atome d'hydrogène ;
Z représente un groupe de formule

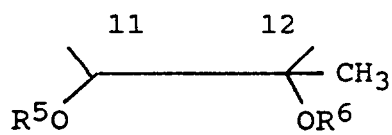

dans laquelle $R^5$ et $R^6$ représentent chacun un atome d'hydrogène ;
$R^a$ représente un groupe de formule

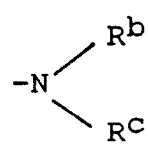

dans laquelle $R^b$ représente un groupe méthyle et $R^c$ représente un groupe éthyle ou isopropyle,
ou bien $R^a$ représente un groupe de formule

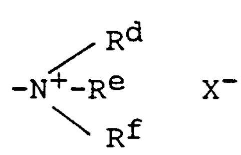

dans laquelle $R^d$ représente un groupe méthyle et $R^e$ et $R^f$,qui peuvent être identiques ou différents, représentent (1) un radical méthyle, éthyle ou isopropyle, qui peut être substitué par hydroxyle, cyano, halogéno ou cyclopropyle, ou bien (2) un radical propargyle, ou encore, pris conjointement, $R^e$ et $R^f$ forment un groupe pyrrolidino ou pipéridino avec l'atome d'azote adjacent, et X représente un anion halogénure ;
et $R^{11}$ et $R^{12}$, pris ensemble, forment une liaison chimique ;
ou d'un sel d'un tel composé,
qui comprend

a) le traitement dans des conditions acides, d'un composé de formule suivante ou de l'un de ses sels :

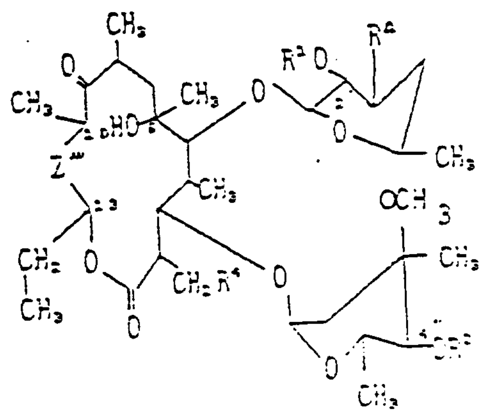

[3]

dans laquelle Z''' possède la même définition que Z, $R^4$ représente un atome d'hydrogène et Z et les autres symboles ont les définitions indiquées plus haut, ou

b) le fait de soumettre à une réaction de N-alkylation ou de N-alcynylation un composé de formule suivante :

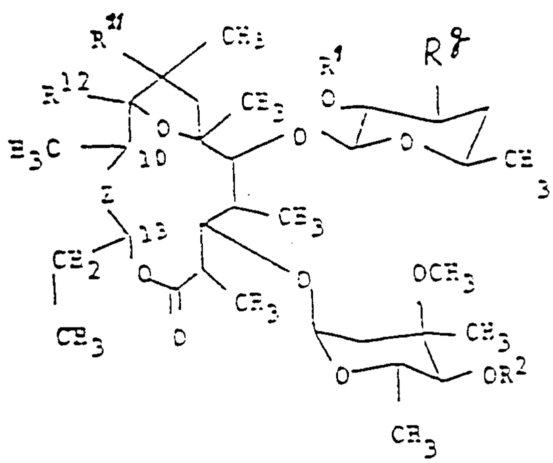

[5]

dans laquelle $R^g$ représente (1) un groupe de formule $-NH-R^b$ ou $-N(R^d)R^e$, (2) un groupe pyrrolidino ou (3) un groupe pipéridino, où $R^b$, $R^d$ et $R^e$ et les autres symboles ont les définitions indiquées plus haut

**2.** Procédé conforme à la revendication 1, dans lequel $R^a$ représente un radical N-méthyl-N-éthyl-amino.

**3.** Procédé conforme à la revendication 1, dans lequel $R^a$ représente un groupe de formule

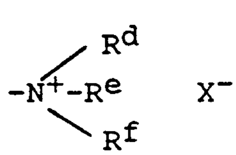

dans laquelle $R^d$ représente un groupe méthyle et $R^e$ et $R^f$,qui peuvent être identiques ou différents, représentent (1) un radical méthyle, éthyle ou isopropyle, qui peut être substitué par hydroxyle, cyano, halogéno ou cyclopropyle, ou bien (2) un radical propargyle, ou encore, pris conjointement, $R^e$ et $R^f$ forment un groupe pyrrolidino ou pipéridino avec l'atome d'azote adjacent, et X représente un anion

halogénure.

4. Procédé conforme à la revendication 1, dans lequel $R^e$ et $R^f$ forment ensemble un cycle pyrrolidine ou pipéridine avec l'atome d'azote adjacent.

5. Procédé conforme à la revendication 1, dans lequel le composé préparé est choisi parmi le 6,9-hémicétal de N-éthyl-dé(N-méthyl)-8,9-anhydroérythromycine A, le 6,9-hémicétal de 8,9-anhydro-érythromycine A-bromure de propargyle, le 6,9-hémicétal de 8,9-anhydroérythromycine A-chlorure de propargyle, le 6,9-hémicétal de 8,9-anhydroérythromycine A-bromure d'éthyle, le 6,9-hémicétal de 8,9-anhydroérythromycine A-bromure de 2-hydroxyéthyle, et le 6,9-hémicétal de N-isopropyl-dé(N-méthyl)-8,9-anhydroérythromycineA.

6. Procédé conforme à la revendication 1, dans lequel le composé préparé est choisi parmi le 6,9-hémicétal de dipropargyl-bis-(dé(N-méthyl))-8,9-anhydroérythromycine A-bromure de propargyle et le 6,9-hémicétal de 8,9-anhydroérytriromycine A-bromure d'allyle.